# EUROPEAN PATENT SPECIFICATION

(11) **EP 3 583 117 B1**
(45) Date of publication and mention of the grant of the patent: **24.04.2024**
(21) Application number: 18708011.4
(22) Date of filing: 13.02.2018
(51) Int. Cl.: C07K 14/47, C12N 5/16, C12N 15/02, C12N 5/077

(54) **IN VITRO METHOD FOR FUSING TWO OR MORE CELLS, IN VITRO, METHOD FOR DELIVERING A GENE OF INTEREST, AND PHARMACEUTICAL COMPOSITION FOR USE IN THE TREATMENT OF A DISEASE**
IN-VITRO VERFAHREN ZUR FUSION ZWEI ODER MEHR ZELLEN, IN-VITRO-VERFAHREN ZUR GENVERABREICHUNG UND PHARMAZEUTISCHE ZUSAMMENSETZUNG ZUR VERWENDUNG BEI DER BEHANDLUNG EINER KRANKHEIT
PROCÉDÉ IN VITRO DE FUSION DE DEUX CELLULES OU PLUS, PROCÉDÉ IN VITRO D'ADMINISTRATION D'UN GÈNE D'INTÉRÊT, ET COMPOSITION PHARMACEUTIQUE DESTINÉE À ÊTRE UTILISÉE DANS LE TRAITEMENT D'UNE MALADIE

(30) Priority: 14.02.2017 US 201762458634 P
(43) Date of publication of application: 25.12.2019
(73) Proprietor: Children's Hospital Medical Center, Cincinnati, OH 45229-3039 (US)
(72) Inventor: MILLAY, Douglas, Park Hills, Kentucky 41011 (US)
(74) Representative: Forresters IP LLP
(86) International application number: PCT/US2018/017991
(87) International publication number: WO 2018/152103

(56) References cited:
- WO-A1-2014/210448
- WO-A1-2018/087720
- WO-A1-2018/156397
- WO-A2-2007/128984
- YAN-MEI CHEN ET AL: "Molecular Cloning and Functional Analysis of ESGP, an Embryonic Stem Cell and Germ Cell Specific Protein", ACTA BIOCHIMICA ET BIOPHYSICA SINICA, vol. 37, no. 12, 1 December 2005 (2005-12-01), pages 789-796, XP055467204, US ISSN: 1672-9145, DOI: 10.1111/j.1745-7270.2005.00120.x -& DATABASE UniProt [Online] 27 September 2017 (2017-09-27), "RecName: Full=Protein myomixer {ECO:0000303|PubMed:28386024}; AltName: Full=Embryonic stem cell- and germ cell-specific protein {ECO:0000303|PubMed:16331322}; Short=ESGP {ECO:0000303|PubMed:16331322}; AltName: Full=Microprotein inducer of fusion {ECO:0000303|PubMed:28569745}; Short=Protein minion {E", XP002780295, retrieved from EBI accession no. UNIPROT:Q2Q5T5 Database accession no. Q2Q5T5
- DATABASE Refseq [Online] NCBI; 5 December 2016 (2016-12-05), "uncharacterized LOC101929726 homolog [Panthera pardus]", XP002780296, retrieved from NCBI accession no. XP_019312828 Database accession no. XP_019312828
- DATABASE UniProt [Online] 31 October 2012 (2012-10-31), "SubName: Full=Uncharacterized protein {ECO:0000313|Ensembl:ENSCAFP00000039846};" , XP002780297, retrieved from EBI accession no. UNIPROT:J9P2R8 Database accession no. J9P2R8
- DATABASE Refseq [Online] 23 May 2016 (2016-05-23), "uncharacterized LOC101929726 homolog [Oryctolagus cuniculus]", XP002780298, retrieved from NCBI accession no. XP_017200354 Database accession no. XP_017200354
- DATABASE UniProt [Online] 16 November 2011 (2011-11-16), "SubName: Full=Myomixer, myoblast fusion factor {ECO:0000313|Ensembl:ENSLAFP00000022282};" , XP002780299, retrieved from EBI accession no. UNIPROT:G3U374 Database accession no. G3U374
- DATABASE EMBL [Online] 14 April 2007 (2007-04-14), "Mus musculus cDNA, Abe mouse ES cell cDNA library, clone:19990722EK06G03.", XP002780300, retrieved from EBI accession no. EM_EST:AV448123 Database accession no. AV448123
- DATABASE EMBL [Online] 14 April 2007 (2007-04-14), "Mus musculus cDNA, Abe mouse ES cell cDNA library, clone:20000321HM02A08.", XP002780321, retrieved from EBI accession no. EM_EST:AV576288 Database accession no. AV576288
- DATABASE EMBL [Online] 9 January 2005 (2005-01-09), "S235P6522FE8.T0 ItalianGreyhound Canis familiaris STS genomic, sequence tagged site.", XP002780301, retrieved from EBI accession no. EM_STS:BV267573 Database accession no. BV267573
- DATABASE EMBL [Online] 2 April 2013 (2013-04-02), "Callithrix jacchus mRNA, clone: MES-033H02, 5'end sequence, expressed in es cells.", XP002780302, retrieved from EBI accession no. EM_EST:HX384689 Database accession no. HX384689
- DATABASE EMBL [Online] 29 October 2016 (2016-10-29), "JP 2014500723-A/18356: Polycomb-Associated Non-Coding RNAs.", XP002780303, retrieved from EBI accession no. EM_PAT:LF210853 Database accession no. LF210853
- DATABASE EMBL [Online] 29 October 2016 (2016-10-29), "JP 2014500723-A/8628: Polycomb-Associated Non-Coding RNAs.", XP002780304, retrieved from EBI accession no. EM_PAT:LF201125 Database accession no. LF201125
- DATABASE EMBL [Online] 14 April 2007 (2007-04-14), "Mus musculus cDNA, Abe mouse ES cell cDNA library, clone:20000321HM02A08.", XP002780305, retrieved from EBI accession no. EM_EST:AV576288 Database accession no. AV576288
- DOUGLAS P. MILLAY ET AL: "Structure-function analysis of myomaker domains required for myoblast fusion", PROCEEDINGS OF THE NATIONAL ACADEMY OF SCIENCES, vol. 113, no. 8, 8 February 2016 (2016-02-08), pages 2116-2121, XP055467574, ISSN: 0027-8424, DOI: 10.1073/pnas.1600101113
- S. M. ABMAYR ET AL: "Myoblast fusion: lessons from flies and mice", DEVELOPMENT, vol. 139, no. 4, 24 January 2012 (2012-01-24), pages 641-656, XP055467518, GB ISSN: 0950-1991, DOI: 10.1242/dev.068353
- PENGPENG BI ET AL: "Control of muscle formation by the fusogenic micropeptide myomixer", SCIENCE, vol. 356, no. 6335, 21 April 2017 (2017-04-21), pages 323-327, XP055440750, ISSN: 0036-8075, DOI: 10.1126/science.aam9361
- QIAO ZHANG ET AL: "The microprotein Minion controls cell fusion and muscle formation", NATURE COMMUNICATIONS, vol. 8, 1 June 2017 (2017-06-01), page 15664, XP055440729, DOI: 10.1038/ncomms15664
- MALGORZATA E. QUINN ET AL: "Myomerger induces fusion of non-fusogenic cells and is required for skeletal muscle development", NATURE COMMUNICATIONS, vol. 8, 1 June 2017 (2017-06-01), page 15665, XP055440754, DOI: 10.1038/ncomms15665
- SRIHARI C. SAMPATH ET AL: "Myoblast fusion confusion: the resolution begins", SKELETAL MUSCLE, vol. 8, no. 1, 31 January 2018 (2018-01-31), XP055467216, London, UK ISSN: 2044-5040, DOI: 10.1186/s13395-017-0149-3
- "Mus musculus cDNA, Abe mouse ES cell cDNA library, clone:20000321HM02A08", EMBL,, 14 April 2007 (2007-04-14), XP002780305,

## Description

### CROSS-REFERENCE TO RELATED APPLICATIONS

This application claims the benefit of U.S. Provisional Application No. 62/458,634, filed February 14, 2017 entitled "INDUCTION OF CELL FUSION BY A NOVEL FUSION FACTOR".

### GOVERNMENT RIGHTS

This invention was made with government support under NIH R01AR068286 awarded by the National Institutes of Health. The government has certain rights in the invention.

### BACKGROUND

The fusion of plasma membranes appears necessary for numerous biological processes from conception to the development of skeletal muscle, osteoclasts, trophoblasts, and giant cells. The molecular regulation of fusion is not as understood as it could be and the reconstitution of fusogenicity has not been achieved with mammalian proteins. Specifically, certain factors that participate in membrane coalescence have not been identified. Also, discoveries of specific fusion proteins and development of reconstitution systems have been historically helpful to decipher multiple types of membrane fusion; however, these systems are lacking for mammalian cellular fusion.

Myoblast fusion is a highly regulated process essential for muscle formation during development and regeneration. While numerous proteins have been shown to contribute to mammalian myoblast fusion, myomaker is the only known muscle-specific protein absolutely required for this process. Expression of myomaker in fibroblasts or mesenchymal stromal cells (MSCs) induces their fusion with muscle cells. Myomaker-expressing fibroblasts do not fuse to each other indicating that these cells harbor a competency to fuse, but only in the presence of a fusogenic cell (such as muscle cell). Thus, additional myocyte factors that confer fusogenicity appear to be required for reconstitution of fusion in myomaker+ fibroblasts.
Yan-Mei Chen et al.: "Molecular Cloning and Functional Analysis of ESGP, an Embryonic Stem Cell and Germ Cell Specific Protein", ACTA BIOCHIMICA ET BIOPHYSICA SINICA, vol. 37, no. 12, 1 December 2005 (2005-12-01), pages 789-796describe the identification of several putative Oct-4 downstream genes from mouse embryonic stem (ES) cells using suppression-subtractive hybridization method. In this study, one of the novel genes encoding an ES cell and germ cell specific protein (ESGP) was cloned by rapid amplification of cDNA ends. WO 2007/128984 A2 describes a method for the detection of the presence of or the risk of cancer in a patient, comprising, with reference to a normal control, the step of: (i) detecting the expression level of a gene characterised by given sequences, in a sample isolated from a patient, wherein an increased expression level of the gene characterised by any of a given sequence, or a decreased expression level of the gene characterised by any of given sequences, indicates the presence of or the risk of cancer in the patient from whom the sample was isolated.
Douglas P. Millay et al.: "Structure-function analysis of myomaker domains required for myoblast fusion",PROCEEDINGS OF THE NATIONAL ACADEMY OF SCIENCES, vol. 113, no. 8, 8 February 2016 (2016-02-08), pages 2116-2121, describe describes the approach for exploring myomaker structure and biochemical properties by disrupting myomaker expression in C2C12 muscle cell lines. The article presents a model of myomaker topology within the membrane and discusses the mechanistic basis of myoblast function.
WO 2014/210448 A1 refers to compositions and methods relating to myomaker induced muscle cell fusion. The methods section of this artice refers to the generation of myomaker-/- mice, and molecular methods like real-time PCR, in situ hybridizations, and so on.
S. M. Abmayr et al.: "Myoblast fusion: lessons from flies and mice", DEVELOPMENT, vol. 139, no. 4, 24 January 2012 (2012-01-24), pages 641-656, describe a study in which the data indicate that the majority of myomaker is embedded in the plasma membrane with seven membrane-spanning regions and a required intracellular C-terminal tail. The study describes that myomaker function is conserved in other mammalian orthologs; however, related family members (TMEM8a and TMEM8b) do not exhibit fusogenic activity.
Pengpeng Bi et al: "Control of muscle formation by the fusogenic micropeptide myomixer", SCIENCE, vol. 356, no. 6335, 21 April 2017 (2017-04-21), pages 323-327, conclude in their study that the Myomixer-Myomaker pair controls the critical step in myofiber formation during muscle development.
Qiao Zhang et al: "The microprotein Minion controls cell fusion and muscle formation",NATURE COMMUNICATIONS, vol. 8, 1 June 2017 (2017-06-01), page 15664, describe
Malgorzata E. Quinn et al: "Myomerger induces fusion of non-fusogenic cells and is required for skeletal muscle development", NATURE COMMUNICATIONS, vol. 8, 1 June 2017 (2017-06-01), page 15665, describe a study that identifies myomerger as a fundamental myoblast fusion protein and establish a system that begins to reconstitute mammalian cell fusion.
Sampath et al.: "Myoblast fusion confusion: the resolution begins", SKELETAL MUSCLE, vol. 8, no. 1, 31 January 2018 (2018-01-31), reviews the findings regarding the biology of Myomaker and Minion-Myomerger, places these findings in the context of known pathways in mammalian myoblast fusion, and highlights areas that require further investigation.
WO 2018/087720 A1 provides compositions, methods, and therapeutic uses related to fusogenic protein MINION (microprotein inducer of fusion).
WO 2018/156397 A1 describes the fusogenic promiting activity of the Myomixer protein.

Certain embodiments of the invention address one or more of the deficiencies described above. For example, some embodiments of the invention according to the independent claims include polypeptides comprising a myomerger polypeptide. Other embodiments of the invention according to the independent claims include myomerger nucleic acid molecules encoding polypeptides comprising a myomerger polypeptide. Still other embodiments of the invention according to the independent claims include modified cells comprising a myomerger nucleic acid molecule, and a myomerger polypeptide. Yet other embodiments of the invention according to the independent claims include methods of using modified cells. Additional embodiments of the invention according to the independent claims are also discussed herein.

### SUMMARY

The invention comprises an in vitro method for fusing two or more cells according to claim 1, an in vitro method for delivering a gene of interest according to claim 5 and a pharmaceutical composition for use in the treatment of a disease according to claim 10. Preferred embodiments of the invention are described in the dependent claims.
Exemplarily, the myomerger polypeptide may be a wt-myomerger polypeptide or a mutant-myomerger polypeptide, and may comprise at least one amino acid modification relative to a wt-myomerger polypeptide. In yet other examples, the polypeptide may comprise at least one amino acid modification relative to a wt-myomerger polypeptide and the at least one amino acid modification is an insertion, a deletion, or a substitution. The myomerger polypeptide of a first cell and an optional myomerger polypeptide of a second cell is selected from SEQ ID Nos: 36-38. In certain examples, the wt-myomerger polypeptide is selected from SEQ ID Nos: 36-38. The polypeptide sequence has at least an 80% sequence identity to one or more of SEQ ID NO: 36, SEQ ID NO: 37, and SEQ ID NO: 38. The polypeptide sequence may exemplarily have at least a 90% sequence identity to a wt-myomerger polypeptide.

The myomerger nucleic acid molecule encodes a myomerger polypeptide. The myomerger nucleic acid sequence of a first cell has at least an 80% identity to one or more sequences selected from SEQ ID Nos: 43-45. The myomerger nucleic acid molecule is in a first cell that is a modified cell and a non-muscle cell.

The inventive pharmaceutical composition a first cell that is a modified cell comprising a myomerger nucleic acid, a first myomerger polypeptide, a first myomaker polypeptide, and a gene of interest, and wherein the first cell is a non-muscle cell. The inventive pharmaceutical composition also comprises a second cell comprising a second myomaker polypeptide and optionally comprising a second myomerger polypeptide, wherein the myomerger nucleic acid sequence has at least an 80% identity to one or more of SEQ ID NO: 43, SEQ ID NO: 44, or SEQ ID NO: 45; and the first myomerger polypeptide sequence and the second myomerger polypeptide sequence each have at least an 80% identity to one or more of SEQ ID NO: 36, SEQ ID NO: 37, and SEQ ID NO: 38.

The inventive in vitro method for fusing two or more cells comprises contacting thefirst cell with a second cell to form a third cell. The third cell is a multinucleated cell. In certain embodiments, the second cell comprises the second myomerger polypeptide. In some embodiments, the second cell is a muscle cell, preferably a myoblast.

The inventive in vitro method for delivering a gene of interest comprises contacting the first cell with the second cell in vitro, which fuse to form a third cell. The third cell is a multinucleated cell and the gene of interest is delivered to the third cell upon fusion of the first cell with the second cell. In some embodiments, the second cell comprises the second myomerger polypeptide. In some embodiments, the contacting occurs *ex vivo.* In other embodiments, the second cell underexpresses the gene of interest, does not express the gene of interest, or expresses a defective version of the gene of interest.

### BRIEF DESCRIPTION OF THE DRAWINGS

The following drawings form part of the present specification and are included to further demonstrate certain aspects. The invention may be better understood by reference to one or more of these drawings in combination with the description of specific embodiments presented herein.

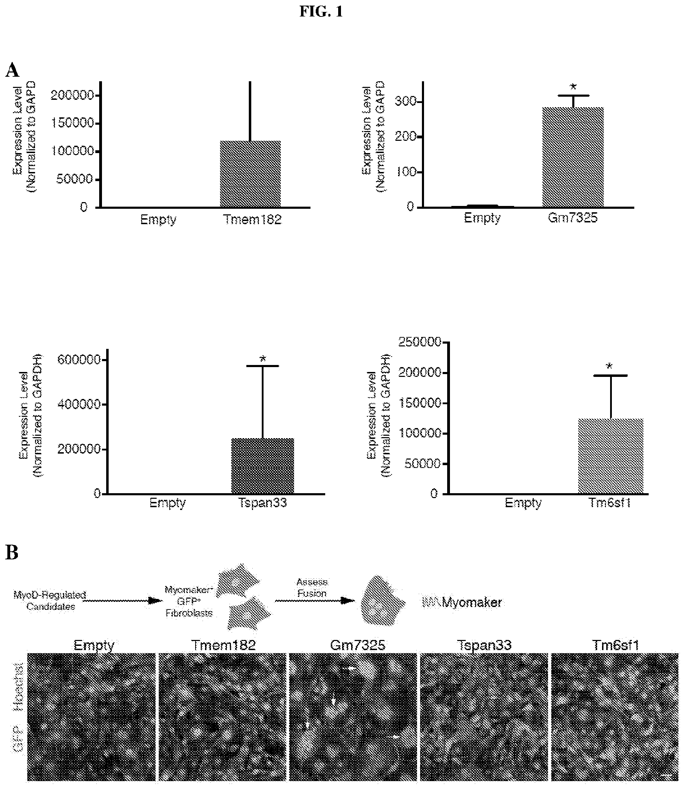

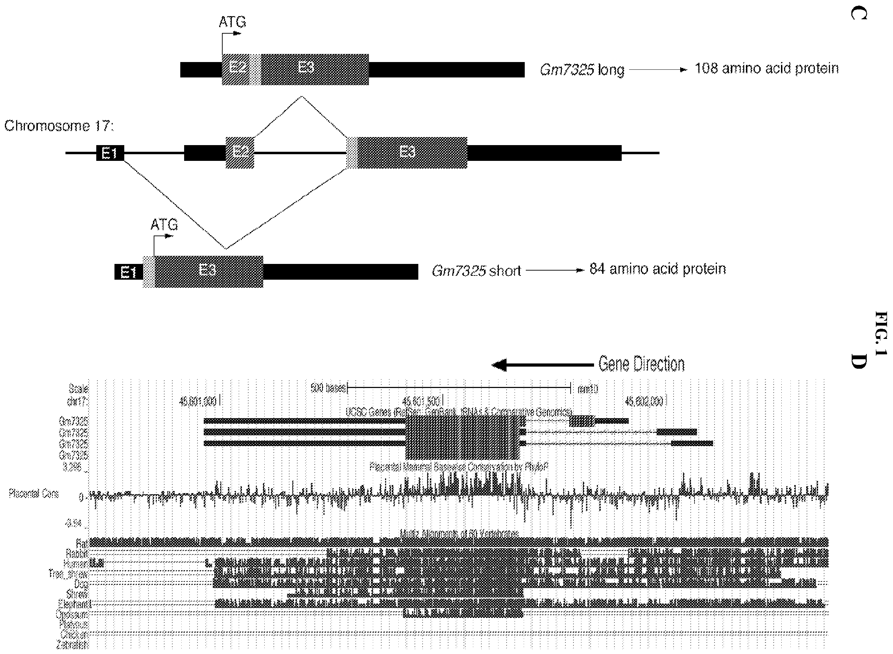

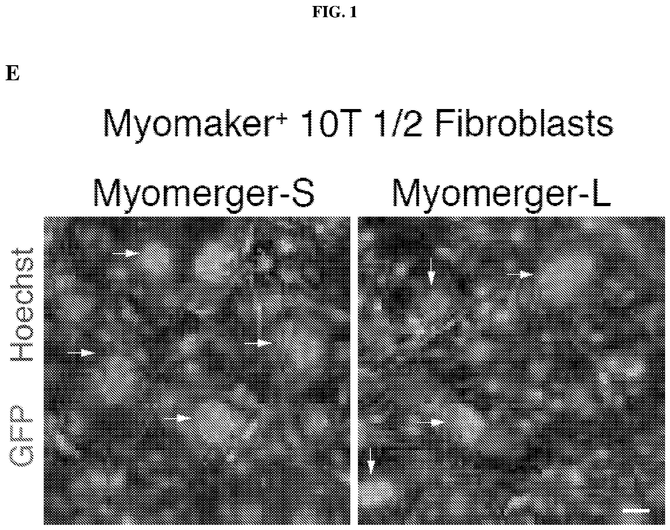

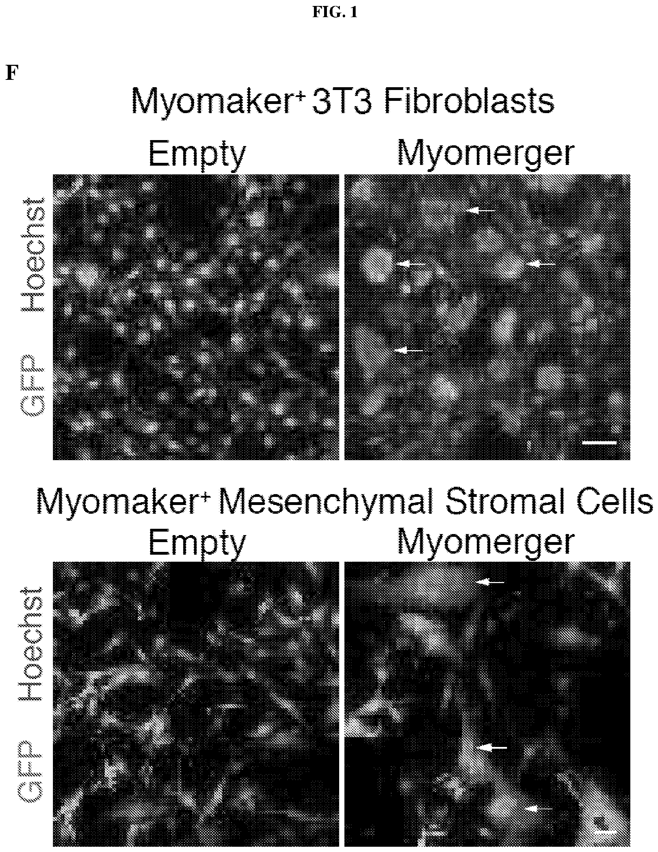

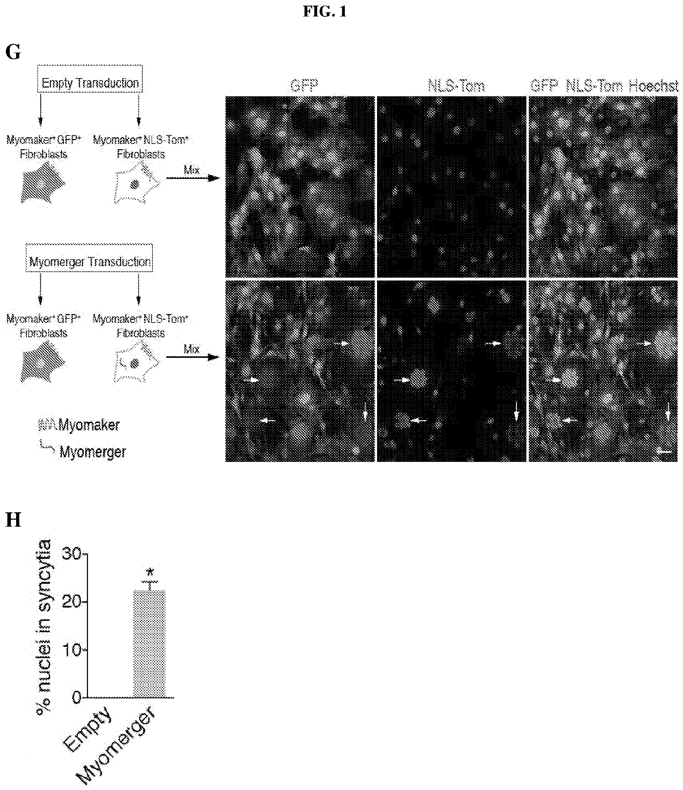

**FIG. 1****: Induction of fibroblast fusion by myomerger.** (A) Expression of MyoD-regulated genes in myomaker⁺ fibroblasts. qRT-PCR analysis for the indicated genes 72 hours after expression in fibroblasts. For *Gm7325,* we used primers specific for the long transcript. (B) Schematic showing a functional assay to screen for muscle genes that could activate fusion of GFP⁺ myomaker⁺ fibroblasts. Representative images of GFP⁺ cells and nuclei after expression of the indicated genes. Arrows depict cells with multiple nuclei. (C) Diagram showing the *Gm7325* locus on chromosome 17. The short transcript is generated by splicing of exon 1 (non-coding) with exon 3, leading to an 84 amino acid protein. The long transcript is produced by splicing of exon 2 with exon 3 and results in a 108 amino acid protein. (D) UCSC genome browser track showing multiple transcripts and conservation across vertebrate species. The short transcript is highly conserved in multiple species, including human, but not present in zebrafish. The upstream exon that produces the longer transcript is not highly conserved. Note that this annotation displays the gene on the reverse strand. (E) The short (S) or long (L) myomerger transcripts were expressed in myomaker⁺ 10T ½ fibroblasts and both induced fusion (n=3). (F) Myomerger also induced fusion of myomaker⁺ NIH/3T3 fibroblasts (n=3) and myomaker⁺ mesenchymal stromal cells (n=3). (G) Illustration of cell mixing approach to show fusion between the populations of fibroblasts. Co-localization of GFP and NLS-TdTomato (NLS-Tom) in the nucleus represents fusion. Representative images demonstrate fusion of myomaker⁺ myomerger⁺ fibroblasts but not empty-infected myomaker⁺ fibroblasts. Arrows indicate fusion between GFP⁺ and NLS-Tom⁺ fibroblasts. (H) The percentage of nuclei in syncytia after expression of empty or myomerger (n=3). Data are presented as mean ± SEM. **P*<0.05 compared to empty using an unpaired t-test. Arrows indicate fusion. Scale bars, 50 µm.

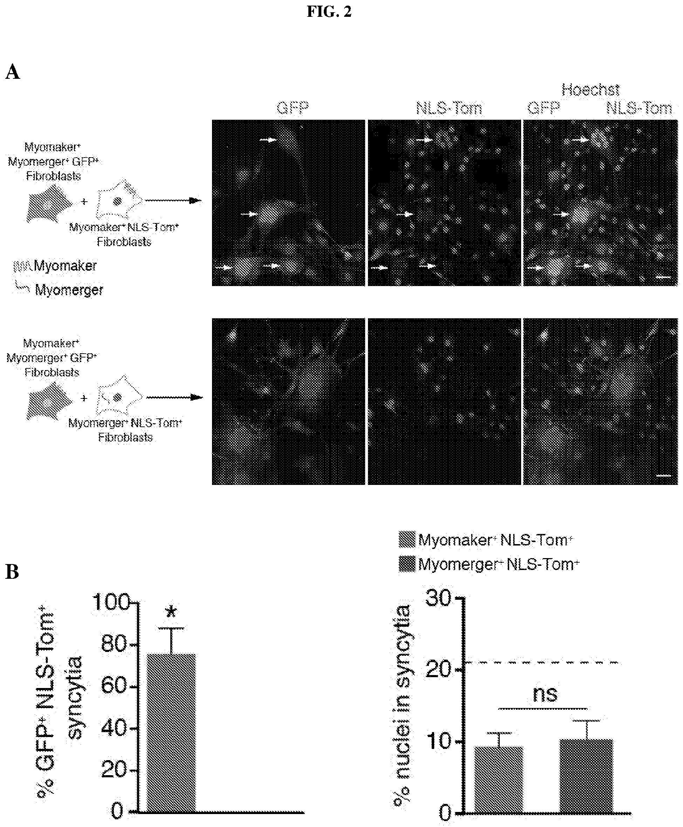

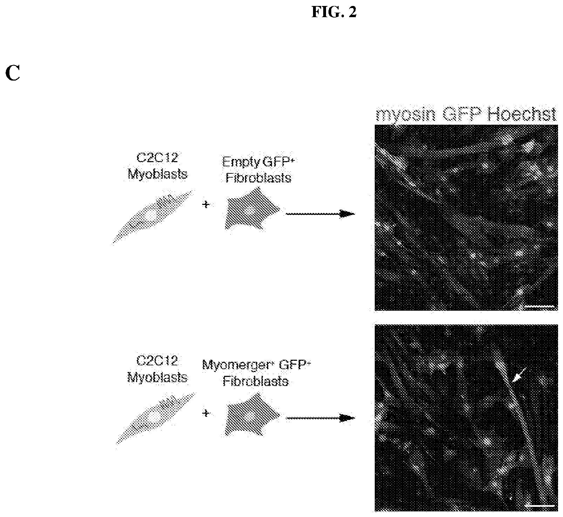

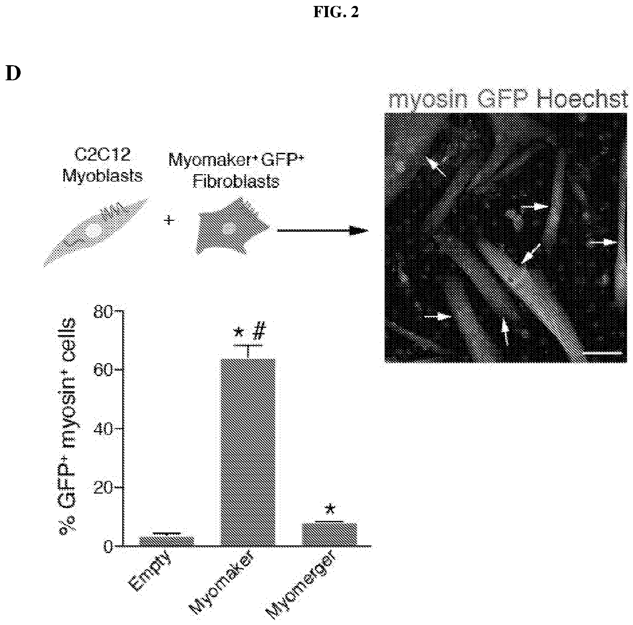

**FIG. 2****: Role of myomerger and myomaker in cell fusion.** (A) Diagram showing the cell mixing approach to assess fusion between the populations of fibroblasts. Co-localization of GFP and NLS-TdTomato (NLS-Tom) in the nucleus represents fusion (arrows). Representative images demonstrate fusion of myomaker⁺ myomerger⁺ GFP⁺ fibroblasts with myomaker⁺ NLS-Tom⁺ fibroblasts but not myomerger⁺ NLS-Tom⁺ fibroblasts. (B) Quantification of the percent of GFP⁺ NLS-Tom⁺ syncytia and the percent of nuclei in syncytia (n=3). Dotted line on right panel represents fusion achieved when both cells express both myomaker and myomerger (from FIG. 1B). (C & D) Heterologous fusion experiment between C2C12 myoblasts and GFP⁺ fibroblasts infected with either empty, myomaker, or myomerger. Representative immunofluorescent images to visualize co-localization of myosin and GFP (arrows), indicating fusion. Quantification of the percentage of GFP⁺ myosin⁺ cells (n=3). Data are presented as mean ± SEM. *P<0.05 compared to myomerger⁺ NLS-Tom⁺fibroblasts in (B) or empty in (C). ^{#}*P*<0.05 between myomaker and myomerger. An unpaired t-test was used to determine significance. Scale bars, 50 µm (A), 100 µm (C).

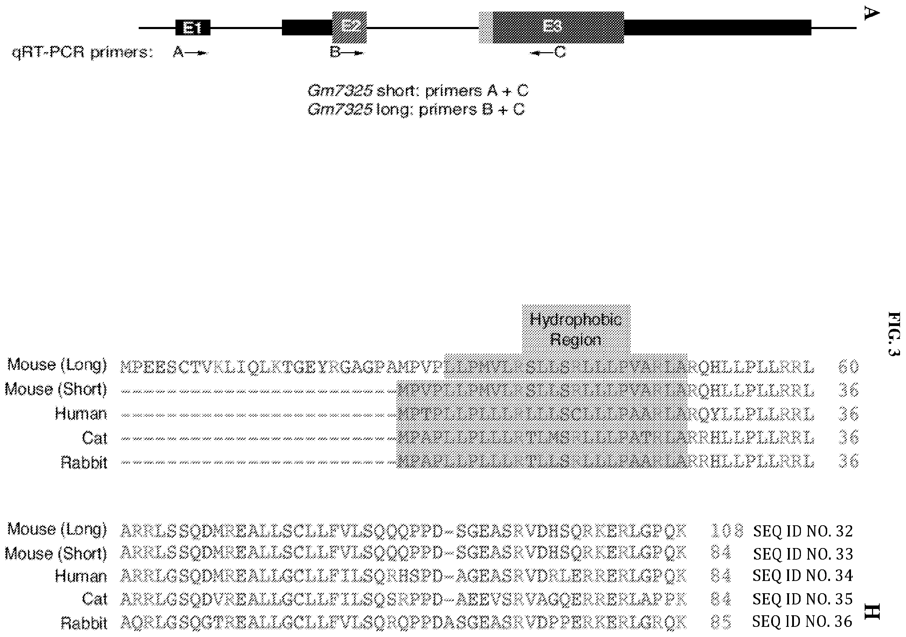

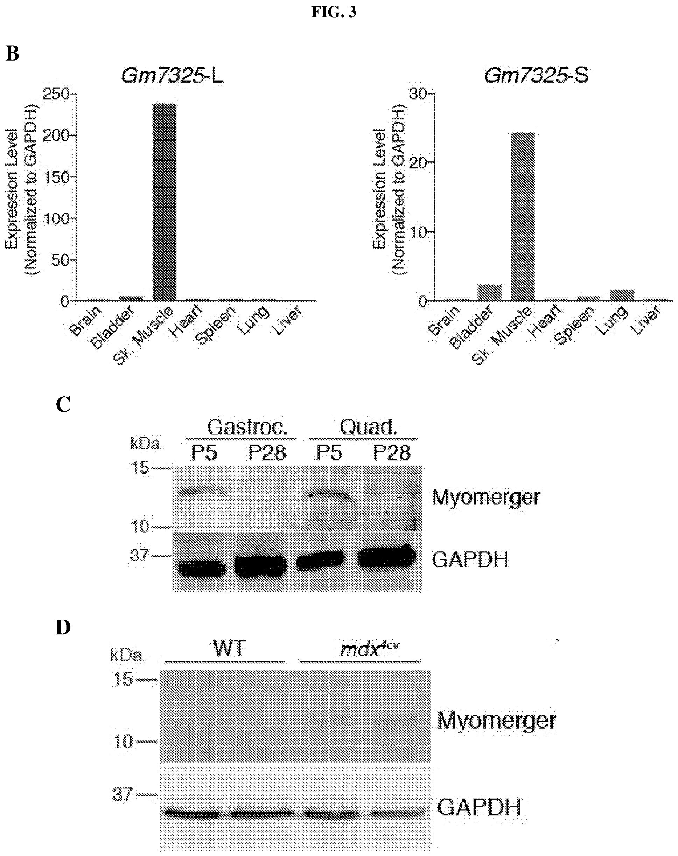

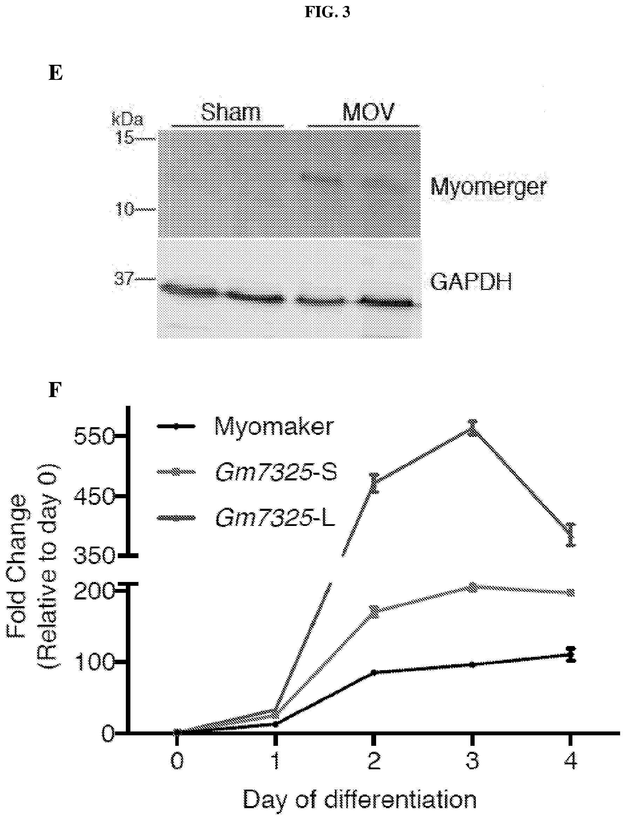

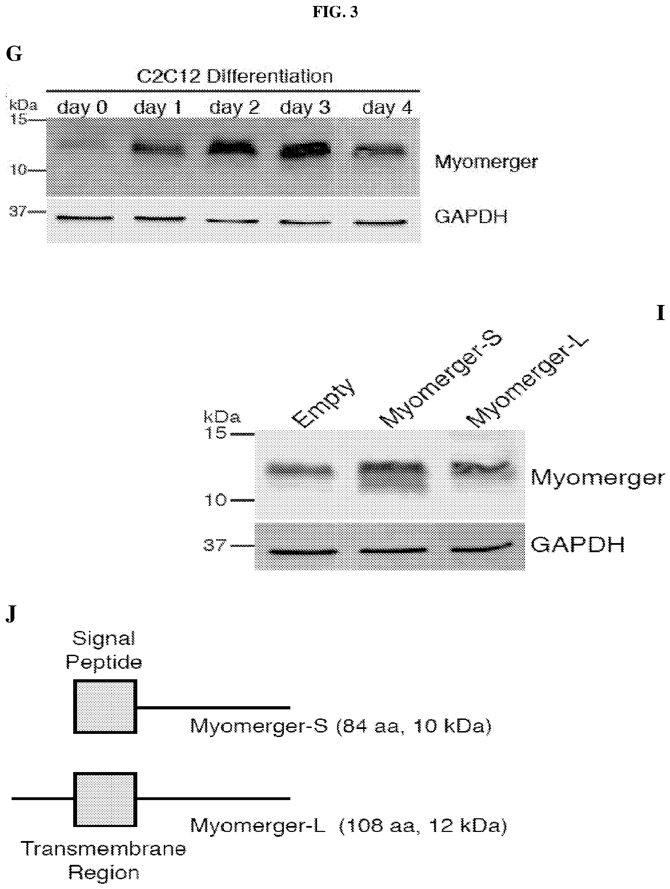

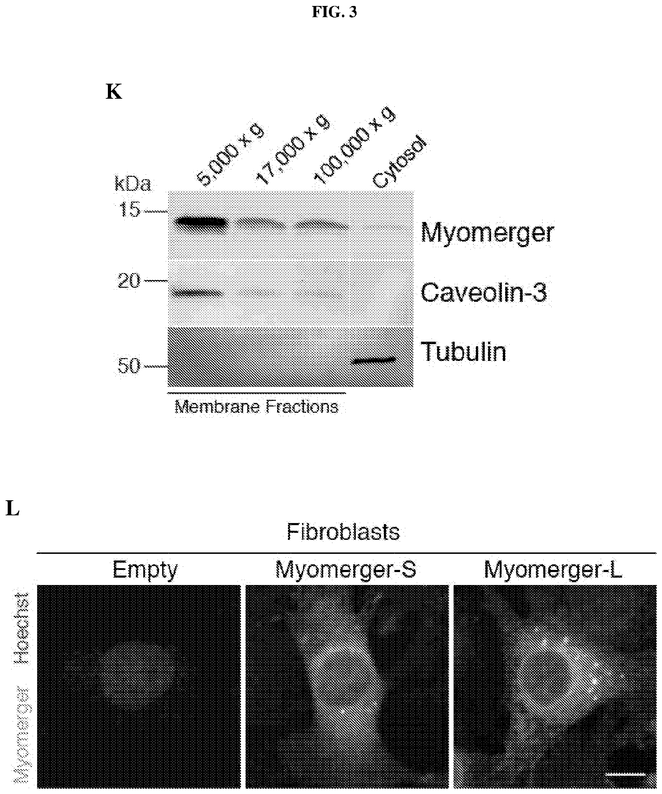

**FIG. 3****: Design of qRT-PCR primers and comparison of myomerger protein variants & Muscle-specific expression and regulation of myomerger.** (A) Schematic showing the location of primers to distinguish short and long transcripts. (B) qRT-PCR for both *Gm7325* long (L) and short (S) transcripts from various postnatal (P) day 5 tissues. (C) Immunoblotting for myomerger comparing P5 muscle to P28 muscle. (D) Immunoblotting for myomerger comparing WT to *mdx^{4cv}* diaphragms (8 weeks of age). (E) Immunoblotting for myomerger comparing sham plantaris to mechanically overloaded (MOV) plantaris (3 months of age). (F) qRT-PCR for *Gm7325* transcript variants and myomaker in C2C12 cells on the indicated days of differentiation (n=3 for each time point). (G) Immunoblotting for myomerger during C2C12 myoblast differentiation. GAPDH was used as a loading control. (H) Sequence alignment of both mouse myomerger protein products with multiple mammalian orthologs using Clustal Omega. A potential hydrophobic region is highlighted in gray. (I) Immunoblotting from C2C12 cells infected with either empty, myomerger-short (S), myomerger-long (L) on day 2 of differentiation. Myomerger migrates as a single band around 12 kDa when endogenously produced (empty). Over-expression of myomerger-S leads to an increase in the endogenous band and a lower band is also detected suggesting that myomerger transcripts may be subjected to intricate mRNA processing or post-translational modifications. (J) Graphic showing the regions of myomerger-S and myomerger-L as predicted by SignalP and Phobius. (K) Fractionation of C2C12 lysates on day 2 of differentiation followed by immunoblotting. (L) Representative immunostaining of fibroblasts infected with either empty, myomerger-short (S), or myomerger-long (L). Scale bar, 10 µm.

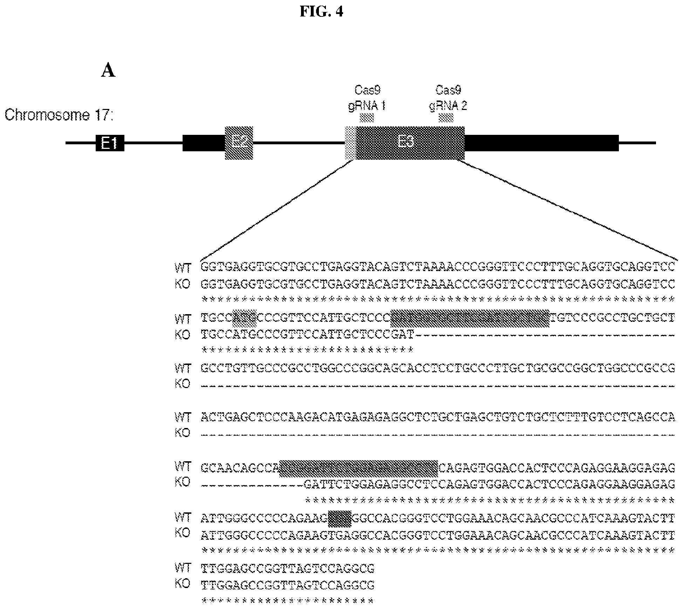

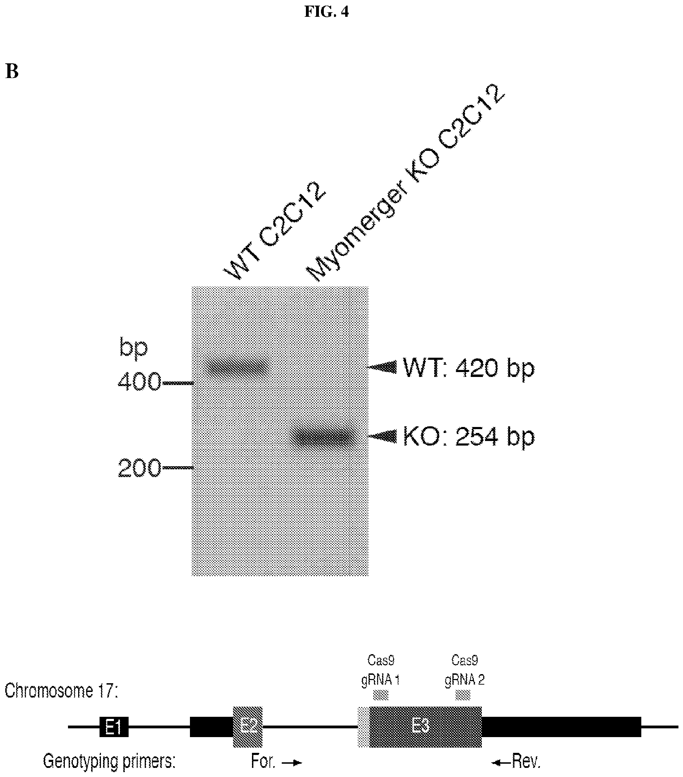

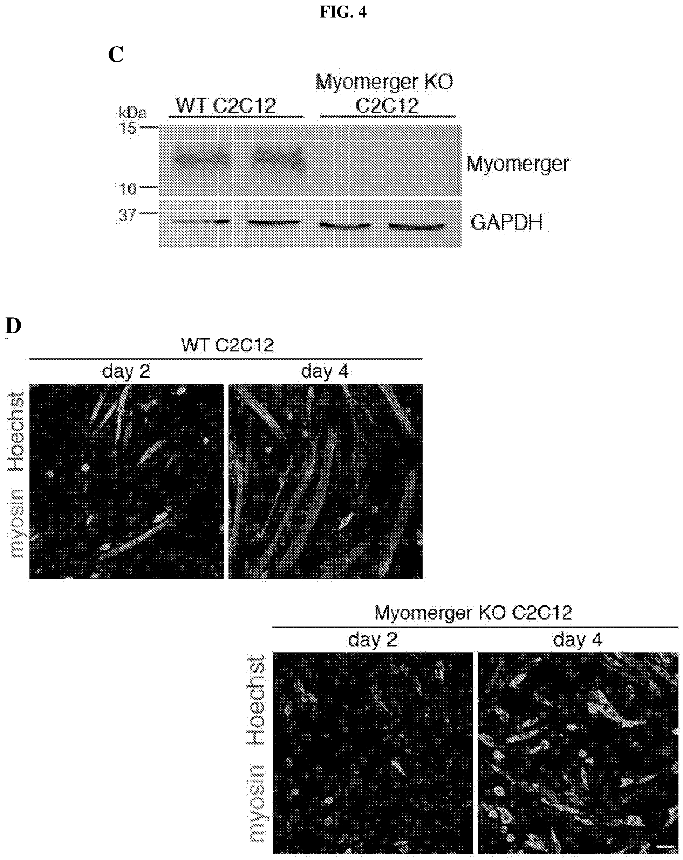

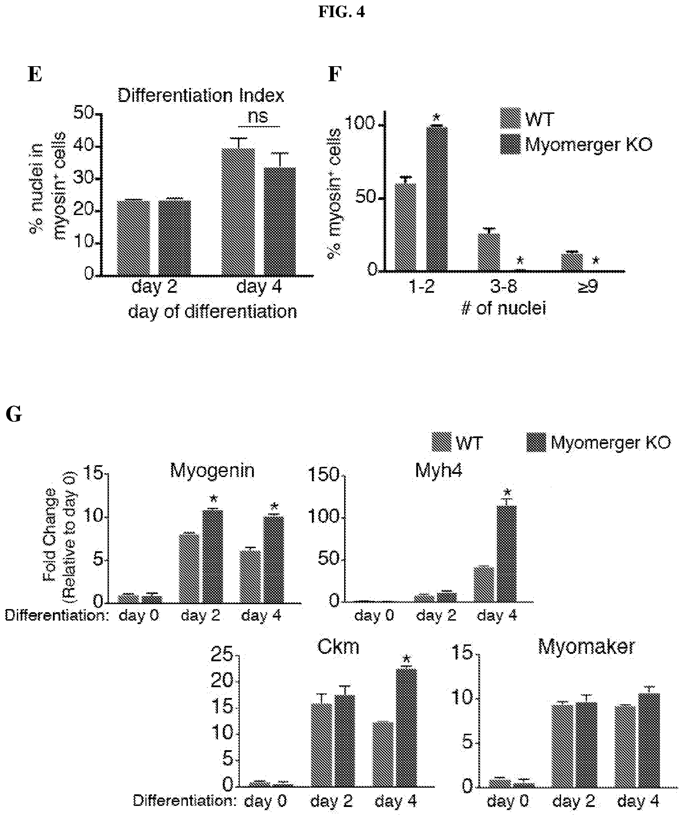

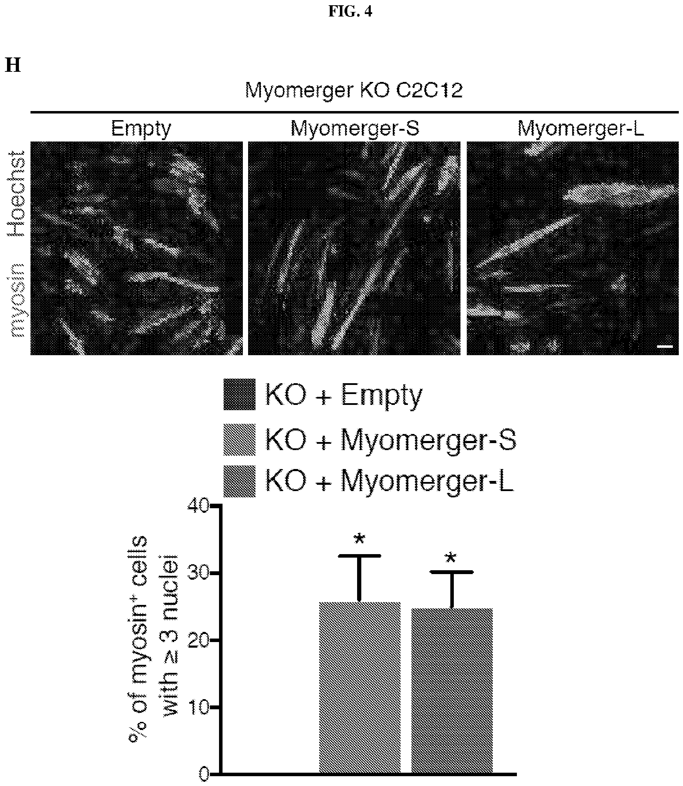

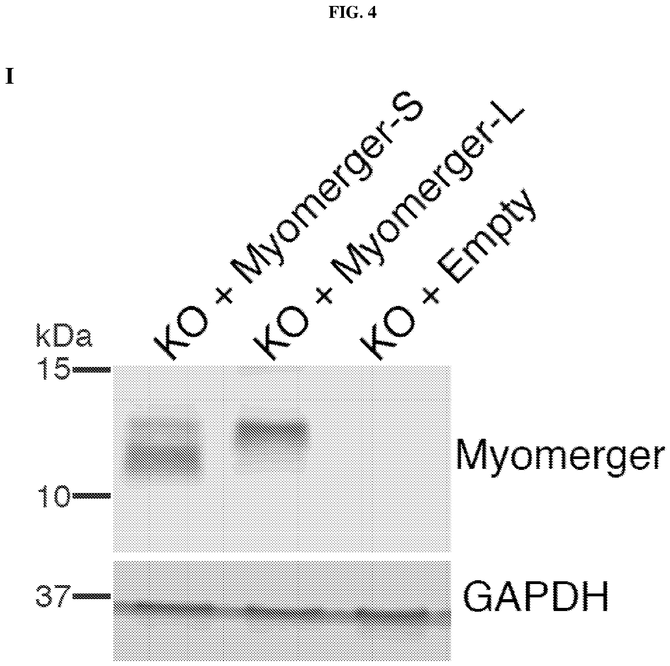

**FIG. 4****: CRISPR/Cas9 disruption of the *Gm7325* locus & Role of myomerger in myoblast fusion *in vitro.*** (A) Schematic showing the *Gm7325* locus and targeting of sgRNAs. (B) Genotyping strategy for myomerger KO C2C12 cells. WT and KO PCR products were sequenced and the result is shown in (A). The use of two sgRNAs results in reproducible cut sites leading to a 166 base pair deletion in both C2C12 cells and mice. The translational start site (ATG, green) for myomerger-S and stop site (TGA, red) for both myomerger-S and myomerger-L are noted. (C) Immunoblotting for myomerger in WT and myomerger KO C2C 12 cells on day 2 of differentiation. GAPDH was used as a loading control. (D) Representative immunofluorescence images on day 2 and day 4 of differentiation for WT and myomerger KO C2C12 cells. Myomerger KO cells differentiate but fail to fuse. (E) Quantification of the differentiation index, the percentage of nuclei in myosin⁺ cells (*n*=4). ns, not significant. (F) The percentage of myosin⁺ cells that contain 1-2, 3-8, or ≥9 nuclei after 4 days of differentiation, as an indicator of fusogenicity (*n*=3). (G) qRT-PCR for the indicated myogenic transcripts (*n*=4). (H) Myomerger KO C2C12 cells were infected with either empty, myomerger-S, or myomerger-L and induced to differentiate. Both myomerger-S and myomerger-L rescued the lack of fusion in myomerger KO cells. Quantification of the fusion index, calculated as the percentage of myosin⁺ cells with ≥ 3 nuclei. Data are presented as mean ± SEM. **P*<0.05 compared to Empty using an unpaired t-test. (I) Immunoblotting for myomerger shows appropriate expression after transduction of myomerger KO cells. Data are presented as mean ± SEM. **P*<0.05 compared to WT using an unpaired t-test. Scale bar, 50 µm.

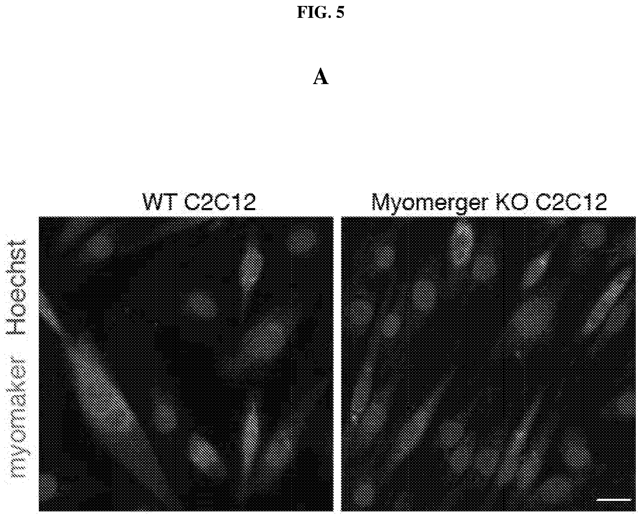

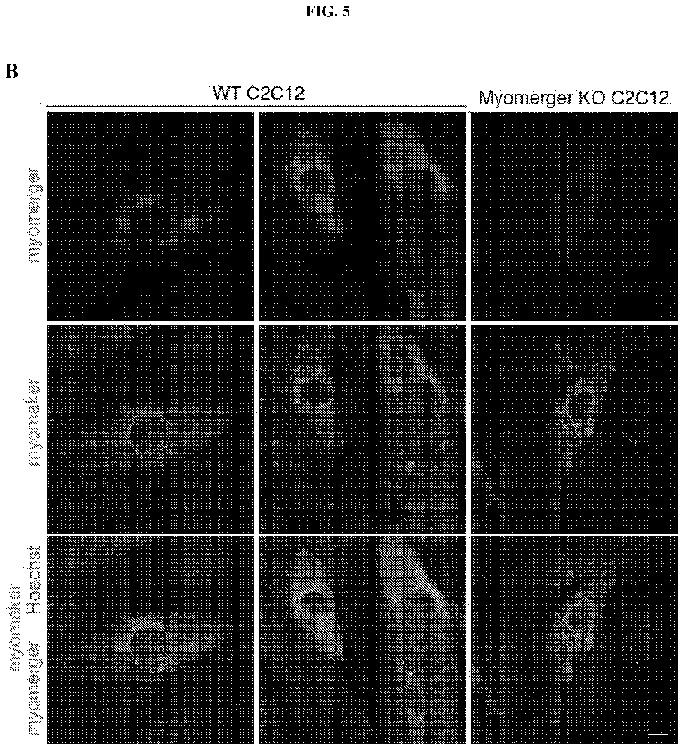

**FIG. 5****: Analysis of myomaker and myomerger co-localization.** (A) Representative immunofluorescence images from WT and myomerger KO C2C12 cells on day 2 of differentiation indicating that loss of myomerger does not alter myomaker expression or localization. (B) Immunofluorescence for myomerger and myomaker on the indicated cells on day 2 of differentiation. These two fusion proteins exhibit different localization patterns. Scale bars, 10 µm A, 5 µm B.

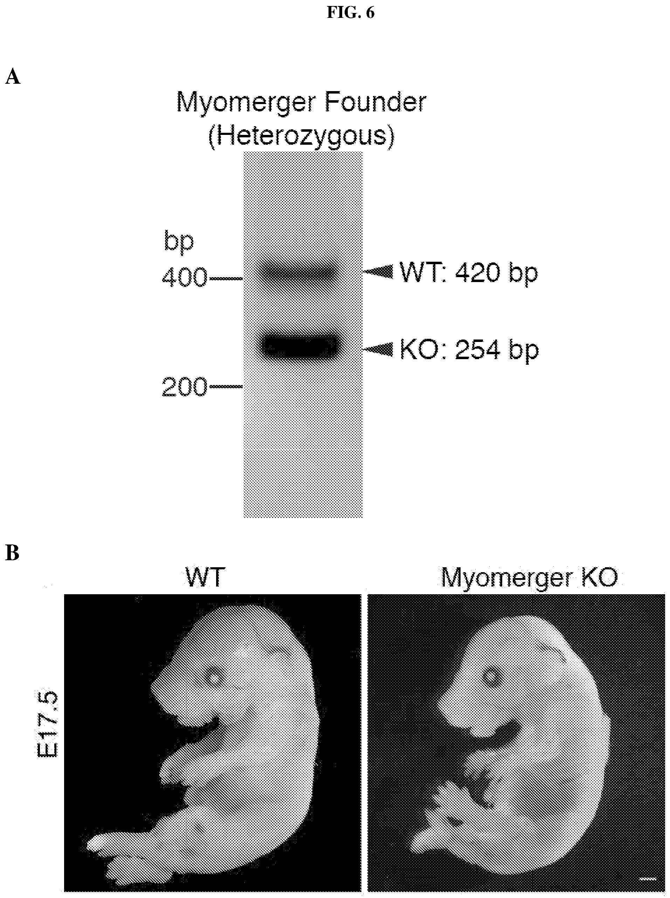

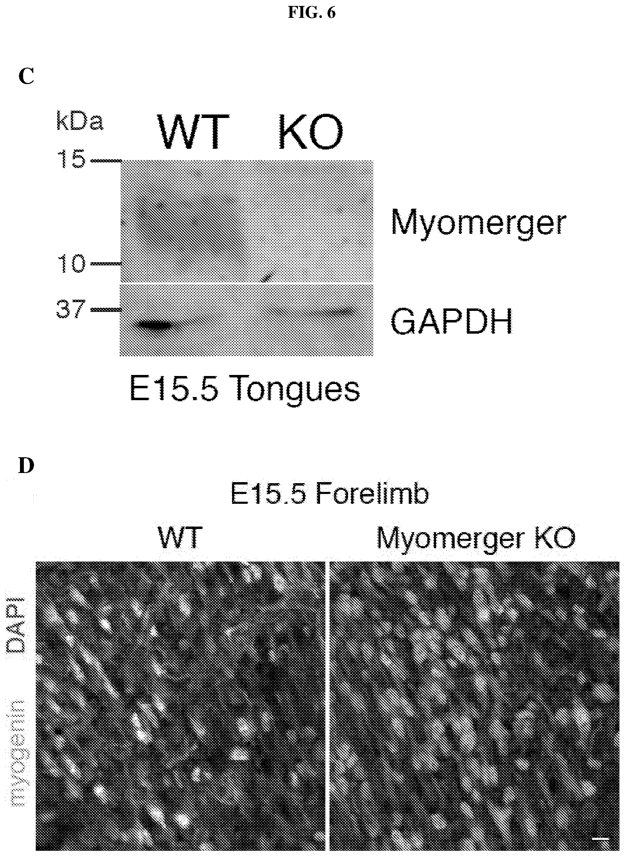

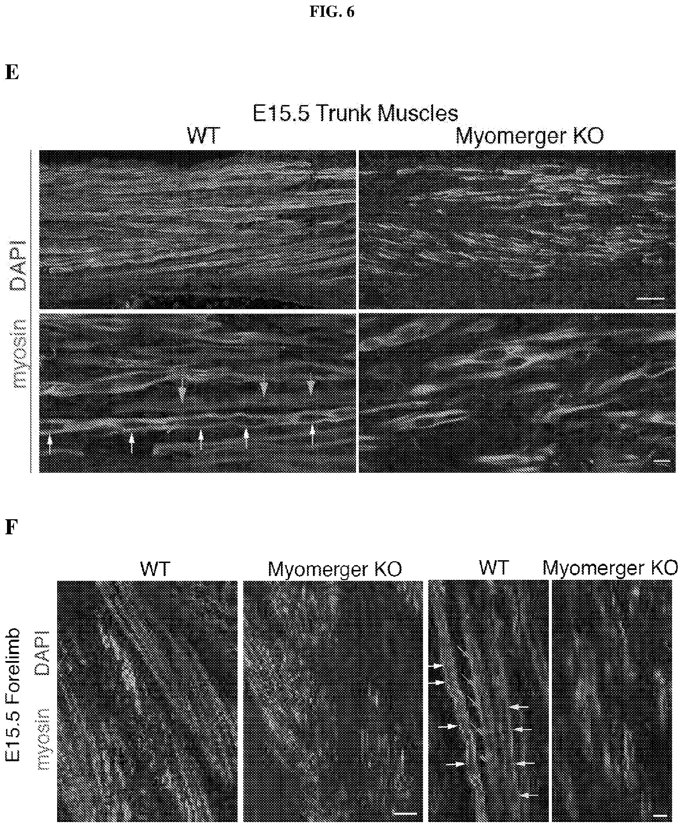

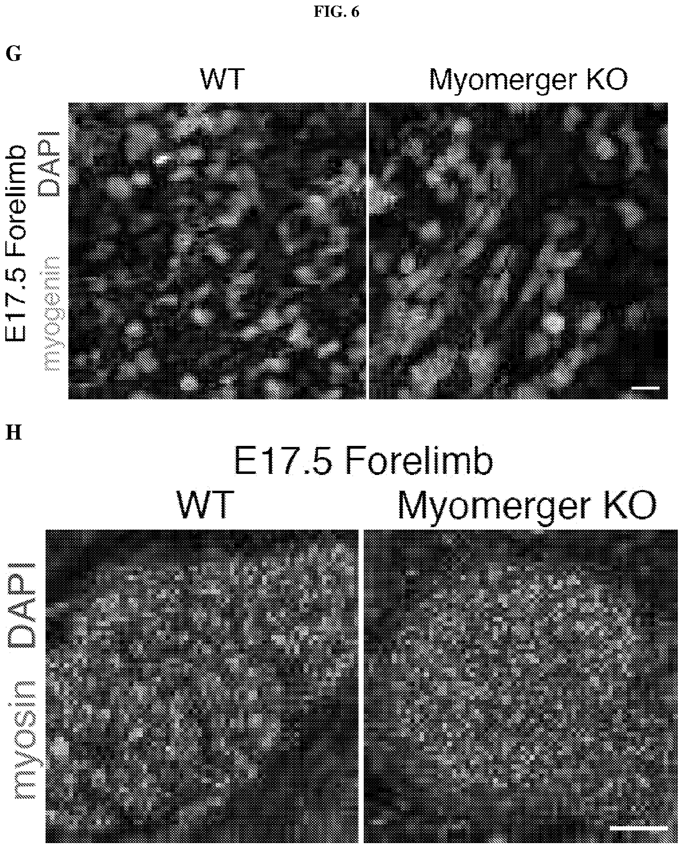

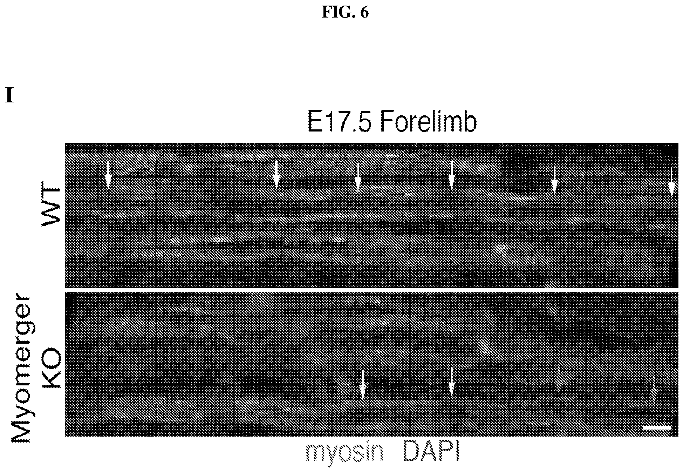

**Fig. 6****: Examination of myomerger KO muscle & Role of myomerger in myoblast fusion and muscle formation during embryonic development.** (A) Genotyping of the one founder harboring the *Gm7325* mutation generated through Cas9-mutagensis. (B) Representative whole-mount images of WT and myomerger KO E17.5 embryos showing improper skeletal muscle formation in KO embryos (*n*=4). (C) Immunoblotting on tongue lysates from WT and myomerger KO mice showing lack of myomerger in KO samples. GAPDH was used as a loading control. (D) Immunofluorescence images for myogenin from WT and myomerger KO E15.5 forelimbs demonstrating that myomerger does not appear to be required for myogenic activation (*n*=3). (E) Myosin immunofluorescence on the indicated E15.5 trunk muscles (*n*=3). Multi-nucleated myofibers (arrows of same color show nuclei within one myofiber) were observed in WT sections. Myomerger KO myocytes were myosin⁺ with sarcomeres but remained mono-nucleated. (F) E15.5 forelimbs (n=3) immunostained with a myosin antibody demonstrates that myomerger KO myoblasts differentiate but are unable to fuse. Arrows of same color show nuclei within same fiber (G, H & I) E17.5 forelimbs from WT and myomerger KO mice were evaluated for myogenin and myosin expression, and multi-nucleation. Arrows of same color in (I) show nuclei within one myofiber. We observed myocytes in myomerger KO samples that contained two nuclei (arrows). The nuclei labeled by the yellow and pink arrows are within different myofibers. Scale bars - 1 mm: B; 50 µm: E top panels, F left panels, H; 10 µm: D, E bottom panels, F right panels, G, I.

### DETAILED DESCRIPTION

While embodiments encompassing the general inventive concepts may take diverse forms, various embodiments will be described herein, with the understanding that the present disclosure is to be considered merely exemplary.

The embodiments of the methods according to the invention include polypeptides comprising a myomerger polypeptide. Other embodiments of methods methods according to the according to the the invention include myomerger nucleic acid molecules encoding polypeptides comprising a myomerger polypeptide.

### Polypeptides, Nucleic Acid Molecules, and Compositions

The myomerger polypeptide can be defined as a polypeptide that (a) induces fusogenicity (e.g., by inducing the fusion of myomaker-expressing fibroblasts), (b) can confer fusogenic activity to normally non-fusogenic cells, (c) is expressed during developmental myogenesis, (d) is expressed during regenerative myogenesis, (e) is expressed only during developmental myogenesis, (f) is expressed only during regenerative myogenesis, or (g) combinations thereof. The term "myomerger polypeptide" encompasses "wt-myomerger polypeptides" (i.e., myomerger polypeptides found in nature without any purposely human-made modification) and "mutant myomerger polypeptides" (e.g., with one or more modifications made to a wt-myomerger polypeptide). Nonlimiting examples of wt-myomerger polypeptides are found in Table 1A. In other examples, the myomerger polypeptide has at least one amino acid modification relative to a wt-myomerger polypeptide. A wt-myomerger polypeptide can, in some examples, be a myomerger polypeptide from any animal including but not limited to a rabbit, a dog, a pig, or an elephant. Table 1A provides nonlimiting examples of wt-myomerger polypeptides, whereby SEQ ID NOs.: 32 - 35 do not form part of the invention. Tables 1B and 1C provide nonlimiting examples of related nucleic acid sequences (including start and stop codons), whereby SEQ ID NOs.: 39 - 42 and SEQ ID NOs.: 46 - 49 do not form part of the invention.

**Table 1A**

| **Source** | **Polypeptide sequence** |
|---|---|
| Mouse (long) | MPEESCTVKLIQLKTGEYRGAGPAMPVPLLPMVLRSLLSRLLLPVAR LARQHLLPLLRRLARRLSSQDMREALLSCLLFVLSQQPPDSGEASR |
| | VDHSQRKERLGPQK (SEQ ID NO: 32) |
| Mouse (short) | MPVPLLPMVLRSLLSRLLLPVARLARQHLLPLLRRLARRLSSQDMRE ALLSCLLFVLSQQQPPDSGEASRVDHSQRKERLGPQK (SEQ ID NO: 33) |
| Human | MPTPLLPLLLRLLLSCLLLPAARLARQYLLPLLRRLARRLGSQDMRE ALLGCLLFILSQRHSPDAGEASRVDRLERRERLGPQK (SEQ ID NO: 34) |
| Cat | MPAPLLPLLLRTLMSRLLLPATRLARRHLLPLLRRLARRLGSQDVRE ALLGCLLFILSQSRPPDAEEVSRVAGQERRERLAPPK (SEQ ID NO: 35) |
| Rabbit | MPAPLLPLLLRTLLSRLLLPAARLARRHLLPLLRRLAQRLGSQGTREA LLGCLLFVLSQRQPPDASGEASRVDPPERKERLGRQK (SEQ ID NO: 36) |
| Dog | MPAPLLPLLLRTLVSRLLLPAARLARRHLLPLLRGLARRLGSQEVRE ALLGCLLFILSQRHPPDAEEASRVAGQERKERLAPPK (SEQ ID NO: 37) |
| Elephant | MPVPLLSLLLRALLSRLLLPAARLARQHLLPLLRRLARRLGSQDMRQ ALLGCLLFVLSQQHPPDAGEASREALSERRGRLAPQK (SEQ ID NO: 38) |

**Table 1B**

| **Source** | **cDNA nucleic acid sequence** |
|---|---|
| Mouse (long) | atgcc agaagaaagc tgcactgtaa aactaatcca gttgaaaact ggggagtaca gaggtgcagg tcctgccatg cccgttccat tgctcccgat ggtgcttcga tcgctgctgt cccgcctgct gctgcctgtt gcccgcctgg cccggcagca cctcctgccc ttgctgcgcc ggctggcccg ccgactgagc tcccaagaca tgagagaggc tctgctgagc tgtctgctct ttgtcctcag ccagcaacag ccaccggatt ctggagaggc ctccagagtg gaccactccc agaggaagga gagattgggc ccccagaagt ga (SEQ ID NO: 39) |
| Mouse (short) | atgcccg ttccattgct cccgatggtg cttcgatcgc tgctgtcccg cctgctgctg cctgttgccc gcctggcccg gcagcacctc ctgcccttgc tgcgccggct ggcccgccga ctgagctccc aagacatgag agaggctctg ctgagctgtc tgctctttgt cctcagccag caacagccac cggattctgg agaggcctcc agagtggacc actcccagag gaaggagaga ttgggccccc agaagtga (SEQ ID NO: 40) |
| Human | atgcccac gccactgctc ccgctgctgc ttcgattgct gctgtcctgc ctgctgctgc ctgctgcccg cctggcccgc caatacctcc tgcccctgct gcgccgattg gcccgccgcc tgggctccca ggacatgcga gaggctttgc tgggctgtct gctgttcatt ctcagccagc gacactcgcc agacgctggg gaggcctcaa gagtggaccg cctggagagg agggagaggt taggccccca aaagtga (SEQ ID NO: 41) |
| Cat | atgcccgc tccactgctc ccactgctgc ttcgaaccct gatgtcccgc ttgctgctgc ctgccacccg cctggcccgc cggcacctcc tgcccctcct gcgccgactg gcccgccgcc tgggctcgca ggatgttcga gaagctttgc tgggctgtct gttgttcatc ctcagccaga |
| | gccgcccgcc cgacgctgag gaggtctcca gagtggctgg ccaggagagg agggagaggc tagctccccc aaaatga (SEQ ID NO: 42) |
| Rabbit | atgcc tgcccccctg ctgccgctgc tgctgcgaac gctgctgtcc cgtctgctgc tgcccgctgc ccgcctggcc cgccggcacc tcctgcccct gctgcgccga ctggctcaac gcctgggctc ccagggcacg cgcgaggctt tgctgggctg tttgctgttt gtcctcagcc agagacagcc gccagatgcc tctggggagg cctccagagt ggacccaccg gagaggaagg agaggttagg ccgccaaaag tga (SEQ ID NO: 43) |
| Dog | atgc ctgctccact gctcccactg ctgctgcgaa cgctggtgtc tcgcctgctg ctgcctgctg cccgcctggc ccggcggcac ctcctgcccc tgctgcgtgg actggcccgc cgcctaggct cgcaggaggt tcgagaggct ttgctgggct gtctgttgtt catcctcagc cagagacatc cgccggacgc cgaggaggcc tccagagtgg ctggccagga gaggaaggag aggctagctc cccccaaatg a (SEQ ID NO: 44) |
| Elephant | atgcccgtcc cgctgctctc gctgctgctg cgcgcgctgc tgtcccgcct gctgctgcct gctgcccgcc tggcccgcca gcacctcctg cccctcctgc gccgacttgc tcgccgcctg ggctcccagg acatgcgaca ggctctcttg ggatgtctgc tctttgtcct cagccagcaa cacccgccgg acgctggtga ggcctccaga gaggccctct cagagaggag agggaggcta gccccccaaa agtga (SEQ ID NO: 45) |

**Table 1C (exons in lowercase)**

| **Source** | **Genomic nucleic acid sequence** |
|---|---|
| Human (+ strand) - start codon is bold & underlined; stop codon is bold and italicized | ctgcccggtgagagctgccgtggattggtggggGTAGGGACTGAGAGGTCAGGGAGT GT CAGGTCAGGGTGGATCAGGAGCCCCAAAAGAAAAATTGAGAATTGCCTGGAGAAGAAC TC CTGCTAGACTGAGGGAGAAGGGTTAGGGAACTCCAGGGGCATTGAGGCTGTGCAAGAG GA GGGGGTGACTAGAGGAAGGGAGGGGCCAGGGAGCAGTAGGAATGCCTGGAGCTGGGAA CG GCAAGCTGTAGGTCTTGGTTTACTCTTGCCTTGGTTCAGTCTCCCCATCTGTGCTATG GT GAGAACCTTCCTGCCTCAGCTGCCTTGCCAAGAGAAAGGCTTCATGAAAGCAAAAAT GA CCTACAAATTGAGGTCAGGAGCAGGAAGGTGTAAACTGAAGGGAGGGGGAACTCCTGC CC ACCCCATGTCCTTGCCAGGTGAGGCAGAACCAGGACATGCAAGCCTAAAGTCTGTGTT GT CTTCCCAGgcactgactcactggccctgccatgcccacgccactgctcccgctgctgc tt cgattgctgctgtcctgcctgctgctgcctgctgcccgcctggcccgccaatacctcc tg cccctgctgcgccgattggcccgccgcctgggctcccaggacatgcgagaggctttgc tg ggctgtctgctgttcattctcagccagcgacactcgccagacgctggggaggcctcaa ga gtggaccgcctggagaggagggagaggttaggcccccaaaagtgaggccacaagtcct gg cagcagctgtatccacaaaatgctttcttttggagtaggataatcctggcaccagcac tg accgaagcctgcccagtggacagaagatatagtgagggttgtgcatgagaggatctg cc |
| | acagacatgcctctccactcccaacagaaatgtctttctggaagaatgccttgcatct ag cacaaaactgattattgcccctctgtcctccagcagttcctcccaaagaccactccta at cacctctggcctcaggcgggaggggaactaacacccacccacccctgccctccctgca aa tgggaacatcaaggttcccagtgcttaactgagggacaagtgacaatttagcagagag gc aagatttgaatccagactgtcttccagactcaggacctaccttaaaataatatctgag tt gcttatggaggcagacctgcctgcaaagcccagcactcagcaagtgctcaataaatat tt gatttgaattctttc (SEQ ID NO: 46) |
| Human (- strand, reverse complement) | gaaagaattcaaatcaaatatttattgagcacttgctgagtgctgggctttgcaggca gg tctgcctccataagcaactcagatattattttaaggtaggtcctgagtctggaagaca gt ctggattcaaatcttgcctctctgctaaattgtcacttgtccctcagttaagcactgg ga accttgatgttcccatttgcagggagggcaggggtgggtgggtgttagttcccctccc gc ctgaggccagaggtgattaggagtggtctttgggaggaactgctggaggacagagggg ca ataatcagttttgtgctagatgcaaggcattcttccagaaagacatttctgttgggag tg gagaggcatgtctgtggcagatccctctcatgcacaaccctcactatatcttctgtcc ac tgggcaggcttcggtcagtgctggtgccaggattatcctactccaaaagaaagcattt tg tggatacagctgctgccaggacttgtggcctcacttttgggggcctaacctctccctc ct ctccaggcggtccactcttgaggcctccccagcgtctggcgagtgtcgctggctgaga at gaacagcagacagcccagcaaagcctctcgcatgtcctgggagcccaggcggcgggcc aa tcggcgcagcaggggcaggaggtattggcgggccaggcgggcagcaggcagcagcagg ca ggacagcagcaatcgaagcagcagcgggagcagtggcgtgggcatggcagggccagtg ag tcagtgcCTGGGAAGACAACACAGACTTTAGGCTTGCATGTCCTGGTTCTGCCTCACC TG GCAAGGACATGGGGTGGGCAGGAGTTCCCCCTCCCTTCAGTTTACACCTTCCTGCTCC TG ACCTCAATTTGTAGGTCATTTTTGCTTTCATGAAGCCCTTTCTCTTGGCAAGGCAGCT GA GGCAGGAAGGTTCTCACCATAGCACAGATGGGGAGACTGAACCAAGGCAAGAGTAAAC CA AGACCTACAGCTTGCCGTTCCCAGCTCCAGGCATTCCTACTGCTCCCTGGCCCCTCCC TT CCTCTAGTCACCCCCTCCTCTTGCACAGCCTCAATGCCCCTGGAGTTCCCTAACCCTT CT CCCTCAGTCTAGCAGGAGTTCTTCTCCAGGCAATTCTCAATTTTTCTTTTGGGGCTCC TG ATCCACCCTGACCTGACACTCCCTGACCTCTCAGTCCCCTACccccaccaatccacgg ca gctctcaccgggcag (SEQ ID NO: 47) |
| Mouse (+ strand) | ccaataacaacacactgtcctcgtttattgactacctgctgcgtaccaagctttgaaa gt actcattctttaacgggaagcaagggcttataattttaaggtagacggacagtttgg at |
| | ttaaataccacctcttagctaaattgtcttgagtctaagtgaaacatcatctcttaac tg accttgatacccgcatttgcaggtccaccctggaggccagagataaggcagagggagc tg cagagaggaagggtcaatcaacacaatctgtagcctgctaggagctaggggagtggga ac tgttcaggtcagagccctcttgcactcagcccggactgtcttcgcccactgggcagtc tg ccgtccatgcccgtgcgtgcggaccgacgcctggactaaccggctccaaaagtacttt ga tgggcgttgctgtttccaggacccgtggcctcacttctgggggcccaatctctccttc ct ctgggagtggtccactctggaggcctctccagaatccggtggctgttgctggctgagg ac aaagagcagacagctcagcagagcctctctcatgtcttgggagctcagtcggcgggcc ag ccggcgcagcaagggcaggaggtgctgccgggccaggcgggcaacaggcagcagcagg cg ggacagcagcgatcgaagcaccatcgggagcaatggaacgggcatggcaggacctgca cC TGCAAAGGGAACCCGGGTTTTAGACTGTACCTCAGGCACGCACCTCACCTGGCAAAGC AG GGTGCGGGGGTGTGGAGTCCTCCCTTCAGCTTATACctctgtactccccagttttcaa ct ggattagttttacagtgcagctttcttctggcatgaaagctggttaaggagttcactc ac tgttatcacagatgggaagggagcccagggctggaaggtggtggggactGAGGCTAGG GC CTTTTCCAGAACCCACTTCCTTTAATCCCTCCCTCCCTTTGCATACTCTGACctgaag cc tgaacttcttgccctcctgctcaccagttctaaccggccagtggcagctctcaccagt ca gaactgctcagaatcaatttcaggatgcttttgcctgcggtggattcagcatcact (SEQ ID NO: 48) |
| Mouse (-strand, reverse complement) - start codon is bold & underlined; stop codon is bold and italicized | agtgatgctgaatccaccgcaggcaaaagcatcctgaaattgattctgagcagttctg ac tggtgagagctgccactggccggttagaactggtgagcaggagggcaagaagttcagg ct tcagGTCAGAGTATGCAAAGGGAGGGAGGGATTAAAGGAAGTGGGTTCTGGAAAAGGC CC TAGCCTCagtccccaccaccttccagccctgggctcccttcccatctgtgataacagt ga gtgaactccttaaccagctttcatgccagaagaaagctgcactgtaaaactaatccag tt gaaaactggggagtacagagGTATAAGCTGAAGGGAGGACTCCACACCCCCGCACCCT GC TTTGCCAGGTGAGGTGCGTGCCTGAGGTACAGTCTAAAACCCGGGTTCCCTTTGCAGg tg caggtcctgccatgcccgttccattgctcccgatggtgcttcgatcgctgctgtcccg cc tgctgctgcctgttgcccgcctggcccggcagcacctcctgcccttgctgcgccggct gg cccgccgactgagctcccaagacatgagagaggctctgctgagctgtctgctctttgt cc tcagccagcaacagccaccggattctggagaggcctccagagtggaccactcccagag ga aggagagattgggcccccagaagtgaggccacgggtcctggaaacagcaacgcccatc aa agtacttttggagccggttagtccaggcgtcggtccgcacgcacgggcatggacggca ga |
| | ctgcccagtgggcgaagacagtccgggctgagtgcaagagggctctgacctgaacagt tc ccactcccctagctcctagcaggctacagattgtgttgattgacccttcctctctgca gc tccctctgccttatctctggcctccagggtggacctgcaaatgcgggtatcaaggtca gt taagagatgatgtttcacttagactcaagacaatttagctaagaggtggtatttaaat cc aaactgtcccgtctaccttaaaattataagcccttgcttcccgttaaagaatgagtac tt tcaaagcttggtacgcagcaggtagtcaataaacgaggacagtgtgttgttattgg (SEQ ID NO: 49) |

One or more modifications, in some instances, can include an insertion, a deletion, a substitution, or combinations thereof. Exemplarily, one or more modifications to a wt-myomerger polypeptide can comprise an insertion, such, but not limited to an insertion at the C-terminus or at the N-terminus of the wt-myomerger polypeptide. In some examples, an insertion can include (e.g., at the C-terminus, at the N-terminus, or at another place in the polypeptide) about 1, about 2, about 3, about 4, about 5, about 6, about 7, about 8, about 9, about 10, about 11, about 12, about 13, about 14, about 15, about 16, about 17, about 18, about 19, or about 20 amino acids (e.g., natural amino acids, or modified or unusual amino acids).

In some examples, the polypeptide does not encompass one or more naturally occurring polypeptides (e.g., does not encompass one or more of the wt-myomerger polypeptides). In other examples, the polypeptide does not encompass any of the wt-myomerger polypeptides. In some examples, the polypeptide does not encompass any naturally occurring polypeptide (e.g., does not encompass any of the wt-myomerger polypeptides or any other naturally occurring polypeptide).

Exemplarily, one or more modifications to a wt-myomerger polypeptide can include one or more substitutions, one or more insertions, or one or more deletions (or combinations thereof) to one or more amino acids in a hydrophobic region of a wt-myomerger polypeptide, in a signal region of a wt-myomerger polypeptide, in a transmembrane region of a wt-myomerger polypeptide, or in a combination thereof. In some examples, one or more modifications to a wt-myomerger polypeptide can include one or more substitutions or one or more deletions (or combinations thereof) to one or more amino acids in a hydrophobic region of a wt-myomerger polypeptide, in a signal region of a wt-myomerger polypeptide, in a transmembrane region of a wt-myomerger polypeptide, or in a combination thereof.

In some examples, the polypeptide can have a polypeptide sequence with an amino acid sequence identity to a wt-myomerger polypeptide (e.g., SEQ ID NO:36) of about 80%, about 85%, about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 80%, at least about 90%, at least about 95%, at least about 99%, or at least about 99.5%. In some examples, the polypeptide sequence has an amino acid sequence identity to SEQ ID NO:36 of about 80%, about 85%, about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 80%, at least about 90%, at least about 95%, at least about 99%, or at least about 99.5%. The amino acid sequence identity (e.g., percent identity) can be determined by any suitable method, such as using BLAST, BLAST-2, ALIGN, ALIGN-2, Clustal Omega, or Megalign software. Unless otherwise indicated, the amino acid sequence identity (e.g., percent identity) is determined using BLAST-2.

In some examples, the polypeptide has (e.g., as compared to a wt-myomerger polypeptide or as compared to the absence of a myomerger polypeptide) an increased ability to activate fusion, a decreased ability to activate fusion, an increased ability to confer fusogenicity, a decreased ability to confer fusogenicity, an increased level of expression during embryonic development, a decreased level of expression during embryonic development, an increased level of expression during myogenesis in adult organisms (e.g., older than embryonic), a decreased level of expression during myogenesis in adult organisms (e.g., older than embryonic), an increased level of induction of myogenesis in adult organisms (e.g., older than embryonic), a decreased of induction of myogenesis in adult organisms (e.g., older than embryonic), an increased affinity for membranes, a decreased affinity for membranes, an increased level of association with membrane compartment, a decreased level association with membrane compartment, or combinations thereof. In other examples, the polypeptide has (e.g., as compared to a wt-myomerger polypeptide or as compared to the absence of a myomerger polypeptide) an increased ability to activate fusion, an increased ability to confer fusogenicity, an increased level of expression during embryonic development, an increased level of expression during myogenesis in adult organisms (e.g., older than embryonic), an increased level of induction of myogenesis in adult organisms (e.g., older than embryonic), an increased affinity for membranes, an increased level of association with membrane compartment, or combinations thereof.

Some examplesinclude nucleic acid molecules that can encode for the polypeptide ("myomerger nucleic acid molecules"). Examplarily, the myomerger nucleic acid molecule may be included in a vector (e.g., a viral vector, a retroviral vector, a lentiviral vector, an adenoviral vector, an adeno-associated viral vector, a herpesviral vector, a chimeric viral vector, a plasmid, a cosmid, an artificial chromosome, a bacteriophage, an animal virus, a plant virus, an expression vector, a conjugative vector, or a nonconjugative vector). The myomerger nucleic acid molecule is in a first cell, which is a non-muscle cell.

Exemplarily, the myomerger nucleic acid molecule may comprise one or more nucleic acid sequences that are not used to encode for the polypeptide (e.g., one or more introns). For example, the myomerger nucleic acid molecule can comprise a nucleic acid sequence as found in nature (e.g., including introns). The myomerger nucleic acid molecule may differ from the one or more nucleic acid molecules in nature because the myomerger nucleic acid molecule does not include one or more introns. In some embodiments, the myomerger nucleic acid molecule is a cDNA molecule ("myomerger cDNA molecule"). In certain examples, the myomerger cDNA molecule is identical to a nucleic acid molecule found in nature. In other examples, the myomerger cDNA molecule is not identical to a nucleic acid molecule found in nature (e.g., due to the myomerger cDNA molecule not including one or more introns in the nucleic acid molecule found in nature).

The myomerger nucleic acid molecule sequence has a sequence identity to a nucleic acid molecule encoding a wt-myomerger polypeptide (e.g., SEQ ID NO:43) of about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 90%, at least about 95%, at least about 99%, or at least about 99.5%. In some embodiments, the myomerger nucleic acid molecule sequence has a sequence identity to SEQ ID NO:43 of about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 90%, at least about 95%, at least about 99%, or at least about 99.5%. Nonlimiting examples of wt-myomerger polypeptides and wt-myomerger nucleic acid molecules can be found in Table 1. The nucleic acid sequence identity (e.g., percent identity) can be determined by any suitable method, such as using BLAST, BLAST-2, ALIGN, ALIGN-2, Clustal Omega, or Megalign software. Unless otherwise indicated, the nucleic acid sequence identity (e.g., percent identity) is determined using BLAST-2.

In some examples, the myomerger nucleic acid molecule encodes for a polypeptide that has one or more modifications to wt-myomerger polypeptide in a hydrophobic region, in a signal region, in a transmembrane region, or in a combination thereof.

The myomerger nucleic acid molecule can be made using any suitable technique, such as but not limited to, chemical synthesis, enzymatic production or biological production. Chemical synthesis of a nucleic acid molecule can include, for example, a nucleic acid molecule made by *in vitro* chemical synthesis using phosphotriester, phosphite or phosphoramidite chemistry and solid phase techniques, or via deoxynucleoside H-phosphonate intermediates. Enzymatically produced nucleic acid molecules can be accomplished using any suitable method including but not limited to Polymerase Chain Reaction (PCR). Biologically produced nucleic acid molecules can be accomplished using any suitable method including but not limited to a recombinant nucleic acid produced (i.e., replicated) in a living cell, such as a recombinant DNA vector replicated in bacteria.

Modifications or changes made in the structure of the nucleic acid molecules and/or polypeptides are encompassed within some embodiments of the present invention. In certain embodiments, a polypeptide can be modified (e.g., by one or more insertions, one or more deletions, or one or more substitutions (e.g., conservative substitutions)). In some examples, the polypeptide which was modified does not have an appreciable loss (e.g., a decrease in a function of less than about 1%, less than about 5%, less than about 10%, less than about 25%, less than about 50%, less than about 75%, less than about 90%, less than about 95%, less than about 99%, or less than about 100%) of one or more chosen functions of the unmodified polypeptide such as, for example, the ability to form a pore in a cell (e.g., in a cell membrane), the ability to make changes to the cytoskeleton of the cell (e.g., reorganizing the cytoskeleton, rearranging the cytoskeleton, making changes to the cytoskeleton to allow the cell to fuse), the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some examples, the polypeptide which was modified retains desired levels (e.g., at least about 20%, at least about 40%, at least about 50%, at least about 75%, at least about 80%, at least about 90%, at least about 95%, or at least about 99%) of one or more functions of the unmodified polypeptide, such as, for example, the ability to form a pore in a cell (e.g., in a cell membrane), the ability to make changes to the cytoskeleton of the cell (e.g., reorganizing the cytoskeleton, rearranging the cytoskeleton, making changes to the cytoskeleton to allow the cell to fuse), the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some examples, the polypeptide after modification has an increased level of one or more functions as compared to the unmodified polypeptide. Nucleic acid molecules can be designed to encode for such a modified polypeptide.

A "functional polypeptide" is defined as a polypeptide (e.g., a myomerger polypeptide or a modified polypeptide) that has desired levels (e.g., at least about 20%, at least about 40%, at least about 50%, at least about 75%, at least about 80%, at least about 90%, at least about 95%, or at least about 99%, as compared to another polypeptide, such as a naturally occurring polypeptide) of one or more functions such as, for example, the ability to form a pore in a cell (e.g., in a cell membrane), the ability to make changes to the cytoskeleton of the cell (e.g., reorganizing the cytoskeleton, rearranging the cytoskeleton, making changes to the cytoskeleton to allow the cell to fuse), the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some examples, the function polypeptide has an increased level of one or more functions as compared to another polypeptide (e.g., a naturally occurring polypeptide). Nucleic acid molecules can be designed to encode for functional polypeptides.

A "functionally equivalent" polypeptide (e.g., a myomerger polypeptide) is defined as a polypeptide that has been modified (e.g., by one or more insertions, one or more deletions, or one or more substitutions (e.g., conservative substitutions)) from an original polypeptide (e.g., a wt-myomerger plypeptide) and that modified polypeptide retains desired levels (e.g., at least about 20%, at least about 40%, at least about 50%, at least about 75%, at least about 80%, at least about 90%, at least about 95%, or at least about 99%) of one or more functions of the original polypeptide, such as, for example, the ability to form a pore in a cell (e.g., in a cell membrane), the ability to make changes to the cytoskeleton of the cell (e.g., reorganizing the cytoskeleton, rearranging the cytoskeleton, making changes to the cytoskeleton to allow the cell to fuse), the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some examples, the functionally equivalent polypeptide has an increased level of one or more functions compared to the original polypeptide. Nucleic acid molecules can be designed to encode for functionally equivalent polypeptides.

In certain embodiments, the shorter the length of a polypeptide, the fewer the modifications (e.g., substitutions) that can be made within the polypeptide while retaining, for example, a desired level of a chosen function. In some instances, longer domains can have a greater number of such changes while retaining, for example, a desired level of a chosen function. In other examples, a full-length polypeptide can have more tolerance for a fixed number of changes while retaining, for example, a desired level of a chosen function, as compared to a shorter length of that polypeptide.

The design of substitutions can take many forms, including but not limited to those described herein. In some examples, the hydropathic index of amino acids may be considered in designing substitutions. In the hydropathic index, each amino acid is assigned a hydropathic index on the basis of their hydrophobicity or charge characteristics, as follows: isoleucine (+4.5); valine (+4.2); Leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); or arginine (-4.5). In some instances, certain amino acids may be substituted for other amino acids having a similar hydropathic index. In making changes based upon the hydropathic index, the substitution of amino acids with hydropathic indices can be made with amino acids that have an index difference of no more than ±2, no more than ±1, or no more than ±0.5.

In some examples, substitutions can also be made based on hydrophilicity values. As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, the substitution of amino acids with hydrophilicity values can be made with amino acids that have a value of no more than ±2, no more than ±1, or no more than ±0.5.

A "conservative substitution" in an amino acid sequence or polypeptide indicates that a given amino acid residue is replaced by a residue having similar physiochemical characteristics (e.g., no more than ±1 when based on hydropathic index or no more than ±1 when base on hydrophilicity values). Examples of conservative substitutions include (a) substitution of one aliphatic residue for another with an aliphatic residue, (b) substitution of one of Ile, Val, Leu, or Ala for one another of He, Val, Leu, or Ala, (c) substitution of one of Gly, Ile, Val, Leu, or Ala for one another of Gly, Ile, Val, Leu, or Ala, (d) substitution of one polar residue for another polar residue, (e) substitution of one of Lys and Arg with another of Lys and Arg, (f) substitution of one of Glu and Asp with another of Glu and Asp, (g) substitution of one of Gln and Asn with another of Gln and Asn, (h) substitution of one hydroxyl or sulfur containing residue with another hydroxyl or sulfur containing residue, (i) substitution of one of Ser, Cys, Thr, or Met with another of Ser, Cys, Thr, or Met, (j) substitution of one aromatic residue for another with an aromatic residue, (k) substitution of one of Phe, Tyr, or Trp with another of Phe, Tyr, or Trp, (l) substitution of one basic residue for another basic residue, (m) substitution of one of His, Lys, or Arg with another of His, Lys, or Arg, (n) substitution of an acidic/amide residue with another acidic/amide residue, (o) substitution of one of Asp, Glu, Asn, or Gln with another of Asp, Glu, Asn, or Gln, (p) substitution of a residue with another residue of a similar size, and (q) substitution of one of Ala, Gly, or Ser with another of Ala, Gly, or Ser. In some examples, each amino acid in a hydrophobic region of a polypeptide can be substituted with conservative substitutions (e.g., any combination of conservative substitutions relating to hydrophobic residues) .

While discussion has focused on amino acid changes, it will be appreciated that these changes may occur by alteration of the encoding DNA; taking into consideration also that the genetic code is degenerate and that two or more codons may code for the same amino acid. A table of amino acids and their codons is presented below for use in such examples, as well as for other uses, such as in the design of probes and primers and the like.

**Tables A and B. Amino acid designations and codon table**

| **Table A - Amino Acid Designations** | | | **Table B - Codons for Amino Acids** |
|---|---|---|---|
| Alanine | Ala | A | GCA GCC GCG GCU |
| Cysteine | Cys | C | UGC UGU |
| Aspartic acid | Asp | D | GAC GAU |
| Glutamic acid | Glu | E | GAA GAG |
| Phenylalanine | Phe | F | UUC UUU |
| Glycine | Gly | G | GGA GGC GGG GGU |
| Histidine | His | H | CAC CAU |
| Isoleucine | Ile | I | AUA AUC AUU |
| Lysine | Lys | K | AAA AAG |
| Leucine | Leu | L | UUA UUG CUA CUC CUG CUU |
| Methionine | Met | M | AUG |
| Asparagine | Asn | N | AAC AAU |
| Proline | Pro | P | CCA CCC CCG CCU |
| Glutamine | Gln | Q | CAA CAG |
| Arginine | Arg | R | AGA AGG CGA CGC CGG CGU |
| Serine | Ser | S | AGC AGU UCA UCC UCG UCU |
| Threonine | Thr | T | ACA ACC ACG ACU |
| Valine | Val | V | GUA GUC GUG GUU |
| Tryptophan | Trp | W | UGG |
| Tyrosine | Tyr | Y | UAC UAU |

The term "functionally equivalent codon" is used herein to refer to codons that encode the same amino acid, such as the six codons for arginine or serine.

In certain instances, the nucleic acid molecule can be engineered to contain distinct sequences while at the same time retaining the capacity to encode a desired polypeptide. In some examples, this can be accomplished owing to the degeneracy of the genetic code (i.e., the presence of multiple codons) which encode for the same amino acids. In other instances, it can be accomplished by including, adding, or excluding introns in the nucleic acid molecule.

In certain examples, a restriction enzyme recognition sequence can be introduced into a nucleic acid sequence while maintaining the ability of that nucleic acid molecule to encode a desired polypeptide. In other examples, a CRISPR system (e.g., a CRISPR system comprising one or more of guide RNA, crRNA, tracrRNA, sgRNA, DNA repair template, and Cas protein, such as but not limited to CRISPR/Cas9) can be used to introduce a nucleic acid molecule while maintaining the ability of that nucleic acid molecule to encode a desired polypeptide.

It will also be understood that amino acid sequences (e.g., polypeptides) and nucleic acid sequences may include additional residues, such as additional N- or C-terminal amino acids or 5' or 3' sequences, and yet still be essentially as set forth in one of the sequences disclosed herein, so long as the sequence meets the criteria set forth above, such as including the maintenance of biological activity where polypeptide expression is concerned. The addition of terminal sequences particularly applies to nucleic acid sequences that may, for example, include various non-coding sequences flanking either of the 5' or 3' portions of the coding region or may include various internal sequences, (i.e., introns) which can occur within genes.

Some examples rely on or use synthesis of polypeptides *in cyto,* via transcription and translation of appropriate nucleic acid molecules (e.g., nucleic acid sequences as discussed herein). These polypeptides will include the twenty "natural" amino acids, and post-translational modifications thereof. *In vitro* peptide synthesis permits the use of modified or unusual amino acids. In some examples, the polypeptide encompasses modifications (e.g., one or more substitutions or one or more insertions) that include one or more modified or unusual amino acids. A table of exemplary, but not limiting, modified or unusual amino acids is provided in Table C.

| **Table C - Modified or Unusual Amino Acids** | | | |
|---|---|---|---|
| Abbr. | Amino Acid | Abbr. | Amino Acid |
| Aad | 2-Aminoadipic acid | EtAsn | N-Ethylasparagine |
| BAad | 3- Aminoadipic acid | Hyl | Hydroxylysine |
| BAla | beta-alanine, beta-Amino-propionic acid | AHyl | allo-Hydroxylysine |
| Abu | 2-Aminobutyric acid | 3Hyp | 3-Hydroxyproline |
| 4Abu | 4- Aminobutyric acid, piperidinic acid | 4Hyp | 4-Hydroxyproline |
| Acp | 6-Aminocaproic acid | Ide | Isodesmosine |
| Ahe | 2-Aminoheptanoic acid | Aile | allo-Isoleucine |
| Aib | 2-Aminoisobutyric acid | MeGly | N-Methylglycine, sarcosine |
| BAib | 3-Aminoisobutyric acid | MeIle | N-Methylisoleucine |
| Apm | 2-Aminopimelic acid | MeLys | 6-N-Methyllysine |
| Dbu | 2,4-Diaminobutyric acid | MeVal | N-Methylvaline |
| Des | Desmosine | Nva | Norvaline |
| Dpm | 2,2'-Diaminopimelic acid | Nle | Norleucine |
| Dpr | 2,3-Diaminopropionic acid | Orn | Ornithine |
| EtGly | N-Ethylglycine | | |

The presently disclosed subject matter further includes a method of producing a polypeptide (e.g., a mutant myomerger polypeptide or a wt-myomeger polypeptide). Any suitable method can used to make the polypeptides including but not limited to expression through any suitable molecular biological technique (e.g., using a prokaryotic or eukaryotic expression system), isolation from a source in nature, or chemical synthesis. Eukaryotic expression systems include plant-based systems; insect cell systems via recombinant baculoviruses; whole insect systems via recombinant baculoviruses; genetically engineered yeast systems, including but not limited to *Saccharomyces sp.* and *Picchia spp*.; and mammalian cell systems, including but not limited to C2C12 cells, 10T 1/2 fibroblasts, NIH/3T3 fibroblasts, mesenchymal stem cells (MSCs), hematopoietic stem cells, Chinese hamster ovary cells or other cell lines commonly used for industrial scale expression of recombinant proteins. In some examples, useful plant-based expression systems can include transgenic plant systems. In some examples, useful plant-based expression systems can include transplastomic plant systems.

In some examples, a method of producing the polypeptide includes providing a host cell comprising a nucleic acid molecule, as disclosed herein, operatively linked to a promoter operable under conditions whereby the encoded polypeptide is expressed; and recovering the polypeptide from the host cell.

### Myomaker Polypeptides and Myomaker Nucleic Acid Molecules

The first cell and the second cell each comprise a myomaker polypeptide. Exemplarily, the myomaker polypeptide can be the myomaker protein disclosed in WO 2014/210448 A1. In other examples, the myomaker polypeptide is the myomaker protein disclosed in Table 10A of WO 2014/210448 A1. The term "myomaker polypeptide" encompasses "wt-myomaker polypeptides" (i.e., myomaker polypeptides found in nature without any purposely human-made modification) and "mutant myomaker polypeptides" (e.g., with one or more modifications made to a wt-myomaker polypeptide). Nonlimiting examples of wt-myomaker polypeptides are found in Table 10A of WO 2014/210448 A1 or in Table 2A. In other examples, the myomaker polypeptide has at least one amino acid modification relative to a wt-myomaker polypeptide. A wt-myomaker polypeptide can, in some examples, be a myomaker polypeptide from any animal including but not limited to a mammal, a rat, a cat, a rabbit, a human, a cow, a chicken, a turkey, a monkey, a tree shrew, a dog, a pig, a shrew, an elephant, or an opossum. Table 2A provides nonlimiting examples of wt-myomaker polypeptides and Tables 2B and 2C provide nonlimiting examples of related nucleic acid sequences (including start and stop codons).

**Table 2A**

| **Source** | **Polypeptide sequence** |
|---|---|
| Human | MGTLVAKLLLPTLSSLAFLPTVSIAAKRRFHMEAMVYLFTLFFVAL HHACNGPGLSVLCFMRHDILEYFSVYGTALSMWVSLMALADFDEP KRSTFVMFGVLTIAVRIYHDRWGYGVYSGPIGTAILIIAAKWLQKM KEKKGLYPDKSVYTQQIGPGLCFGALALMLRFFFEDWDYTYVHSF YHCALAMSFVLLLPKVNKKAGSPGTPAKLDCSTLCCACV (SEQ ID NO: 50) |
| Dog | MGTLAAKLLLPTLSSLAFLPTVSIAAKRRFHMEAMVYLFTMFFVAL HHACNGPGLSVLCFMRHDVLEYFSVYGTALSMWVSLMALADFDE PKRSTFVMFGVLTIAVRIYHDRWGYGVYSGPIGTAVLIIATKWLQQ MKEKKSLYPDKSVYTQQIGPGLCFGALALMLRFFFEDWDYTYVHS FYHCALAMSFVLLLPKVNKKAGSAGPPAKLDCSTLCCACI (SEQ ID NO: 51) |
| Pig | MGTVMAKLLLPTLSSLAFLPTVSIAAKRRFHMEAMVYLFTTFFVAF YHACHGPGLAMICFLRLDILEYFSVYGTALSMWVSLMALADFDEP KRSTFVMFGVLTIAVRIYHDRWGYGVYSGPIGTAALIIAAKWLQQ MKDQRRLYPDKSVYTQQIGPGLCFGALALMLRFFFEEWDYTYVHS FYHCALAMSFVLLLPKANKKAGSAGPPAKLDCSTLCCACI (SEQ ID NO: 52) |
| Mouse | MGTVVAKLLLPTLSSLAFLPTVSIATKRRFYMEAMVYLFTMFFVAF SHACDGPGLSVLCFMRRDILEYFSIYGTALSMWVSLMALADFDEP QRSTFTMLGVLTIAVRTFHDRWGYGVYSGPIGTATLIIAVKWLKK MKEKKGLYPDKSIYTQQIGPGLCFGALALMLRFFFEEWDYTYVHS FYHCALAMSFVLLLPKVNKKAGNAGAPAKLTFSTLCCTCV (SEQ ID NO: 53) |
| Opossum | MGTLVTKLLLPTISSLAFLPTISIAAKRRFHMEAMVYLFTMFFIAIYH ACDGPGLSVLCFMRYDILEYFSIYGTALSMWVSLMALAEFDEPKRS TFVMFGVLTIAVRIYQDRWGYGVYSGPIGTAVLIIATKWLQKMKE KKGLYPDKSVYTQQIGPGFCFGALALMLRFFFQEWDYTYVHSFYH CSLAMSFVLLLPKVNKKAGNAGTPAKLDCSTLCCACI (SEQ ID NO: 54) |
| Zebrafish | MGAFIAKMLLPTISSLVFVPAASVAAKRGFHMEAMVYFFTMFFTAI YHACDGPGLSILCFMKYDILEYFSVYGTAISMWVTLLALGDFDEPK RSSLTMFGVLTAAVRIYQDRLGYGIYSGPIGTAVFMITVKWLQKM KEKKGLYPDKSVYTQQVGPGCCFGALALMLRFYFEEWDYAYVHS FYHVSLAMSFILLLPKKNRYAGTGRNAAKLNCYTLCCCV (SEQ ID NO: 55) |

**Table 2B**

| **Source** | **cDNA nucleic acid sequence** |
|---|---|
| Human | atggggac gctggtggcc aagctgctcc tgcccaccct cagcagcctg gccttcctcc ccactgtcag catcgcggcc aagaggcggt tccacatgga ggccatggtc tacctcttca ccctgttctt cgtggcgctc caccatgcct gcaatggacc cggcttgtct gtgctgtgct tcatgcgtca cgacatcctg gagtatttca gtgtctacgg gacagccctg agcatgtggg tctcgctgat ggcactggcc gacttcgacg aacccaagag gtcaacattt gtgatgttcg gcgtcctgac cattgctgtg cggatctacc atgaccgatg gggctacggg gtgtactcgg gccccatcgg cacagccatc ctcatcatcg cggcaaagtg gctacagaag atgaaggaga agaagggcct gtacccagac aagagcgtct acacccagca gataggcccc ggcctctgct tcggggcgct ggccctgatg ctacgcttct tctttgagga ctgggactac acttatgtcc acagcttcta ccactgtgcc ctggctatgt cctttgttct gctgctgccc aaggtcaaca agaaggctgg atccccgggg accccggcca agctggactg ctccaccctg tgctgtgctt gtgtctga (SEQ ID NO: 56) |
| Dog | atgggga cgctcgcggc gaagctgctc ctgcccaccc tcagcagcct ggccttcctc cccaccgtca gcatcgccgc caagcggcgg ttccacatgg aggccatggt ctacctcttc accatgttct tcgtggcact ccaccacgcg tgcaacgggc ccgggctatc ggtgctctgc ttcatgcgcc acgacgtcct ggagtacttc agcgtctatg ggacggcact gagcatgtgg gtctcgctga tggcactggc tgacttcgac gaacccaaga ggtcgacttt tgtgatgttt ggcgtcctga ccatcgccgt gcggatctac catgaccgct ggggctacgg ggtgtactcg ggccccattg gcacggctgt cctcatcatc gccacaaagt ggctgcagca gatgaaggag aagaagagtc tgtacccgga caagagtgtc tacacccagc agataggccc tggcctctgt ttggggcac tggcccttat gctgcgcttc tttttgagg actgggatta cacctatgtc cacagcttct accactgtgc cctggccatg tccttcgtcc tcctgctccc caaggtcaac aagaaggctg gaagcgcggg gccccctgcc aagctagact gctctaccct ttgctgtgct tgcatctga (SEQ D NO: 57) |
| Pig | atgg ggaccgtcat ggccaaactg ctgctaccca cgctgagcag cctggccttc ctccccacgg tcagcatcgc tgccaagcgg cggttccaca tggaggccat ggtctatctc ttcaccacgt tcttcgtggc gttctaccac gcctgccacg ggccgggcct ggctatgatc tgctttctgc gccttgacat cctggagtat ttcagcgtct acggaaccgc cctgagcatg tgggtctcgc tgatggcgct ggctgacttc gacgagccca agaggtcgac tttcgtgatg tttggcgtcc tgaccatcgc cgtgcggatc taccacgacc gctggggcta cggcgtgtac tcgggcccca tcggcacggc cgccctcatc atcgcggcca agtggctgca gcagatgaag gaccaacggc gcctgtatcc agacaagagc gtgtacacac agcagatagg ccccggcctc tgcttcgggg cgctggccct catgctgcgc tttttcttcg aggagtggga ttatacctac gtccacagct tctaccactg cgccctggcc atgtccttcg tcctgctgct gcccaaggcc aacaagaagg ctggaagcgc agggccaccc gccaagctgg actgctccac cctctgctgt gcttgtatct ga (SEQ ID NO: 58) |
| Mouse | atgg ggacagttgt agccaaactg ctcctgccta ccctcagcag cctggccttc ctcccgacag tgagcatcgc taccaagagg cgtttctaca tggaggccat ggtctacctc ttcaccatgt tctttgtggc gttctcccat gcctgtgatg ggcctggttt gtctgtgctg tgcttcatgc gccgtgacat |
| | tctggagtac ttcagcatct atggaacagc cctgagcatg tgggtctccc tgatggcact ggccgacttt gatgaacccc agagatcgac cttcacaatg cttggcgtcc ttaccatcgc tgtgcggact tttcatgacc gctggggtta cggggtatac tccggtccca taggcacggc caccctcatc attgctgtaa agtggctgaa gaagatgaaa gagaagaagg gcctgtaccc cgacaagagc atctacaccc agcagatagg ccccggcctg tgctttgggg ccctggccct gatgcttcga ttcttctttg aggaatggga ttacacctac gtccacagct tctaccactg tgccctggcc atgtcctttg tcctgctgct gcccaaggtc aacaagaagg ctgggaacgc aggggccccc gccaagctga ccttctccac cctctgctgc acttgtgtct ga (SEQ ID NO: 59) |
| Opossum | atggg gactcttgtt accaagttgc ttcttcccac aatcagcagc ctcgcctttc tccccaccat cagcatcgct gctaagagga gattccacat ggaagccatg gtctacctct tcaccatgtt cttcatagca atatatcatg catgtgacgg gccaggctta tcagtgctat gcttcatgcg ctatgacata ctggagtatt tcagcatcta tgggacagca ctgagcatgt gggtgtcatt aatggcactg gcagagttcg atgaaccaaa aaggtcaacc tttgtaatgt ttggcgtgtt gactattgcc gtgaggatct accaagaccg gtggggatat ggggtatact cggggcctat tggcacagct gtccttatca ttgcaacaaa atggctgcaa aagatgaaag agaagaaggg tctgtaccct gacaagagtg tgtacaccca acagataggc cctggtttct gttttggagc gttagcactg atgctgcgtt tctttttcca ggagtgggat tacacctatg ttcacagctt ctaccactgt tcactagcca tgtcctttgt cttgctgctg cccaaggtaa acaagaaagc tgggaatgct gggacacctg ccaaattgga ctgttctaca ctctgctgtg cttgcatctg a (SEQ ID NO: 60) |
| Zebrafish | atgggag cgtttatcgc caagatgttg ctgcccacta ttagcagttt ggtgtttgtg cctgcagcca gcgtggctgc aaagaggggc ttccacatgg aggccatggt ctatttcttc acaatgttct tcaccgcgat ttaccacgca tgtgacggtc cgggcttgtc cattctctgt ttcatgaagt atgacattct ggagtacttc agcgtgtacg ggacagccat ctccatgtgg gtcacgctac tggcgcttgg ggatttcgat gagcccaaac gctcttcgct caccatgttt ggggtgttga ccgcagctgt gaggatttac caggaccgac tgggctacgg catttactcc ggccccatcg ggacagctgt ctttatgatc acagtcaaat ggttacagaa aatgaaggaa aagaaaggcc tttatccaga caaaagtgtt tacactcaac aagtgggccc agggtgctgc ttcggtgctc ttgctttgat gcttcgcttc tattttgagg agtgggacta cgcttatgtt cacagtttct accacgtgtc tctggccatg tcctttattc tgctgctgcc caagaagaac cgttatgctg gaacgggacg taacgcagcc aaactcaact gctacaccct ctgctgctgt gtatga (SEQ ID NO: 61) |

**Table 2C (exons in lowercase)**

| **Source** | **Genomic nucleic acid sequence** |
|---|---|
| Human (+ strand) | caagtgtgagctggggagggcaggggctcagagccgggctgggcgcagcatcagacac aa gcacagcacagggtggagcagtccagcttggccggggtccccggggatccagccttct tg ttgaccttgggcagcagcagaacaaaggacatagccagggcacagtggtagaagctgt gg acataagtgtagtcccagtcCTGCGGGGGCAAGCGGTCAGTCTGGGGCCTCAGCCCC CT |
| | CCCCGAGGCTCCTCCCTCTCCAAGACCCAGCAGAGCCCCTTCAGGCCCCCGCCTCTGC CA GGGCACTGGGACACCTGCAGGAAGCCTCCCCCACGGTCGCGCTCACAGTGGTTTTTCT CT CCACCTAAACCCAGAGCAGTGAGGGCCTGTGCCATCCTCCAGGCTGCACTCCTTCCTT CT TCCCCATCCCCTCTCTCTGCTGTCCTTCTCTTCCTCCATCCTTCTCTCCCTCCTACCC TC CCTCCCTCCATCTCCCCCTCTTTTCTCTCCTTATCCCTCTTCCCCTGTTCCTCCCTCC CT CCTCCACTTTCTCCCTCCTTCCTTCCCTGTCTCCTCCCCTCCCTCCCTCCCTCCTCCA GG TGTTGGGCACCTGCCCCAGGCGTCTCCCAGGCTGTGCTGCCGTCTGAGATGCCAGCTG TC TGTAGGCAGCCAGCTTTGGTCTCTGTGACCTCCAGGTCCACACAGGCCATGGTGCTGG TG GTGCTGGGGACGGCATTGCCCCCGACATAGCCCTGGGAGGGGCTAGTGAGCAGGGACT AA TACCAGACTTTGGCCTGGGGCTGTCAGAGTCCCCCCAGCGTGGGCACAGCCCTGGTAT CC CAGCTGAGCAGAGCCATGCCGAGTGGGCTCTGGGGCACAGGACACCTCCCCGCTGGGC TT GGTACctcaaagaagaagcgtagcatcagggccagcgccccgaagcagaggccggggc ct atctgctgggtgtagacgctcttgtctgggtacaggcccttcttctccttcatcttct gt agCTGTGAGGACAGGAGGCCACAGCAAAGCTTTTAGGTCACAGCACTGGGGAACGCCC CT CCCCAAACCAGCCCGAGAGCTGGCCCTGCACAGGCTCACCCCAGCCCTCTCCCGGCAG GA GAGGAGGCTCAGGAGCCTCCTGCCGCACCCAGCCTCAGATGGCTTCTGCTGGACAGGG CC CTTCACGGTGCGACCCAGCAGAGACCCCAGCCTGGATGGCTGGGAAGGAAGCCACTGG GC CATGTGCCCCACAAAGACCCCGCTGCCCTCCCGCCTCTTTGAGATGTAACAACGCCAC CC TCGCATGTCTCCTCCTCCCTGGAGGGGAGCTCTGGGGGGACTAGACTCCATGATTGCT TA CCAAGGAAAGTACTGGAGTACTTGGGACCTGCCAGCCCAGTGTGGCCCATGGGGATGG CA CTTGTGGTGATCCCTGAGCCATGGACAAGCATCGTTTGCTTTCCTAGTTAAAGGACCT AT CTCACTCTTCATTAGACAAACTTGGCCAGCACTGCTTCTCAGGTCCCAGTGCTTAGGA AG GCTCGCGTGGGCGTTTCCACTTACAGAGGGGTTTGCATTCCGAGGAAGATGCGGGAAG TG TGGGGCCACATCCCTGGAGCCGGCCTTGTGTTTTCTAGGCCACTTCACATGGAGTCTA TT TGGGATTTTCAAGGGCAGTTGTTTCCTGGAATGAGGGTGGATTTTTCTCCCTGAGCCT GG TCCCCTCTTGGGAGGGGCTGGGGAACGACAGCCTTGTTGGGGAGGAAGGAGGGAGGGT TG GGTGATGGCGGCCTCGGAGTGGGGCCAGACCCGTGGGGGTACACTCAGGAGGCTATAG AT TTCAGTGGAATCAACTGTTAGACACACAGCGTGTGGCACAAGCCCCTGGGGGTGGGGG CA GCACCCCATAACTGCACCCATTGCTGAGTGGCCTATGCAAAGAGCACAAAGAGCCTTA TG CTGGGTCAGGTCAGGTTTTGCCACCCAGTGAATTATGAATTGATGCCCGGCTTTCCAT TT TCTGGAATTCCATTGCCAACAAGGAATTGAGCACCTGCAGTCCTGCAGTGGCCTGAAG |
| | AC AGCTGGACCGTGTGACCCTGGGTGCGGTGGTCAAGGCTGCCAGCCCACCTCTGGCCAG CC CTGCAGTAGTAACACCAGGGAGAAGAGAGGTGCCTGCCCCAGGTCACACAGTGGGCCT GG CACTATTGAAAGGGCGCCATCACCCAACCCTCCCTCCTTCTTCCTCCCGGGCTGCCAT TG CCCAACCCCTCCCAAGAGGGGACGAGGCTCAGGGAGAAAAATCCACCCTCATTCCAGG AA ACAATTGCCCTTGAAAATCCTAAATAAACTCCATACTAAATGGTCTAGAAGACAACAA TT TGAGCCCCAGATGCGGGGAGGCGGGCAGCCCATCCTCGGCTCCTGTGGCTGGATCTGC AG CCTGAGGGCCTTGGCAGTCTCGTGGCTCTTGGTGGGAAACACAGCAGTGAATTCTCTT CT GGGCAATTACAGTTCAGCCCAGTTCAGACCTGGCCAAGACCAGCGGGAGGAGCAACCT TC AGGGGCAGAAGGAGGCGAGAGGCGGGTGGCCAGGACCCAGGGCCCCAGCACGCTCCTT CC TGCCACCCACCTTGGTCCAGCCCACTTATGCCCAGCGCTCCCTCTCTCCCCACCAGGT GA CTCCCAGGGGCCTCCTGGGTCAGCCCAGGATTAGTGCTGCTTCCTCAGGTTGCAGACA GA AAGCAGGTCCTCTGTCTCCTGCTCAAAAAGTCAAGTCCAGCCAGGCGTGGTGGCTCAT GC CTGTAATTCCAGCACTTTGGGAGACTGAGGCAGGCAGATTACCTGAAGTCAGGAGCTC AG GACCAGCCGGGCCAACGTGGTGAAACCCCATCGCTACTAAAAATATAAAAATTACCTG GG CGTGATGGCATGCGCCTATAATCCCAGCTACTCGGGAGGCTGAGACAGGAGAATCGCT TC AACCCGGGAGGCGGAGGTTGCAGTGAGCCAAGATGGCGCCATTGCACTCCAGCCTGGG TG ACAAGAGCAAAACTCCGTCTCAAAAAAAAAAAAAAAAAAGTCAGGTTCTGGCCCCGCC AC TGCCCTGCCATGACGTCCTGTTAAGTTGCTGAGGCCTCCATGCTTTGGTTCCTTCATA GG CCAAATGGCAAATCAGTCCCATGCTCCTTGGCTGTGGGGAGGATTGGGACGGGCTTTG CA AGCTGCCCACCAGAACTCGAGCGCTCTCCCCACAGCCGTGGGCCCTCCTGCACTGAGA GC TGCCCTCTGTCTTGCTGGGTGTCCTGCGGCTCTGGCCGGGGCTGGCAGTGTGGCTGGG CT GGACCAGGCCAGGTCCTCTCTTGGCACTTGAAACTGACCCTGAGACTTCAGGTCCACT CC AAAGAGGTGAAATGCAGCACAGGGATGTTCAGGCGGTGCCTGGGCTGCTGCAGGCCTG GA GAGCAGGCTCAGGCTGAAGCCTGCTGGCTCCCCAGGTCTGGGAGACCCTTGCAAGGGT GA GCTCCCTCCTGCTCTGGGGTCCCAGGAGATGCCCCGGGTCTATTTTTCCCTAAGATCC CT CTTTAGCTTGGGCGAGTTTGAGTGGGGTTTGGTCCCTGAGCCAGGAGGGTCTTGGTAG GA CGGAGAGAGCAGGGAGCACTGAAGACCACGTGAGGGCCTTGCTGCTCTGCAAGGGGCT GT CTGTGCTAGAAGGTCTGGCCCAGGCTGCCTCACTGTCATACCACACTCTCCCTCCTGG CT AGAACCAAGCTCGAGGCTCACTCCCTCCAGGAAGTCTTCCCAGATTACCCCAGGCCAT TT TCCAAGTTGATGTTGCATCTCTAAAGCAGCTGGTAGTAAGAGCGGTGATGAGAGTGAT AA |
| | CAAATAGCTCTTATGTGCGGAGCACATTGGAAGCCAGGCTCCATGCCAGGACTTCAGG TG CCTGATCTCAGTGAGTCTTTGAACCACCCCATGAGACAGGCAGGGGGCTGTAATGACA AC ACCTGCTTTACAGGTACGGGCGTGGAGGTGAGACATTGGGTAACTTGGGCTCAGTCTG GA GCTGGTGAGTACAGACAAGCGTCACACACAGTCTACACAGCCGGAGCACCTCATGGCT AT TTTCTACGTGGTTTTGCTGAATTCCTGCATCCACCCATTTGCCTATGAGGGCAGGAGG TA AATGAAGATCCGAGGCAGGAGGAGTCAGACAGGGGAGAGGTGACGGGCCTCCTGGGTC CC CGTTCATCGAGGCTCGCGCAGTACGCACccactttgccgcgatgatgaggatggctgt gc cgatggggcccgagtacaccccgtagccccatcggtcatggtagatccgcacagcaat gg tcaggacgccgaacatcacaaatgttgacctcttgggttcgtcgaagtcggccagtgC TG GAGGGGCCAGGGAGACACAGGGGGAGGTGAGTGGTCTCTCTTGCTCCTCCTGGCTACC CC CCCACCCCCCAGCCCCCAGGAGGCATCCTGTAGATGCCCTCTCTCGGTGTCCCCTCAG CC AGCGAGACCCTGAGGCCCAGCCTGGTCATGGAGGGGTCTGAATTCCAGCCAGTTTGAG AG GACAGGCAGCCTGCTGCTTCCCCATGGACACAGCAGCTTGGATTGTGCTCCCAGCACC TC ATTTTAATAAACAGACCACAGCTGGTTGTGGTGGCTCAGGTCTGCAATCCCAGTGCTT TG GGAGGCAGAGGCAGGAGGATCGTCTGAGACCAGGAGTTCAAGACTAGCCTGGGCAACA TA GCGGGACCCCCATCTCCACAAAAAATTCGGTGGGTGTCGTGGTGCATGCCTGTCATCC CA GCTACTTGGGAGGCTGAGGTGGGAGGATGGCTTGAGGCTGTGAGTTCGAGGCTGCAGT GA GCCGTGTTTGTGCCACTGTACTCTGGCCTGAGTGACAGAGTGAGACCCTGTGGCTAAA AA TCAATAATCACTATGCAAAGTGAATAGGATCGAATCTATCCCATAGGATCACAGGACA AA GACACTAAGATTCAAGAGAAGAAATGAAGCCCCTCACAGGCCCGGTTAGATGGCAAGG AG CCTCAGGTCATGGGGACCTTGCCACAGACAACAGTTACGTGGAAAAAAACATGGTGGG AA AGGGGGCTTATGAACAGTCCCGTCTTCCAGGCTGGATATCACCCGTGTGTGTGGATGT TT GTATGACAGTCTGGGAAGCCAACCCCCCTGAGCAGTGAACAGCGGTCCTCCCAGGGAA GG AGTGACGGGAGGGAGCCCTTTCACTTTTTCCTTTGTATGCCTCTGCTGTTGAAATGTG TC ACAACAAGCTTTTACTAAATGAGTCATTTTAAAAGGATATAAAAAATCGGCATCAGGG CA TTTAAGAGGTGCATATTCTTTTTCATAGATTAAGCACAACCCTGAAACCCAGACAAGG GA AGACATTCCTGGGGCTGGGAGTGAGTGGGGATAGAGGGCTGCAGCGGGACTGGTTTGA GG CTGGGTGTGCGGACACTGGGGAGCCGGTCCTTGTCCGCAAGGCTTGTCTGCAGGGGTT GA CCACTCACccatcagcgagacccacatgctcagggctgtcccgtagacactgaaatac tc caggatgtcgtgacgcatgaagcacagcacagacaagccgggtccattgcaggcatgg tg gagCTGCCAGAAAACCCACAGGTGGTCACAGCACAAAGAGGCCAGAGCTGGTCCCCGA |
| | GC CACGGCCCCCAGAGTGCCAGGTCACTTGCTGGCTGTGAGAAGTCACTTTGGCGAGTCA CT TAATGACTGTGTGCCTCAGTCTCCCCGTCTGAAAAATGGGGGTACTGCCGAGCACTCC CG CAGAGGGTCCTGTGGGGATTAAGTGGCACATGCCAGCGAGGTGTTTAGGGGCTGGGGT GT GCCAAGGGTTCACTCAATGTCACCTCAGCAGAATTCGCTCATCTGCACTGGCAGGACT GG GCGGAGACTGAGTGGTCACTCAGGTGAAGCCCGCTTAGGTGGGGCGGTCTCCGGGAGG GA CCCTACACGGCTCTCCCCGGACCTTCAGCATCTGTGCTTCCTTGAAGCACACAGCTGC GT GTTCACTCGCCAATCTTTGGATGTGAGGTCAGAGCCTCTCTGGGGGCTCCTTTGCTCT TT GGGGGCTCCTGGGGCCTTCTCTTGCACAAATTACCCCTCTGATGACTGGTCTACACTG CA GCAGCGTTCTCAGGCTTGAGTGGGCATCAGAACGCCTGGGGCCTTGTTTAGACACAGG TT ACTGAGCCCTGCCTAGGGTTGCTGATTAGGGAGGGCTGGGTTGGGTAGAAAATGTGCA TT TTGAACACATTCCCTGTGGCACTGCTGAGGCTGGCAGGGCCCACACTGAGAGCCGGGC TG TAGCTCCTGGTTTCTGTTGCCTTAACGTGGACGAAGATCTCTGAGACCCCCTTGCAGA AG CTGAACACAGCCCCCTAGGCTCATCCATCTCTGCCCTATACTCTCGTCGTCGCCTCCC CA ACACCCACTTTCATGGCAATTTTTAAGGCAAAAGGCTTATAGGGAGTGTTTTCAAAGC AG TCAACTACTTTTCTACGGAAAACAACTCTCTCTCCTTTTGCATTCGCATTTCATCATT TT AGGTAATATTTAATTACATGACATAATTATTTTGACAGGTTCAACTGGCACAAACAAG CT TGGGAAACAGCACGGTGGACTCTTGGTCAGCCCAGCTCAGCGGGAGGAGCAGGCGTGC TG GAAAGCAGCCCGTGTCTGGAGGCGACAGGGACAGCACAGAGGGAGCGGGGGCCCTGGG TG ATCTGGGGGGCAGGCAATTCGGGGTCAAAGTGGAGTGCTTCTACTGATGGCAATTGTA CA CGGCCTAAAGTGACGGTGCACCTAGGAGGCATTAATAGGGATCCAGCATCTAAAATGA GG GAGGCGGCGGTCCTGCTTCTCTCTGTTCTTGTCAGGCTCATCCAGAAGACTATGCCGA GC TCTGTGTGGTGCACCTTTCTTGAAGTGAGACTGGGAAAGACGCGGCTAGAGGAGGGTG AC CAGCGGTGGACATGACTGTTTACCTTGGGGACAGGGAAGCTTCAGGAGGGGCCTGATC AA GGTGCTTACACCTCTGTGGGAAAGAGGAGCGAGGAAGACTCCGGCCCTAGGCTGTTCT CC TGTTCTCCTGGCTTCTTCCCATCCCCCACCCCAGCCCCATCACCTGCTGTCTGTGTGC CT CAATGTAGCACAGATGGTCATGTGTGATTAAGGCATTCACTGTGAGATTGTGATAAGG CC TGTGCCCTTGCCCTGCCAGGAGCAGGAATGGCTCTGTCTGGTCCCAGTTGCATGGACG GC TCCCAGCATAGAGTGCTTGCTGCATGTGTTCAGGGAGGGGGACGCCAGGCTCTGAGAA TT CTAAAGGACAGCCAGCTCACCCTGGGGACCCAGAGCCTCTGCCACTAGGCCCTTGGCT CC TCCCAATGGTGGGAACTTAGCTCCATTCGACAGATGGGGAAAGTGAACTTCAGAGCAG CA |
| | CTGCCTGCCCAAAGAGGTGAAACAGAGCAGTGCTTGGCACCTGGCCACTTCCTCCCAT CC TGCAGTGCAGGGGGCAGACCTGGCCCAGCCGGGGCACTGGTGGGGTGGGTGCGGCTGA GG GCCTGGGGGGTCAGAGCTCAGGCTCGGGGAGTCTGACTTTGCAGATGTTCCCAGTGGG GG CTCAGGTGAGTGGCTGTCGGGGGGGGGCCTCCTCTGTTGTGTGGGGACAAGCACACTG TC TCCGTGGGGTTTGCACCCATAGCAAGGTGTCCGGCACAGAGATGGAGATTGTCACGGG AG GGGCCTGATTGGAAGGGAAGGGACGCCATGCGGGTGGCAGAACTTTGGGAGGGACTGA GT GTGGCTTTGAGTTCAGAAGACGTTTGTCACAAGAGGCAGCTGCCCCTGCCACTCTGGG TG GGGCAGGGTGGGGCCTCTGAGACCAGTGCAGAGGCAGCTGCGGGGCCAGCCTAGGCCC AG GCAGGGAGGTGTGGCCTGGTGGGTGCTTGTGGTTTGCTGGGCTAGGTCTAACAGGAGC CT TGAGAACAAGACCTCAGCTTTTCTCCCTGCGCTAAGGCCATGGGACCTGCAGAGAAAT CC TGGCTCTGCTCTGGGCTTCAGTCTCTCATCTGCCCAAGAGGCTTCCTAGCCCTAGCCC AG GCTGGAGTCCCAGAGGAGCGAATGCAGTGGCATTTGGGTGAGTCAGGAGCTCTGGAGA GC TTGATGGTCACAGTGACACAAGTGACTCTGTCTCTCTGGGATTTGGTTTCTTCATCTG CC AAATGGGAATCAAGATCCTAGGCTTGTGGGGAAGGTGAAAAGGCTGAATCAGACACTG TG CACAGAGCGCCTAGCCGAGTCCTCTGCCCTGGGTACTGGCGCTCGAGGTGGACTCAGA AG CTCCAGGGCATCTGGTTCCACAAAGGACCCAGCCTGTCCCAGGCCACTGTCACCCCTG GG AGTGGCACACACTGGAGGGAATGCCTCGCTCCCAGCCCACACGTGCACACTCAGCTTC TG CCATTGCGGGCAAAATTGGACTTGACCAATTCAGGATACAAGCATAACATGTGAATAT AT GCTTGCAAACACACGTGTGAGCTCACGGGCCTCACCCGCTCAGGACTCCCTCTGTGCA CT CACATGCACTTGGCATTCTTGCCCATAGAGGCCCTGCTGCTGGAGAAGGAGGCTGTCT GG GGAGAGGAGGTGGAGTTTTCACAGGTTGGGCCCAGCACTGCCCCAAGAAGGAGGCTAG TG GGACGCTTGCCTCCCCAGAGCAGGTGTCATGCTGGGGATTGGGCTGTCAGTGAAGGAG GG GTGTGATGGAAGGTGAGCAAGGAAGGCTTCGGGAGAGCAAGAGGTGGGGCACCACTTG TG GGAGTCCAGGAGTGAGGGCATGTTAGTGGAGAAAGTCGGAAAGACCCAGAGGCAAGAA GG CAGGGGGTACCGAGACATATAAATGATGGCTGAATGGCGAGATGGTAATAGACGAATA GA TCACAGGTAGATGGATGCGTAGATAGAGAGATAGATGGAGAGAGAGAGAGAGAGAGAG AG AGAGAGAGAGAGAGACAAGCTGGAGAAGGTGGATAGCTAAAGCCAGAGAGACACATGG AG AGTCAGGGGACTAAAACCAGGGAGGGTGGACCAAGAGCTTTAGAGAGAGTGAATTCC AT GGGGATCGAGTTCCAGAAATCAAAAGAGAACCAGACAGAGAGAGAAAGGAAAAAAAGA GA AACAGAGAAAACTAGACACAGAAAACCAATACGAGAAACACAGAGTGAAAGAGACCCA GA AAGAGAGAGAGAGAAGACAGGGGAGACAGGGGTCCCAGAAACAGCGACCTCAGAAACA |
| | AG GACAGATGGGGTTCTGGGCGCTCCACTGAAAGCCGGATAAGATCACCCAATGACAGGT AC CAGGAAACAGAGAGCAGGAGAGAGCCAGAGAGAGAGCAAGCGGAGACAGTCAGCCAGC CA GACACATAAATAGAAAGAGAAAGACGGACCCACAGAGAGAGAAGTAGGCCCCAGAAGA GG GAGAGACCAGCAGGCCCTCCTGAACCAGAGCAGCTCCAGGATTCTGGAATCAGACTCA CT CACCCAGGCCTTCACACTCCCTGAACCCTGCAGACCCCTTCCCAGGCCTGGCTTGCCC CA CTCATCTCTGCTCCATCGTGGCCTATGGGTAGAGCTCGAAGAGAGGTGGGGAGGGGAG GT GGCCCCATGGGCAGCCGTGGGGGCTTTGATTAGCAGCTGAGAAAAGGGGCACGCTGGA AG GGTTTATCCTCAACTCAATGGCCCTGCTTCACCCCAGGCTTGGTCTCACACAGGCAGT GA TCCCAGAGCAACTTCCTGGCACAGATGGGAAAACTGAGGTCCAGATAGGGGAAGGGAC TC CCCTAGTCCTCTCTCTTCAGTCTCCAGACCCCACCTGGGCCTGCTGTTTCATTTTCAA AT CACTTCTGCTCATCACCCAATACAAGAACGCTGTGGACAGAGAGCCTCTCCTCTACCT CC AGGATGGGGCCTGTGTGGGACTTCCTCCCAGCCCCCAGACTCACcgccacgaagaaca gg gtgaagaggtagaccatggcctccatgtggaaccgcctcttggccgcgatgctgacag tg gggaggaaggccaggctgctgagggtgggcaggagcagcttggccaccagcgtcccca tg ggccaggaggaaagcactggctggggtggggagggtgctggtgtcccaggtccccagc ac aggagcacgaagtgggaaggccagctccctttgggcagggc (SEQ ID NO: 62) |
| Human (- strand, reverse complement) - start codon is bold & underlined; stop codon is bold and italicized | gccctgcccaaaggagctggccttcccacttcgtgctcctgtgctggggacctggga ca ccagcaccctccccaccccagccagtgctttcctcctggcccatgggacgctggtgg cc aagctgctcctgcccaccctcagcagcctggccttcctccccactgtcagcatcgcgg cc aagaggcggttccacatggaggccatggtctacctcttcaccctgttcttcgtggcgG TG AGTCTGGGGGCTGGGAGGAAGTCCCACACAGGCCCCATCCTGGAGGTAGAGGAGAGGC TC TCTGTCCACAGCGTTCTTGTATTGGGTGATGAGCAGAAGTGATTTGAAAATGAAACAG CA GGCCCAGGTGGGGTCTGGAGACTGAAGAGAGAGGACTAGGGGAGTCCCTTCCCCTATC TG GACCTCAGTTTTCCCATCTGTGCCAGGAAGTTGCTCTGGGATCACTGCCTGTGTGAGA CC AAGCCTGGGGTGAAGCAGGGCCATTGAGTTGAGGATAAACCCTTCCAGCGTGCCCCTT TT CTCAGCTGCTAATCAAAGCCCCCACGGCTGCCCATGGGGCCACCTCCCCTCCCCACCT CT CTTCGAGCTCTACCCATAGGCCACGATGGAGCAGAGATGAGTGGGGCAAGCCAGGCCT GG GAAGGGGTCTGCAGGGTTCAGGGAGTGTGAAGGCCTGGGTGAGTGAGTCTGATTCCAG AA TCCTGGAGCTGCTCTGGTTCAGGAGGGCCTGCTGGTCTCTCCCTCTTCTGGGGCCTAC TT CTCTCTCTGTGGGTCCGTCTTTCTCTTTCTATTTATGTGTCTGGCTGGCTGACTGTCT |
| | CC GCTTGCTCTCTCTCTGGCTCTCTCCTGCTCTCTGTTTCCTGGTACCTGTCATTGGGTG AT CTTATCCGGCTTTCAGTGGAGCGCCCAGAACCCCATCTGTCCTTGTTTCTGAGGTCGC TG TTTCTGGGACCCCTGTCTCCCCTGTCTTCTCTCTCTCTCTTTCTGGGTCTCTTTCACT CT GTGTTTCTCGTATTGGTTTTCTGTGTCTAGTTTTCTCTGTTTCTCTTTTTTTCCTTTC TC TCTCTGTCTGGTTCTCTTTTGATTTCTGGAACTCGATCCCCATGGAATTCACTCTCTC TA AAGCTCTTGGTCCCACCCTCCCTGGTTTTAGTCCCCTGACTCTCCATGTGTCTCTCTG GC TTTAGCTATCCACCTTCTCCAGCTTGTCTCTCTCTCTCTCTCTCTCTCTCTCTCTCTC TC TCTCCATCTATCTCTCTATCTACGCATCCATCTACCTGTGATCTATTCGTCTATTACC AT CTCGCCATTCAGCCATCATTTATATGTCTCGGTACCCCCTGCCTTCTTGCCTCTGGGT CT TTCCGACTTTCTCCACTAACATGCCCTCACTCCTGGACTCCCACAAGTGGTGCCCCAC CT CTTGCTCTCCCGAAGCCTTCCTTGCTCACCTTCCATCACACCCCTCCTTCACTGACAG CC CAATCCCCAGCATGACACCTGCTCTGGGGAGGCAAGCGTCCCACTAGCCTCCTTCTTG GG GCAGTGCTGGGCCCAACCTGTGAAAACTCCACCTCCTCTCCCCAGACAGCCTCCTTCT CC AGCAGCAGGGCCTCTATGGGCAAGAATGCCAAGTGCATGTGAGTGCACAGAGGGAGTC CT GAGCGGGTGAGGCCCGTGAGCTCACACGTGTGTTTGCAAGCATATATTCACATGTTAT GC TTGTATCCTGAATTGGTCAAGTCCAATTTTGCCCGCAATGGCAGAAGCTGAGTGTGCA CG TGTGGGCTGGGAGCGAGGCATTCCCTCCAGTGTGTGCCACTCCCAGGGGTGACAGTGG CC TGGGACAGGCTGGGTCCTTTGTGGAACCAGATGCCCTGGAGCTTCTGAGTCCACCTCG AG CGCCAGTACCCAGGGCAGAGGACTCGGCTAGGCGCTCTGTGCACAGTGTCTGATTCAG CC TTTTCACCTTCCCCACAAGCCTAGGATCTTGATTCCCATTTGGCAGATGAAGAAACCA AA TCCCAGAGAGACAGAGTCACTTGTGTCACTGTGACCATCAAGCTCTCCAGAGCTCCTG AC TCACCCAAATGCCACTGCATTCGCTCCTCTGGGACTCCAGCCTGGGCTAGGGCTAGGA AG CCTCTTGGGCAGATGAGAGACTGAAGCCCAGAGCAGAGCCAGGATTTCTCTGCAGGTC CC ATGGCCTTAGCGCAGGGAGAAAAGCTGAGGTCTTGTTCTCAAGGCTCCTGTTAGACCT AG CCCAGCAAACCACAAGCACCCACCAGGCCACACCTCCCTGCCTGGGCCTAGGCTGGCC CC GCAGCTGCCTCTGCACTGGTCTCAGAGGCCCCACCCTGCCCCACCCAGAGTGGCAGGG GC AGCTGCCTCTTGTGACAAACGTCTTCTGAACTCAAAGCCACACTCAGTCCCTCCCAAA GT TCTGCCACCCGCATGGCGTCCCTTCCCTTCCAATCAGGCCCCTCCCGTGACAATCTCC AT CTCTGTGCCGGACACCTTGCTATGGGTGCAAACCCCACGGAGACAGTGTGCTTGTCCC CA CACAACAGAGGAGGCCCCCCCCCGACAGCCACTCACCTGAGCCCCCACTGGGAACATC TG |
| | CAAAGTCAGACTCCCCGAGCCTGAGCTCTGACCCCCCAGGCCCTCAGCCGCACCCACC CC ACCAGTGCCCCGGCTGGGCCAGGTCTGCCCCCTGCACTGCAGGATGGGAGGAAGTGGC CA GGTGCCAAGCACTGCTCTGTTTCACCTCTTTGGGCAGGCAGTGCTGCTCTGAAGTTCA CT TTCCCCATCTGTCGAATGGAGCTAAGTTCCCACCATTGGGAGGAGCCAAGGGCCTAGT GG CAGAGGCTCTGGGTCCCCAGGGTGAGCTGGCTGTCCTTTAGAATTCTCAGAGCCTGGC GT CCCCCTCCCTGAACACATGCAGCAAGCACTCTATGCTGGGAGCCGTCCATGCAACTGG GA CCAGACAGAGCCATTCCTGCTCCTGGCAGGGCAAGGGCACAGGCCTTATCACAATCTC AC AGTGAATGCCTTAATCACACATGACCATCTGTGCTACATTGAGGCACACAGACAGCAG GT GATGGGGCTGGGGTGGGGGATGGGAAGAAGCCAGGAGAACAGGAGAACAGCCTAGGGC CG GAGTCTTCCTCGCTCCTCTTTCCCACAGAGGTGTAAGCACCTTGATCAGGCCCCTCCT GA AGCTTCCCTGTCCCCAAGGTAAACAGTCATGTCCACCGCTGGTCACCCTCCTCTAGCC GC GTCTTTCCCAGTCTCACTTCAAGAAAGGTGCACCACACAGAGCTCGGCATAGTCTTCT GG ATGAGCCTGACAAGAACAGAGAGAAGCAGGACCGCCGCCTCCCTCATTTTAGATGCTG GA TCCCTATTAATGCCTCCTAGGTGCACCGTCACTTTAGGCCGTGTACAATTGCCATCAG TA GAAGCACTCCACTTTGACCCCGAATTGCCTGCCCCCCAGATCACCCAGGGCCCCCGCT CC CTCTGTGCTGTCCCTGTCGCCTCCAGACACGGGCTGCTTTCCAGCACGCCTGCTCCTC CC GCTGAGCTGGGCTGACCAAGAGTCCACCGTGCTGTTTCCCAAGCTTGTTTGTGCCAGT TG AACCTGTCAAAATAATTATGTCATGTAATTAAATATTACCTAAAATGATGAAATGCGA AT GCAAAAGGAGAGAGAGTTGTTTTCCGTAGAAAAGTAGTTGACTGCTTTGAAAACACTC CC TATAAGCCTTTTGCCTTAAAAATTGCCATGAAAGTGGGTGTTGGGGAGGCGACGACGA GA GTATAGGGCAGAGATGGATGAGCCTAGGGGGCTGTGTTCAGCTTCTGCAAGGGGGTCT CA GAGATCTTCGTCCACGTTAAGGCAACAGAAACCAGGAGCTACAGCCCGGCTCTCAGTG TG GGCCCTGCCAGCCTCAGCAGTGCCACAGGGAATGTGTTCAAAATGCACATTTTCTACC CA ACCCAGCCCTCCCTAATCAGCAACCCTAGGCAGGGCTCAGTAACCTGTGTCTAAACAA GG CCCCAGGCGTTCTGATGCCCACTCAAGCCTGAGAACGCTGCTGCAGTGTAGACCAGTC AT CAGAGGGGTAATTTGTGCAAGAGAAGGCCCCAGGAGCCCCCAAAGAGCAAAGGAGCCC CC AGAGAGGCTCTGACCTCACATCCAAAGATTGGCGAGTGAACACGCAGCTGTGTGCTTC AA GGAAGCACAGATGCTGAAGGTCCGGGGAGAGCCGTGTAGGGTCCCTCCCGGAGACCGC CC CACCTAAGCGGGCTTCACCTGAGTGACCACTCAGTCTCCGCCCAGTCCTGCCAGTGCA GA TGAGCGAATTCTGCTGAGGTGACATTGAGTGAACCCTTGGCACACCCCAGCCCCTAAA CA CCTCGCTGGCATGTGCCACTTAATCCCCACAGGACCCTCTGCGGGAGTGCTCGGCAGT |
| | AC CCCCATTTTTCAGACGGGGAGACTGAGGCACACAGTCATTAAGTGACTCGCCAAAGTG AC TTCTCACAGCCAGCAAGTGACCTGGCACTCTGGGGGCCGTGGCTCGGGGACCAGCTCT GG CCTCTTTGTGCTGTGACCACCTGTGGGTTTTCTGGCAGctccaccatgcctgcaatgg ac ccggcttgtctgtgctgtgcttcatgcgtcacgacatcctggagtatttcagtgtcta cg ggacagccctgagcatgtgggtctcgctgatggGTGAGTGGTCAACCCCTGCAGACAA GC CTTGCGGACAAGGACCGGCTCCCCAGTGTCCGCACACCCAGCCTCAAACCAGTCCCGC TG CAGCCCTCTATCCCCACTCACTCCCAGCCCCAGGAATGTCTTCCCTTGTCTGGGTTTC AG GGTTGTGCTTAATCTATGAAAAAGAATATGCACCTCTTAAATGCCCTGATGCCGATTT TT TATATCCTTTTAAAATGACTCATTTAGTAAAAGCTTGTTGTGACACATTTCAACAGCA GA GGCATACAAAGGAAAAAGTGAAAGGGCTCCCTCCCGTCACTCCTTCCCTGGGAGGACC GC TGTTCACTGCTCAGGGGGGTTGGCTTCCCAGACTGTCATACAAACATCCACACACACG GG TGATATCCAGCCTGGAAGACGGGACTGTTCATAAGCCCCCTTTCCCACCATGTTTTTT TC CACGTAACTGTTGTCTGTGGCAAGGTCCCCATGACCTGAGGCTCCTTGCCATCTAACC GG GCCTGTGAGGGGCTTCATTTCTTCTCTTGAATCTTAGTGTCTTTGTCCTGTGATCCTA TG GGATAGATTCGATCCTATTCACTTTGCATAGTGATTATTGATTTTTAGCCACAGGGTC TC ACTCTGTCACTCAGGCCAGAGTACAGTGGCACAAACACGGCTCACTGCAGCCTCGAAC TC ACAGCCTCAAGCCATCCTCCCACCTCAGCCTCCCAAGTAGCTGGGATGACAGGCATGC AC CACGACACCCACCGAATTTTTTGTGGAGATGGGGGTCCCGCTATGTTGCCCAGGCTAG TC TTGAACTCCTGGTCTCAGACGATCCTCCTGCCTCTGCCTCCCAAAGCACTGGGATTGC AG ACCTGAGCCACCACAACCAGCTGTGGTCTGTTTATTAAAATGAGGTGCTGGGAGCACA AT CCAAGCTGCTGTGTCCATGGGGAAGCAGCAGGCTGCCTGTCCTCTCAAACTGGCTGGA AT TCAGACCCCTCCATGACCAGGCTGGGCCTCAGGGTCTCGCTGGCTGAGGGGACACCGA GA GAGGGCATCTACAGGATGCCTCCTGGGGGCTGGGGGGTGGGGGGGTAGCCAGGAGGAG CA AGAGAGACCACTCACCTCCCCCTGTGTCTCCCTGGCCCCTCCAGcactggccgacttc ga cgaacccaagaggtcaacatttgtgatgttcggcgtcctgaccattgctgtgcggatc ta ccatgaccgatggggctacggggtgtactcgggccccatcggcacagccatcctcatc at cgcggcaaagtggGTGCGTACTGCGCGAGCCTCGATGAACGGGGACCCAGGAGGCCCG TC ACCTCTCCCCTGTCTGACTCCTCCTGCCTCGGATCTTCATTTACCTCCTGCCCTCATA GG CAAATGGGTGGATGCAGGAATTCAGCAAAACCACGTAGAAAATAGCCATGAGGTGCTC CG GCTGTGTAGACTGTGTGTGACGCTTGTCTGTACTCACCAGCTCCAGACTGAGCCCAAG TT |
| | ACCCAATGTCTCACCTCCACGCCCGTACCTGTAAAGCAGGTGTTGTCATTACAGCCCC CT GCCTGTCTCATGGGGTGGTTCAAAGACTCACTGAGATCAGGCACCTGAAGTCCTGGCA TG GAGCCTGGCTTCCAATGTGCTCCGCACATAAGAGCTATTTGTTATCACTCTCATCACC GC TCTTACTACCAGCTGCTTTAGAGATGCAACATCAACTTGGAAAATGGCCTGGGGTAAT CT GGGAAGACTTCCTGGAGGGAGTGAGCCTCGAGCTTGGTTCTAGCCAGGAGGGAGAGTG TG GTATGACAGTGAGGCAGCCTGGGCCAGACCTTCTAGCACAGACAGCCCCTTGCAGAGC AG CAAGGCCCTCACGTGGTCTTCAGTGCTCCCTGCTCTCTCCGTCCTACCAAGACCCTCC TG GCTCAGGGACCAAACCCCACTCAAACTCGCCCAAGCTAAAGAGGGATCTTAGGGAAAA AT AGACCCGGGGCATCTCCTGGGACCCCAGAGCAGGAGGGAGCTCACCCTTGCAAGGGTC TC CCAGACCTGGGGAGCCAGCAGGCTTCAGCCTGAGCCTGCTCTCCAGGCCTGCAGCAGC CC AGGCACCGCCTGAACATCCCTGTGCTGCATTTCACCTCTTTGGAGTGGACCTGAAGTC TC AGGGTCAGTTTCAAGTGCCAAGAGAGGACCTGGCCTGGTCCAGCCCAGCCACACTGCC AG CCCCGGCCAGAGCCGCAGGACACCCAGCAAGACAGAGGGCAGCTCTCAGTGCAGGAGG GC CCACGGCTGTGGGGAGAGCGCTCGAGTTCTGGTGGGCAGCTTGCAAAGCCCGTCCCAA TC CTCCCCACAGCCAAGGAGCATGGGACTGATTTGCCATTTGGCCTATGAAGGAACCAAA GC ATGGAGGCCTCAGCAACTTAACAGGACGTCATGGCAGGGCAGTGGCGGGGCCAGAACC TG ACTTTTTTTTTTTTTTTTTTGAGACGGAGTTTTGCTCTTGTCACCCAGGCTGGAGTGC AA TGGCGCCATCTTGGCTCACTGCAACCTCCGCCTCCCGGGTTGAAGCGATTCTCCTGTC TC AGCCTCCCGAGTAGCTGGGATTATAGGCGCATGCCATCACGCCCAGGTAATTTTTATA TT TTTAGTAGCGATGGGGTTTCACCACGTTGGCCCGGCTGGTCCTGAGCTCCTGACTTCA GG TAATCTGCCTGCCTCAGTCTCCCAAAGTGCTGGAATTACAGGCATGAGCCACCACGCC TG GCTGGACTTGACTTTTTGAGCAGGAGACAGAGGACCTGCTTTCTGTCTGCAACCTGAG GA AGCAGCACTAATCCTGGGCTGACCCAGGAGGCCCCTGGGAGTCACCTGGTGGGGAGAG AG GGAGCGCTGGGCATAAGTGGGCTGGACCAAGGTGGGTGGCAGGAAGGAGCGTGCTGGG GC CCTGGGTCCTGGCCACCCGCCTCTCGCCTCCTTCTGCCCCTGAAGGTTGCTCCTCCCG CT GGTCTTGGCCAGGTCTGAACTGGGCTGAACTGTAATTGCCCAGAAGAGAATTCACTGC TG TGTTTCCCACCAAGAGCCACGAGACTGCCAAGGCCCTCAGGCTGCAGATCCAGCCACA GG AGCCGAGGATGGGCTGCCCGCCTCCCCGCATCTGGGGCTCAAATTGTTGTCTTCTAGA CC ATTTAGTATGGAGTTTATTTAGGATTTTCAAGGGCAATTGTTTCCTGGAATGAGGGTG GA TTTTTCTCCCTGAGCCTCGTCCCCTCTTGGGAGGGGTTGGGCAATGGCAGCCCGGGAG GA AGAAGGAGGGAGGGTTGGGTGATGGCGCCCTTTCAATAGTGCCAGGCCCACTGTGTGA |
| | CC TGGGGCAGGCACCTCTCTTCTCCCTGGTGTTACTACTGCAGGGCTGGCCAGAGGTGGG CT GGCAGCCTTGACCACCGCACCCAGGGTCACACGGTCCAGCTGTCTTCAGGCCACTGCA GG ACTGCAGGTGCTCAATTCCTTGTTGGCAATGGAATTCCAGAAAATGGAAAGCCGGGCA TC AATTCATAATTCACTGGGTGGCAAAACCTGACCTGACCCAGCATAAGGCTCTTTGTGC TC TTTGCATAGGCCACTCAGCAATGGGTGCAGTTATGGGGTGCTGCCCCCACCCCCAGGG GC TTGTGCCACACGCTGTGTGTCTAACAGTTGATTCCACTGAAATCTATAGCCTCCTGAG TG TACCCCCACGGGTCTGGCCCCACTCCGAGGCCGCCATCACCCAACCCTCCCTCCTTCC TC CCCAACAAGGCTGTCGTTCCCCAGCCCCTCCCAAGAGGGGACCAGGCTCAGGGAGAAA AA TCCACCCTCATTCCAGGAAACAACTGCCCTTGAAAATCCCAAATAGACTCCATGTGAA GT GGCCTAGAAAACACAAGGCCGGCTCCAGGGATGTGGCCCCACACTTCCCGCATCTTCC TC GGAATGCAAACCCCTCTGTAAGTGGAAACGCCCACGCGAGCCTTCCTAAGCACTGGGA CC TGAGAAGCAGTGCTGGCCAAGTTTGTCTAATGAAGAGTGAGATAGGTCCTTTAACTAG GA AAGCAAACGATGCTTGTCCATGGCTCAGGGATCACCACAAGTGCCATCCCCATGGGCC AC ACTGGGCTGGCAGGTCCCAAGTACTCCAGTACTTTCCTTGGTAAGCAATCATGGAGTC TA GTCCCCCCAGAGCTCCCCTCCAGGGAGGAGGAGACATGCGAGGGTGGCGTTGTTACAT CT CAAAGAGGCGGGAGGGCAGCGGGGTCTTTGTGGGGCACATGGCCCAGTGGCTTCCTTC CC AGCCATCCAGGCTGGGGTCTCTGCTGGGTCGCACCGTGAAGGGCCCTGTCCAGCAGAA GC CATCTGAGGCTGGGTGCGGCAGGAGGCTCCTGAGCCTCCTCTCCTGCCGGGAGAGGGC TG GGGTGAGCCTGTGCAGGGCCAGCTCTCGGGCTGGTTTGGGGAGGGGCGTTCCCCAGTG CT GTGACCTAAAAGCTTTGCTGTGGCCTCCTGTCCTCACAGctacagaagatgaaggaga ag aagggcctgtacccagacaagagcgtctacacccagcagataggccccggcctctgct tc ggggcgctggccctgatgctacgcttcttctttgagGTACCAAGCCCAGCGGGGAGGT GT CCTGTGCCCCAGAGCCCACTCGGCATGGCTCTGCTCAGCTGGGATACCAGGGCTGTGC CC ACGCTGGGGGGACTCTGACAGCCCCAGGCCAAAGTCTGGTATTAGTCCCTGCTCACTA GC CCCTCCCAGGGCTATGTCGGGGGCAATGCCGTCCCCAGCACCACCAGCACCATGGCCT GT GTGGACCTGGAGGTCACAGAGACCAAAGCTGGCTGCCTACAGACAGCTGGCATCTCAG AC GGCAGCACAGCCTGGGAGACGCCTGGGGCAGGTGCCCAACACCTGGAGGAGGGAGGGA GG GAGGGGAGGAGACAGGGAAGGAAGGAGGGAGAAAGTGGAGGAGGGAGGGAGGAACAGG GG AAGAGGGATAAGGAGAGAAAAGAGGGGGAGATGGAGGGAGGGAGGGTAGGAGGGAGAG AA GGATGGAGGAAGAGAAGGACAGCAGAGAGAGGGGATGGGGAAGAAGGAAGGAGTGCAG CC |
| | TGGAGGATGGCACAGGCCCTCACTGCTCTGGGTTTAGGTGGAGAGAAAAACCACTGTG AG CGCGACCGTGGGGGAGGCTTCCTGCAGGTGTCCCAGTGCCCTGGCAGAGGCGGGGCC TG AAGGGGCTCTGCTGGGTCTTGGAGAGGGAGGAGCCTCGGGGAGGGGGCTGAGGCCCCA GA CTGACCGCTTGCCCCCCGCAGgactgggactacacttatgtccacagcttctaccact gt gccctggctatgtcctttgttctgctgctgcccaaggtcaacaagaaggctggatccc cg gggaccccggccaagctggactgctccaccctgtgctgtgcttgtgtctgatgctgcg cc cagcccggctctgagcccctgccctccccagctcacacttg (SEQ ID NO: 63) |
| Mouse (+ strand) | ttccaggaactagaatgtatgttaggcgaagctaatgactagtggctgatcaagagtt ta ctgtgaatggcttgatcgaaaacctgcagaagggatgggactcaggcaggggtatgca ag gttcgctggctccagcttcctaagtggagagctttcagagcctgggcaggggttaaaa gg gcaatcccagtttcctagggaaagcagacgattctgacaggcaggacctgggaaatag at aaccctgcatgctgctgggtatttactggtctagggttctctgccaggcacacctatg gt tgtgaggccttggggggataaagttcttttttttcctgaacagagtgaagcaactggtg aa cacagaaccagtgggtccctaagcagcactcagcagaatgcagcaggcctgctggtct ct tggggtgtagagaagaccatttctcatgtacaggccgcataacaaagtataggaagta cc ttgggagagacagcaggactgccaggcaggaaggcaggggcctggtgtgtgtgtgtgt gt gggggggtatagtcagacacaagtgcagcagagggtggagaaggtcagcttggcgggg gc ccctgcgttcccagccttcttgttgaccttgggcagcagcaggacaaaggacatggcc ag ggcacagtggtagaagctgtggacgtaggtgtaatcccattcCTGTGGAGGAGAATGA GT CAGTCTGGGCCTCCATCCCTTCCCTAAACCAAGTCCTAGCCATTTGGTGCCTCTGTCA GC CAGCCCACCCTGAGAAGGTGGCAGAAAGGCTTGCTGCCTTCCTCTGTTCCATGCCTCC TG GGTGCTGGGCACCAGCTCCTGGTTCCTTCCAGGACATGCGTGCATCTTGGGTGCAGGC TT CCTAAAGTCAGGGCCTGACTTGTCCACTCAGGCAGTGAGGCTAGTACACTGGGGATGG TG AGTACCATCCTCAAGAGGACAGAATTTACAACTTGGAGCCTCCATATGTGGCTGTTAG TT AACTATTTCCAGAGGCTCTTGCTCCCCTTCCCCATAGGCCAGGTACctcaaagaagaa tc gaagcatcagggccagggccccaaagcacaggccggggcctatctgctgggtgtagat gc tcttgtcggggtacaggcccttcttctctttcatcttcttcagCTGCAGGCACAAGGT GG GGACATCAAAGTTCTTGGGGTGCAGCACAGGAAGGACCCCTCCATGAACTGTAGAAG AG CCCTACCCCCATTCCTCTGTATGCCTGACTGATGGGACTCTCTGGGCCAATTTCCCCT GG GTCCTCTACTGCCCGCATCTGGTGGGCTTTGGCACTTCAGTGGCAGACGTGATCAGTT TT |
| | CCCAGCTAAGGGGTTTTCCTCTGTTAACCTTGGTTTCATAGGCCCTGTGTGTTCAAGC TT GGTAAGATGGAGTGTTACATGGAATAGATGGGAGTCCCATGGTTCCTCACTGGAATGC AC ATCCTTGGGGCCCAAAGGTATTTTAGGTATTCAAGATTGTTCAGGTTTCAGTGGGGAA GA TCATTATAAATACCACTGTCAGGTGTGCACAGAGGGCACAGGACAGCAGCCCTGACTG AG TGATGTGCACAGTGGGCACAGGACAGCGGCCCTAATTGCACACCTCACTAAATACATT AT ATGTACAAATGCTGTCAATGGCCTCGTGCAAATCAGGGCAAGCTTTGTCACTCTGAGT GA TGATATGTTGCTGTTTCCAAGTGTTCTAAAACTTGCCATTAGTAACAGGAGTGGAGGT CC CAGTGAGCAGTGCCAGTGACATGGGCACCGCCTATTAGCCTGAGTGTAGGCCGTATGA CC ATCAATCACACAGTTCTAACACTGGGGCCCAGAGAGGAGAAGAATATTGAAGATCAC CC ATGGGCCCTGTCTTGCCCCGGGAACCCCTATTTCCCATTTCACTCAGCTTCTTCTCCC CA AATGTTGTATTCATGTTCCTTTCCTGAAAGGGTGAGACATGGGAAAGAATTGTACTCC GT TCTAAGAAGTAAGTCCAAACCACCTGCCTATCTAAGATCTAGGAGATGGGGTCTGTGC CC CAGGCATGGGTGGCTGCAGCCCCTCACTCCCATTCTCACCAGAGACCTGGGGAGGCTG GC ATTTAGTGGAGGGGGGCACTGGCACATGTATGCTATCCTGGCTAATTAAAATCCCATC AG GATGGGTGTGCTGGGCTTGGACACCAGCATTCAAGAGGCAGAGGCGGGCAGATCTCTA TG AGTTTGAAGCCATCCAGAGATACAAAGTGAGAGTCTATCTTTAAAAACAAACAAACAA AC AAACAAACAAACAAACAATCAAGTCAGATCCAGAACCAGTGAAGAGCAGCAAGGGGCC AT GATAGGCAAGACAAAGAGGCAGTTATCAGAGCAAGCCTTCTTGTTTATGCATTCCAGC TT GTTAACTAGCCATGCAGAAGCCCAACACCTCTGCCTTGGGTCAGAGAGGGCCAGCTTC GG CTCCTCAAACTGGAGTGGGATGGAAGCTTCTCCCCTCGAAAGTCAAGCACAGCTGCCA TT ACCTACTAGGGCTGCAGGTTAGGCTGCTGAGCTCTGTGCATTTCAGGTTCATCCTTAA CT TAAAATCAGAATAAGCCCGGGTTCCTCGGAGCCCACAGGAGTAGGATGTGGCTTGGAA GC TTCCTCCCTGACTATACCTGTCCCCACTTTGCTGAAGATGGATCAGAGCTCTCCCACC CC TGGCCCTGCCACTCCCCTCTGACACAGACACAGACACAGACACAGACACAGACACAGA CA CAGACACAGACACAGACACAGACACAGACACAGACACAGACACAGACAGACACAGACA CA GACAGACACAGACACAGACACAGACACAGACACAGACACAGACACAGACACAGACACA GA CACAGACACAGACACAGACACACAGGCATAGACACAGACACAGACAGACACAGACACA GA CACAGACACAGACACAGACACAGACACAGACACAGACACAGACACAGACACAGACACA GA CACAGACGACACAGACACAGACAGACACAGACACAGACAGACACAGACACAGACAGAC AC AGACACAGACACAGACACAGACGACACAGACACAGACAGACACAGACACAGACACAGA CA CAGACGACACAGACACAGACAGACACAGACACAGACACAGACACAGACACAGACACAG |
| | AC ACAGACACAGACACAGACACAGATGACACAGACACAGACGACACAGACACAGTCACAG AC ACAGACACAGACGACACAGACACAGACAGACACAGACACAGACAGACACAGACACAGA CG ACACAGACACAGACACAGACACAGACGACACAGACACAGACAGACACAGACACAGACA CA GACAGACACAGACACAGACAGACACAGACACAGACACAGACACAGACACAGACACAGA CG ACACAGACACAGACAGACACAGACACAGACACAGACACAGACATAGACACAGACACAG AG ACACAGACACAGACAACACAGACACAGACACAGACACAGACACAGACACAGACTCAGA CA CAGACACAGACACAGACACAGACACAGACACAGACTCAGACTCAGACTCAGACTCAGA CT CAGACTCAGACTCAGACTCAGACTCAGACACACAGTCACACAGACACACACAGACACA CA CAGACACACACACAAAGGCACACACACACACAAAGGCACACACACACACACACACCCC AC CGCCTGCCCCAATCTGCACTGCTGTAGCTCTACTTCCAGGAACCTGCAAGATCCCAAA TG GTGCTTCCTGCATGAGGTAGCAGACAGGTGAGAACTTGAAGCCTGAGTGCTGTCTGCT TG GTCTGAAGCCTGCTGGCCTGGAAGGTCTGTGTTTTGGGCCTAACTGCTCTGGGTGGAG CT CAGAAAACATCCCTGGGTCTTTCCTGCTATTGGGAAATTTGTTCACGATGGCTAACTT AG GTGGGTTTTAGGCCATGGAGGTGAGAGGGCCTTGAGACCATAGAGGGGTTAGGAGCCT AT ACAGCAGAGTAGATGCCAAGCGCCAGGCCCTCCTCTAGGCCTCCCACTCAATACCCTG CT TCACCCCCACCTCACACCTTCCTTCCTCATGAGAACCATTTCCAAGGCTTGCTTCTTT CG GGAAGACTATCCAGATTAACCTATCTGCTCTCCAAACTGGTATTATACCTGTAAGCAG TG TTGTCTCTCAGAATGATAATGATAGTGATCTTATGTTGATGAAAAGACTGACAGTGAC AG TCATGATGACAAAAGGTCTCCTCAGCTCTGGGTATATTAAAAATCACACCTGTGCCTG TG CCTGTGCTTAGAAGCATTCTTTATGGGTATTTGGATGCAGAGCAGAGGTCAAGAGAAA AG GAGTTTTGGCTTTATCCAGGACCAATAAACCAGCAGGGCATGGGACCCGAGCATGAGC CA CCATTTTTAGGAATTTTAGGGTTTTTGGCCCAATTCTTACTAATTCACCTGCATATAA GG ATATGGGGTATAGGACCCTACATAGGAGAAACCAAGATCAGGGAAGAAATGCAGGTTC CG TGGTCTCCGACAGTGGAGATACTGGAAGTACTCACccactttacagcaatgatgaggg tg gccgtgcctatgggaccggagtataccccgtaaccccagcggtcatgaaaagtccgca ca gcgatggtaaggacgccaagcattgtgaaggtcgatctctggggttcatcaaagtcgg cc agtgCTGGGGAGAGGCATAGCTATGGTGAGCAGCGTCCCTCACATGGCTGTGCCTCCA TC CTTGGGAACCTATTGGTATGTCCTCTCAATCTGTAGGGCCAGCCTGGTTTCCATAAGG TC TGAATTTTGGTTATTTGGAGGGAGTGGGTGATGCTGCTTCCCTGGAGCAGGGTGGCTG AA ATAAACTGGTAGACTGAGTGACCAGCATTTCCTAGGAATCCTGAGACAAAAGTGTTAA GA |
| | CTAATGATTGGTGCGCAGAGCTGAGTCTCAGGAGGGACCCCGGGACTGCATCCCTGGG AG ACAAGGGTGAGCTTGCTTGGTTTCTCCCTTTTCTCTTTCCTTCCCTTCCCTTTCTTTC CC CTTTCTTTGCTCTCTCCCTCCTTCTCTCCCTGTCTCCCTTCCTCCCTCCCTCCCTGTC TC CTTTCCTTTCTTCTTCCTTCATCCTTTCTGCTTTCTACTTTTCTCTATCTCTTCCTTC TT TTTCTTTCAAAATTTTGCAGTTGCTGGTAATGGAATGAAGGGCCTATTGATTACCAGG CG AGCGATCTGCCATTGAGCTGTATATACCCCAGGTCCAAGGTGAGGATTTTGAATGGTC TG CCTTCCTAATACACAGAGCTGAGCTGACCCATGAGGGCAAATGCTCCTCTGAGCCTGG AG GACAAGCTGGGAGGCTAGGTCCAGGATGCCTTTGGCCTCTCCTTTGTATGCTTCTGTT TT TTAAATGTCACAAGTGCTAACTACTGGAGTCACTTAAGGATGGTGGAAATGAGAGTGC AG GCATCAGAGAAATGTGCATGTCTCTTTAAGCAGATTAAGCTCTGCAAAGCAGCAAGGA GG GAGGATCTCAGAGAGGGGCTGGGTACTGGCTGGGGTTCAGGACTGGCTCCCACCCATT GG CCAAGATGGCCACTTACccatcagggagacccacatgctcagggctgttccatagatg ct gaagtactccagaatgtcacggcgcatgaagcacagcacagacaaaccaggcccatca ca ggcatgggagaaCTGTAGGGAAATCACATGAGGTCAGCAGGCAGTGGCAGCCCAGGA GT GGGTGAGAACTGGTCCCAAGGCTCAGGTTCACTAGCTGTGAGCCCCTAATGGTTTTGT AC CTCAGCCTCCTCCCTCACACTATCAGAGCCCTTGTGGAGATTAAACAGGTGAGTCCAT CT AGCCTGGGAGTGCAAAAGTCTTTGTAAATATCCCTTTCAGACTCAGCACTGGCCCAAG GC TGGTGAGAAGCATGCTCAGAAGGGCATCCTTAAAGACCACTTAcctttgcccatga ct gactgaaagtgtacacattcctatgccagtctttgcataggagccttttatcctggac cc ctgtctctccataaaagaggaagcccttagattccccccaagcaagtgctgatTCTGA CA CACTGGTTTCTTTCCCCCATATGCCAGCAGGTGTGTCCCTGACTCGTAGTTGAATAGA TT TGCTTCTAAGCAAAAGGTTCTATATGCAGGATTTCCAAGCAGACAACTTATTTCTTGC AG AAAACAACTTGCTCTCCCTTTGCTTCACATTTCATCATTTTAAGTAATATTTAATTAC AT GACATAATTATTTTGACAAGTGCAACTGGCACAAACAAGCCCAGCAGCCAGCACAATG AG CTCTTGGTAAGCCCAACTTAGCAGGAGGGAGCAGGCAAGCTGGAAAACAGCCTTGTCT GG AGGCAGCAGGGGCACCACCGAGGGAGGCAGGCGGAGAGCTGGGGACCCTGGATGATGG AT GTATCAGTCAAGCACATAGGGCCTACTTAGAAGCTCAGAGACCTCCTGCTGGTCACAG TT GCACATGGACTCTGTCAATCAATAGAGAGCATCCAGGGGAAGGAGGAGGTGGTCCAG CC TCTGTGTTGGGTCAGCCCCAGCCTTGAGCTTTGGGTTCTGCACCTTTTAAAAGGGAGA TT GGTGAAGAGGAGGTTAACCAACTAGGTATGAGCTCAGGAAAAGACAAGCTTTGGGTTG GG CCAGACCAAGGTACGCAAGTGGAGAAGGAAAGGAACTCAGCTCTGGGAGGGGACTCTG |
| | CT CTTCTGGCTCCTTACAGAACCACATGACCCCACCCCACCCCACCTGAGCCCATCATCT GT AGCATCTTGCTTCCTTCTCTTGTATAGGCCCCCATGAATGAGTAAAACTTACTTACTG TG AGATCCTGGGAAACACTCATGCCTTCCCCCACGAGGAGAAGAGCTTCCTTAGGCTTGA TC TCAACATAGAATACTTGGCTACATGTGAAGGCCAGAGGAGCAGGCTTTCTAACAAGGG AT CTAACTGTCCTCAGGCCCTGAGGATTAATTTTTTGGGGGGTGGGTGACCTGTGTGACA GT GAACTTCCCTGGGGAACCTCCTGCCCAAGGAGGCAGGGGCAAGGCTGTGATGTGTACC CT TTCTCCCCAGAGGCAGGGAGATCTGGTCCAGCTGGTGCCAGGCTAGGACACAGCTGGG TG TGACAGGAGCCCTAACCCTGCTGTCAGCTCAGAGCTGGCAGAGGGGCCCAGGTTCTCT CA GGTCTCTCAGGCCCACCTTGTCTAATGGCATGAGAACACCTGTTCTGTGGGGCTTACA AG GGGACCCTAACGATAACTGCGGAGCATGGCACCCCACACTGCAAAAATGAAATGCTGT TT AAAGTTTGCTTTCATTAATCAAACTTTCCCCCAACCTGAAACCAAGTTAATATGTGCG TT ATGGGCATTTAAACAATGTGCTTGCCCTGGGCAGAATTAGCTCACCTCTGGGAAAAAC AA TTCAATCGATCTTATTATGCTTTGCATTTCTGGTGGAGGACTCTAGTGAGTCTTTGTG AC TCTTTCATGCCCGACTCAGAACAGTATATGTTTGTGTGAGATGTGGTGACCAGGTCTA AG ACCACGTGTGTTAGAAACAGCAAGGTATGGAGACCATGTTGAAAGCAAAATGTGGGTG TA GGCTGATAATATCTGATTGTGGATTTGTGTGCTACTGAGTCAAAGGGCCAGAGAGACA GC TGTCTGCTATAAAAGCCTAAGACTCAGATCCCATTCTTTTTGTCCCTGTTTGTTGTGC TG TTCAGCAAGTAGAAAGGATGATATTGTCTAAGATTCTTAGATTAGAACCTGATTTTAG AT TAGATGACTATCAGGTTAGAACAGGAGAGGGCAGAATTCTTTGGAATACATCAGATCC AC CCGCTGTGTAACTGACACCAAGAGTCATTCTTCTATTCAGCAGCAGCATACCATACAA CT GGTAGTTGTCATGGAGAGTCCTACAGCAGCCACGTGGAAGGCAGAACTCTGTGAGGAA CA GATTGTGGCTTTGAGGCCAGAGGACATTTGTCATAAGAGACAGCTGGCCCTGCCACTC TG GGTGGGGTGTGGCAGGGTGGGCCTCCAAGGCCAGTGCAGAGGCAGCTGTAGGCCAATT AG ACCCAGGCAGGCAGGGGTGACCTGATTGGGGCTGTGATTTGCTGGACTGTATCTAACA CA GGCCTTGGGAACAAGACCCTGGCTTATGTCCTTGACCGTGGGGTCTCATCTTGGCTCT GA CCTTGGCCAGGTCTCAAGAGGAACAAATGACAGTGTGGGACAAAGTACTGTGGGGCAG AC CAGGATCTGAGTGTTCATGGTGACACTGGTGGCCCAGTTTCTCTGAGACTCAGTTTCC TC TTCTATCAAATTGAAATCACTATGTTAGGCTCGTGGGTGATAATGAGTCCAACCCCAC CA TGGTTGCTTTCTTGTGACTTATCATTGGCCTAATGTCCTCCCCTACTGAAGTGAACTC AA GAGCCATAGAGTTTCCAGTTCCTTGGGTTACCTATGGGACCACCACAACCAGGAGGTA GA |
| | CAGGTGCCAAGCCCTCCCCCACTGTTCTCAGCCCACATGCATTGTGGCTTCTCCCACC AC TAGAAAGTCATGCCAGCTGACTCAGGATATGGAACACGCATGTGAGCACAGATGTGTG AG TTTGTGGGCTCACTCATTGAGAGCCAGCTGGATACCTTCACATACTCTATGCCCTTGC CT TACTGAGACCTGCTGCAGGAAGGGCAGGCCTAAGGAGAGGATGCTAGTCTCTAAAAGT TT GGCTCTGCTCTAAGGAGGAGACTAGCAGGCTGCTTGCCAACCCTGAGCATGTATCCTA CC AGTGTGTGGGCCTCACACCAGACAAACTAGTGAGGCATAGTGTGATGAGAGAGAAACG AA GGTTACAGAGTGGTAAAAGAGACAGTGTGACTCCTGGTTAGAGGATAGCTGAGAGGGC CA TCATGAGAGGTACTCAGAAGGACTAAAGGGCAAAGTGAGAGGAGGCCTTTAAGACAGA GA GTAGATGGGTAGATGAATGGACAGGGAGAGAGATGGTTGGTTAGCAGATAATAGAGAA AT GATAGACAGATAGACAGACAGACAGATGATGGATAGACACATAGAACAAGACAAATGA TA AATGAATAGATGATAGACAAAGGAGATAGAGAGACAGAAGCAAGTTGAATGGGCAGGA AG ATAAAGTCAGGAAGACACAGAGCTCTGGTCAAGAACCCAGGGGAGAGCAGACCAGGGA GA AGAGGAGAGTGAACTCCTCGGGGGAGTGTAACTCTAGAAATCAGAAAAAAACAAAAAA AA AAAACCCCAAAAAACAAACAAACAAACAAACAAAAAAATTGGATACAGGCAGGAAGAA GG AAGAGATAGAGACTGGGAGAAACTAGACACAGAACCAGTCAAAGAAGCAGAGGGAGAG AG ACCCATGGCGGGAATAAAGAGAAGCAGAAACCCAGACACAAGGCTTCAGCAAAGCTGG GC CAGTGCCAGACATGCCCCGAACGAACGACAGAGGAGTCACCCAGTACTGTTGCCTGGG AA CAGAGTGGAGAAGGAACTAAGAGGCAGCCAGCCAGCTAGACACATAACAGGAAGAGAA AG AAGGACTCAGGGAGAGGCTGGCTCCTCTCAGTGGGGGTAGTTCCAAATTCTGGAGCTG CA GTCACCCAGGCCCTCTACCTTTCCTGAACCTAGTAGATCCATTCCTAGGCCTGCTCAC TC ACCTTGTTCCTCCTCAGCTGAGCAACTCATGGAACAACGTTGGTAGAAAGGAGAGAGA GT CTGAGGAGCACCAGGCTTGACCTTAACTGACACCGGGCTCTCATGGGCCTGGCCTCAG TC TCAGGTGTCAATCACCCCCCTCAAATGTCTGGCGCACATGGAGAAACTGAGGTCCACA GA GGAAGACAGATTCCAGGAACCTTCTCTTCCCAGTCACCACCCCCACTGCTCCCCCAGA CC CAGACTCTTTCTCTTCCAAATCCTGTTTCTGCATCACCTGGCACAGGACAATGGTGGT AA CCCTCCCGTGAGGACTTCCTCCTAATTTCCTCCTTCCACACTTACcgccacaaagaac at ggtgaagaggtagaccatggcctccatgtagaaacgcctcttggtagcgatgctcact gt cgggaggaaggccaggctgctgagggtaggcaggagcagtttggctacaactgtcccc at ggaccaggaggaaggcactgactggggagaaggtggtaaaggccccccctggtctccag gg caggaagaaaaagagcccacttctttgcttctccagcagccctgaccgcagctgtggc ag |
| | cacccacaaggagggcttaagtgctc (SEQ ID NO: 64) |
| Mouse (- strand, reverse complement) - start codon is bold & underlined; stop codon is bold and italicized | gagcacttaagccctccttgtgggtgctgccacagctgcggtcagggctgctggagaa gc aaagaagtgggctcttttcttcctgccctggagaccagggggcctttaccaccttc tc cccagtcagtgccttcctcctggtccatggggacagttgtagccaaactgctcctgcc ta ccctcagcagcctggccttcctcccgacagtgagcatcgctaccaagaggcgtttcta ca tggaggccatggtctacctcttcaccatgttctttgtggcgGTAAGTGTGGAAGGAGG AA ATTAGGAGGAAGTCCTCACGGGAGGGTTACCACCATTGTCCTGTGCCAGGTGATGCAG AA ACAGGATTTGGAAGAGAAAGAGTCTGGGTCTGGGGAGCAGTGGGGGTGGTGACTGGG AA GAGAAGGTTCCTGGAATCTGTCTTCCTCTGTGGACCTCAGTTTCTCCATGTGCGCCAG AC ATTTGAGGGGGGTGATTGACACCTGAGACTGAGGCCAGGCCCATGAGAGCCCGGTGTC AG TTAAGGTCAAGCCTGGTGCTCCTCAGACTCTCTCTCCTTTCTACCAACGTTGTTCCAT GA GTTGCTCAGCTGAGGAGGAACAAGGTGAGTGAGCAGGCCTAGGAATGGATCTACTAGG TT CAGGAAAGGTAGAGGGCCTGGGTGACTGCAGCTCCAGAATTTGGAACTACCCCCACTG AG AGGAGCCAGCCTCTCCCTGAGTCCTTCTTTCTCTTCCTGTTATGTGTCTAGCTGGCTG GC TGCCTCTTAGTTCCTTCTCCACTCTGTTCCCAGGCAACAGTACTGGGTGACTCCTCTG TC GTTCGTTCGGGGCATGTCTGGCACTGGCCCAGCTTTGCTGAAGCCTTGTGTCTGGGTT TC TGCTTCTCTTTATTCCCGCCATGGGTCTCTCTCCCTCTGCTTCTTTGACTGGTTCTGT GT CTAGTTTCTCCCAGTCTCTATCTCTTCCTTCTTCCTGCCTGTATCCAATTTTTTTGTT TG TTTGTTTGTTTGTTTTTTGGGGTTTTTTTTTTTTGTTTTTTTCTGATTTCTAGAGTTA CA CTCCCCCGAGGAGTTCACTCTCCTCTTCTCCCTGGTCTGCTCTCCCCTGGGTTCTTGA CC AGAGCTCTGTGTCTTCCTGACTTTATCTTCCTGCCCATTCAACTTGCTTCTGTCTCTC TA TCTCCTTTGTCTATCATCTATTCATTTATCATTTGTCTTGTTCTATGTGTCTATCCAT CA TCTGTCTGTCTGTCTATCTGTCTATCATTTCTCTATTATCTGCTAACCAACCATCTCT CT CCCTGTCCATTCATCTACCCATCTACTCTCTGTCTTAAAGGCCTCCTCTCACTTTGCC CT TTAGTCCTTCTGAGTACCTCTCATGATGGCCCTCTCAGCTATCCTCTAACCAGGAGTC AC ACTGTCTCTTTTACCACTCTGTAACCTTCGTTTCTCTCTCATCACACTATGCCTCACT AG TTTGTCTGGTGTGAGGCCCACACACTGGTAGGATACATGCTCAGGGTTGGCAAGCAGC CT GCTAGTCTCCTCCTTAGAGCAGAGCCAAACTTTTAGAGACTAGCATCCTCTCCTTAGG CC TGCCCTTCCTGCAGCAGGTCTCAGTAAGGCAAGGGCATAGAGTATGTGAAGGTATCCA GC TGGCTCTCAATGAGTGAGCCCACAAACTCACACATCTGTGCTCACATGCGTGTTCCAT AT CCTGAGTCAGCTGGCATGACTTTCTAGTGGTGGGAGAAGCCACAATGCATGTGGGCTG |
| | AG AACAGTGGGGGAGGGCTTGGCACCTGTCTACCTCCTGGTTGTGGTGGTCCCATAGGTA AC CCAAGGAACTGGAAACTCTATGGCTCTTGAGTTCACTTCAGTAGGGGAGGACATTAGG CC AATGATAAGTCACAAGAAAGCAACCATGGTGGGGTTGGACTCATTATCACCCACGAGC CT AACATAGTGATTTCAATTTGATAGAAGAGGAAACTGAGTCTCAGAGAAACTGGGCCAC CA GTGTCACCATGAACACTCAGATCCTGGTCTGCCCCACAGTACTTTGTCCCACACTGTC AT TTGTTCCTCTTGAGACCTGGCCAAGGTCAGAGCCAAGATGAGACCCCACGGTCAAGGA CA TAAGCCAGGGTCTTGTTCCCAAGGCCTGTGTTAGATACAGTCCAGCAAATCACAGCCC CA ATCAGGTCACCCCTGCCTGCCTGGGTCTAATTGGCCTACAGCTGCCTCTGCACTGGCC TT GGAGGCCCACCCTGCCACACCCCACCCAGAGTGGCAGGGCCAGCTGTCTCTTATGACA AA TGTCCTCTGGCCTCAAAGCCACAATCTGTTCCTCACAGAGTTCTGCCTTCCACGTGGC TG CTGTAGGACTCTCCATGACAACTACCAGTTGTATGGTATGCTGCTGCTGAATAGAAGA AT GACTCTTGGTGTCAGTTACACAGCGGGTGGATCTGATGTATTCCAAAGAATTCTGCCC TC TCCTGTTCTAACCTGATAGTCATCTAATCTAAAATCAGGTTCTAATCTAAGAATCTTA GA CAATATCATCCTTTCTACTTGCTGAACAGCACAACAAACAGGGACAAAAAGAATGGGA TC TGAGTCTTAGGCTTTTATAGCAGACAGCTGTCTCTCTGGCCCTTTGACTCAGTAGCAC AC AAATCCACAATCAGATATTATCAGCCTACACCCACATTTTGCTTTCAACATGGTCTCC AT ACCTTGCTGTTTCTAACACACGTGGTCTTAGACCTGGTCACCACATCTCACACAAACA TA TACTGTTCTGAGTCGGGCATGAAAGAGTCACAAAGACTCACTAGAGTCCTCCACCAGA AA TGCAAAGCATAATAAGATCGATTGAATTGTTTTTCCCAGAGGTGAGCTAATTCTGCCC AG GGCAAGCACATTGTTTAAATGCCCATAACGCACATATTAACTTGGTTTCAGGTTGGGG GA AAGTTTGATTAATGAAAGCAAACTTTAAACAGCATTTCATTTTTGCAGTGTGGGGTGC CA TGCTCCGCAGTTATCGTTAGGGTCCCCTTGTAAGCCCCACAGAACAGGTGTTCTCATG CC ATTAGACAAGGTGGGCCTGAGAGACCTGAGAGAACCTGGGCCCCTCTGCCAGCTCTGA GC TGACAGCAGGGTTAGGGCTCCTGTCACACCCAGCTGTGTCCTAGCCTGGCACCAGCTG GA CCAGATCTCCCTGCCTCTGGGGAGAAAGGGTACACATCACAGCCTTGCCCCTGCCTCC TT GGGCAGGAGGTTCCCCAGGGAAGTTCACTGTCACACAGGTCACCCACCCCCCAAAAAA TT AATCCTCAGGGCCTGAGGACAGTTAGATCCCTTGTTAGAAAGCCTGCTCCTCTGGCCT TC ACATGTAGCCAAGTATTCTATGTTGAGATCAAGCCTAAGGAAGCTCTTCTCCTCGTGG GG GAAGGCATGAGTGTTTCCCAGGATCTCACAGTAAGTAAGTTTTACTCATTCATGGGGG CC TATACAAGAGAAGGAAGCAAGATGCTACAGATGATGGGCTCAGGTGGGGTGGGGTGGG GT |
| | CATGTGGTTCTGTAAGGAGCCAGAAGAGCAGAGTCCCCTCCCAGAGCTGAGTTCCTTT CC TTCTCCACTTGCGTACCTTGGTCTGGCCCAACCCAAAGCTTGTCTTTTCCTGAGCTCA TA CCTAGTTGGTTAACCTCCTCTTCACCAATCTCCCTTTTAAAAGGTGCAGAACCCAAAG CT CAAGGCTGGGGCTGACCCAACACAGAGGCTGGACCACCTCCTCCCTTCCCCTGGATGC TC TCTATTGATTGACAGAGTCCATGTGCAACTGTGACCAGCAGGAGGTCTCTGAGCTTCT AA GTAGGCCCTATGTGCTTGACTGATACATCCATCATCCAGGGTCCCCAGCTCTCCGCCT GC CTCCCTCGGTGGTGCCCCTGCTGCCTCCAGACAAGGCTGTTTTCCAGCTTGCCTGCTC CC TCCTGCTAAGTTGGGCTTACCAAGAGCTCATTGTGCTGGCTGCTGGGCTTGTTTGTGC CA GTTGCACTTGTCAAAATAATTATGTCATGTAATTAAATATTACTTAAAATGATGAAAT GT GAAGCAAAGGGAGAGCAAGTTGTTTTCTGCAAGAAATAAGTTGTCTGCTTGGAAATCC TG CATATAGAACCTTTTGCTTAGAAGCAAATCTATTCAACTACGAGTCAGGGACACACCT GC TGGCATATGGGGGAAAGAAACCAGTGTGTCAGAatcagcacttgcttgggggggaatct aa gggcttcctcttttatggagagacaggggtccaggataaaaggctcctatgcaaagac tg gcataggaatgtgtacactttcagtcagtcatgggcaaaggtGTAAGTGGTCTTTAAG GA TGCCCTTCTGAGCATGCTTCTCACCAGCCTTGGGCCAGTGCTGAGTCTGAAAGGGATA TT TACAAAGACTTTTGCACTCCCAGGCTAGATGGACTCACCTGTTTAATCTCCACAAGGG CT CTGATAGTGTGAGGGAGGAGGCTGAGGTACAAAACCATTAGGGGCTCACAGCTAGTGA AC CTGAGCCTTGGGACCAGTTCTCACCCACTCCTGGGCTGCCCACTGCCTGCTGACCTCA TG TGATTTCCCTACAGttctcccatgcctgtgatgggcctggtttgtctgtgctgtgctt ca tgcgccgtgacattctggagtacttcagcatctatggaacagccctgagcatgtgggt ct ccctgatggGTAAGTGGCCATCTTGGCCAATGGGTGGGAGCCAGTCCTGAACCCCAGC CA GTACCCAGCCCCTCTCTGAGATCCTCCCTCCTTGCTGCTTTGCAGAGCTTAATCTGCT TA AAGAGACATGCACATTTCTCTGATGCCTGCACTCTCATTTCCACCATCCTTAAGTGAC TC CAGTAGTTAGCACTTGTGACATTTAAAAAACAGAAGCATACAAAGGAGAGGCCAAAGG CA TCCTGGACCTAGCCTCCCAGCTTGTCCTCCAGGCTCAGAGGAGCATTTGCCCTCATGG GT CAGCTCAGCTCTGTGTATTAGGAAGGCAGACCATTCAAAATCCTCACCTTGGACCTGG GG TATATACAGCTCAATGGCAGATCGCTCGCCTGGTAATCAATAGGCCCTTCATTCCATT AC CAGCAACTGCAAAATTTTGAAAGAAAAAGAAGGAAGAGATAGAGAAAAGTAGAAAGCA GA AAGGATGAAGGAAGAAGAAAGGAAAGGAGACAGGGAGGGAGGGAGGAAGGGAGACAGG GA GAGAAGGAGGGAGAGAGCAAAGAAAGGGGAAAGAAAGGGAAGGGAAGGAAAGAGAAAA GG GAGAAACCAAGCAAGCTCACCCTTGTCTCCCAGGGATGCAGTCCCGGGGTCCCTCCTG |
| | AG ACTCAGCTCTGCGCACCAATCATTAGTCTTAACACTTTTGTCTCAGGATTCCTAGGAA AT GCTGGTCACTCAGTCTACCAGTTTATTTCAGCCACCCTGCTCCAGGGAAGCAGCATCA CC CACTCCCTCCAAATAACCAAAATTCAGACCTTATGGAAACCAGGCTGGCCCTACAGAT TG AGAGGACATACCAATAGGTTCCCAAGGATGGAGGCACAGCCATGTGAGGGACGCTGCT CA CCATAGCTATGCCTCTCCCCAGcactggccgactttgatgaaccccagagatcgacct tc acaatgcttggcgtccttaccatcgctgtgcggacttttcatgaccgctggggttacg gg gtatactccggtcccataggcacggccaccctcatcattgctgtaaagtggGTGAGTA CT TCCAGTATCTCCACTGTCGGAGACCACGGAACCTGCATTTCTTCCCTGATCTTGGTTT CT CCTATGTAGGGTCCTATACCCCATATCCTTATATGCAGGTGAATTAGTAAGAATTGGG CC AAAAACCCTAAAATTCCTAAAAATGGTGGCTCATGCTCGGGTCCCATGCCCTGCTGGT TT ATTGGTCCTGGATAAAGCCAAAACTCCTTTTCTCTTGACCTCTGCTCTGCATCCAAAT AC CCATAAAGAATGCTTCTAAGCACAGGCACAGGCACAGGTGTGATTTTTAATATACCCA GA GCTGAGGAGACCTTTTGTCATCATGACTGTCACTGTCAGTCTTTTCATCAACATAAGA TC ACTATCATTATCATTCTGAGAGACAACACTGCTTACAGGTATAATACCAGTTTGGAGA GC AGATAGGTTAATCTGGATAGTCTTCCCGAAAGAAGCAAGCCTTGGAAATGGTTCTCAT GA GGAAGGAAGGTGTGAGGTGGGGGTGAAGCAGGGTATTGAGTGGGAGGCCTAGAGGAGG GC CTGGCGCTTGGCATCTACTCTGCTGTATAGGCTCCTAACCCCTCTATGGTCTCAAGGC CC TCTCACCTCCATGGCCTAAAACCCACCTAAGTTAGCCATCGTGAACAAATTTCCCAAT AG CAGGAAAGACCCAGGGATGTTTTCTGAGCTCCACCCAGAGCAGTTAGGCCCAAAACAC AG ACCTTCCAGGCCAGCAGGCTTCAGACCAAGCAGACAGCACTCAGGCTTCAAGTTCTCA CC TGTCTGCTACCTCATGCAGGAAGCACCATTTGGGATCTTGCAGGTTCCTGGAAGTAGA GC TACAGCAGTGCAGATTGGGGCAGGCGGTGGGGTGTGTGTGTGTGTGTGCCTTTGTG TG TGTGTGTGCCTTTGTGTGTGTGTCTGTGTGTGTCTGTGTGTGTCTGTGTGACTGTGTG TC TGAGTCTGAGTCTGAGTCTGAGTCTGAGTCTGAGTCTGAGTCTGAGTCTGAGTCTGTG TC TGTGTCTGTGTCTGTGTCTGTGTCTGTGTCTGAGTCTGTGTCTGTGTCTGTGTCTGTG TC TGTGTCTGTGTTGTCTGTGTCTGTGTCTCTGTGTCTGTGTCTATGTCTGTGTCTGTGT CT GTGTCTGTGTCTGTCTGTGTCTGTGTCGTCTGTGTCTGTGTCTGTGTCTGTGTCTGTG TC TGTGTCTGTCTGTGTCTGTGTCTGTCTGTGTCTGTGTCTGTGTCTGTCTGTGTCTGTG TC GTCTGTGTCTGTGTCTGTGTCTGTGTCGTCTGTGTCTGTGTCTGTCTGTGTCTGTGTC TG TCTGTGTCTGTGTCGTCTGTGTCTGTGTCTGTGACTGTGTCTGTGTCGTCTGTGTCTG TG |
| | TCATCTGTGTCTGTGTCTGTGTCTGTGTCTGTGTCTGTGTCTGTGTCTGTGTCTGTGTCTGTGT CT GTGTCTGTCTGTGTCTGTGTCGTCTGTGTCTGTGTCTGTGTCTGTGTCTGTCTGTGTC TG TGTCGTCTGTGTCTGTGTCTGTGTCTGTGTCTGTCTGTGTCTGTGTCTGTCTGTGTCT GT GTCTGTCTGTGTCTGTGTCGTCTGTGTCTGTGTCTGTGTCTGTGTCTGTGTCTGTGTC TG TGTCTGTGTCTGTGTCTGTGTCTGTGTCTGTGTCTGTGTCTGTCTGTGTCTGTGTCTA TG CCTGTGTGTCTGTGTCTGTGTCTGTGTCTGTGTCTGTGTCTGTGTCTGTGTCTGTGTC TG TGTCTGTGTCTGTGTCTGTGTCTGTCTGTGTCTGTGTCTGTCTGTGTCTGTGTCTGTG TC TGTGTCTGTGTCTGTGTCTGTGTCTGTGTCTGTGTCTGTGTCTGTGTCTGTGTCTGTG TC TGTGTCAGAGGGGAGTGGCAGGGCCAGGGGTGGGAGAGCTCTGATCCATCTTCAGCAA AG TGGGGACAGGTATAGTCAGGGAGGAAGCTTCCAAGCCACATCCTACTCCTGTGGGCTC CG AGGAACCCGGGCTTATTCTGATTTTAAGTTAAGGATGAACCTGAAATGCACAGAGCTC AG CAGCCTAACCTGCAGCCCTAGTAGGTAATGGCAGCTGTGCTTGACTTTCGAGGGGAGA AG CTTCCATCCCACTCCAGTTTGAGGAGCCGAAGCTGGCCCTCTCTGACCCAAGGCAGAG GT GTTGGGCTTCTGCATGGCTAGTTAACAAGCTGGAATGCATAAACAAGAAGGCTTGCTC TG ATAACTGCCTCTTTGTCTTGCCTATCATGGCCCCTTGCTGCTCTTCACTGGTTCTGGA TC TGACTTGATTGTTTGTTTGTTTGTTTGTTTGTTTGTTTGTTTTTAAAGATAGACTCTC AC TTTGTATCTCTGGATGGCTTCAAACTCATAGAGATCTGCCCGCCTCTGCCTCTTGAAT GC TGGTGTCCAAGCCCAGCACACCCATCCTGATGGGATTTTAATTAGCCAGGATAGCATA CA TGTGCCAGTGCCCCCCTCCACTAAATGCCAGCCTCCCCAGGTCTCTGGTGAGAATGGG AG TGAGGGGCTGCAGCCACCCATGCCTGGGGCACAGACCCCATCTCCTAGATCTTAGATA GG CAGGTGGTTTGGACTTACTTCTTAGAACGGAGTACAATTCTTTCCCATGTCTCACCCT TT CAGGAAAGGAACATGAATACAACATTTGGGGAGAAGAAGCTGAGTGAAATGGGAAATA GG GGTTCCCGGGGCAAGACAGGGCCCATGGGTGATCTTCAATATTCTTCTCCTCTCTGGG GC CCCAGTGTTAGAACTGTGTGATTGATGGTCATACGGCCTACACTCAGGCTAATAGGCG GT GCCCATGTCACTGGCACTGCTCACTGGGACCTCCACTCCTGTTACTAATGGCAAGTTT TA GAACACTTGGAAACAGCAACATATCATCACTCAGAGTGACAAAGCTTGCCCTGATTTG CA CGAGGCCATTGACAGCATTTGTACATATAATGTATTTAGTGAGGTGTGCAATTAGGGC CG CTGTCCTGTGCCCACTGTGCACATCACTCAGTCAGGGCTGCTGTCCTGTGCCCTCTGT GC ACACCTGACAGTGGTATTTATAATGATCTTCCCCACTGAAACCTGAACAATCTTGAAT AC CTAAAATACCTTTGGGCCCCAAGGATGTGCATTCCAGTGAGGAACCATGGACTCCCA TC TATTCCATGTAACACTCCATCTTACCAAGCTTGAACACACAGGGCCTATGAAACCAAG |
| | GT TAACAGAGGAAAACCCCTTAGCTGGGAAAACTGATCACGTCTGCCACTGAAGTGCCAA AG CCCACCAGATGCGGGCAGTAGAGGACCCAGGGGAAATTGGCCCAGAGAGTCCCATCAG TC AGGCATACAGAGGAATGGGGGTAGGGCTCTTCTACAGTTCATGGAGGGGTCCCTTCCT GT GCTGCACCCCAAGAACTTTGATGTCCCCACCTTGTGCCTGCAGctgaagaagatgaaa ga gaagaagggcctgtaccccgacaagagcatctacacccagcagataggccccggcctg tg ctttggggccctggccctgatgcttcgattcttctttgagGTACCTGGCCTATGGGGA AG GGGAGCAAGAGCCTCTGGAAATAGTTAACTAACAGCCACATATGGAGGCTCCAAGTTG TA AATTCTGTCCTCTTGAGGATGGTACTCACCATCCCCAGTGTACTAGCCTCACTGCCTG AG TGGACAAGTCAGGCCCTGACTTTAGGAAGCCTGCACCCAAGATGCACGCATGTCCTGG AA GGAACCAGGAGCTGGTGCCCAGCACCCAGGAGGCATGGAACAGAGGAAGGCAGCAAGC CT TTCTGCCACCTTCTCAGGGTGGGCTGGCTGACAGAGGCACCAAATGGCTAGGACTTGG TT TAGGGAAGGGATGGAGGCCCAGACTGACTCATTCTCCTCCACAGgaatgggattacac ct acgtccacagcttctaccactgtgccctggccatgtcctttgtcctgctgctgcccaa gg tcaacaagaaggctgggaacgcaggggcccccgccaagctgaccttctccaccctctg ct gcacttgtgtctgactatacccccccacacacacacacacaccaggcccctgccttcc tg cctggcagtcctgctgtctctcccaaggtacttcctatactttgttatgcggcctgta ca tgagaaatggtcttctctacaccccaagagaccagcaggcctgctgcattctgctgag tg ctgcttagggacccactggttctgtgttcaccagttgcttcactctgttcaggaaaaa aa agaactttatcccccaaggcctcacaaccataggtgtgcctggcagagaaccctagac ca gtaaatacccagcagcatgcagggttatctatttcccaggtcctgcctgtcagaatcg tc tgctttccctaggaaactgggattgcccttttaacccctgcccaggctctgaaagctc tc cacttaggaagctggagccagcgaaccttgcataccccctgcctgagtcccatcccttc tg caggttttcgatcaagccattcacagtaaactcttgatcagccactagtcattagctt cg cctaacatacattctagttcctggaa (SEQ ID NO: 65) |

One or more modifications, in some instances, can include an insertion, a deletion, a substitution, or combinations thereof. In some examples, the polypeptide does not encompass one or more naturally occurring polypeptides (e.g., does not encompass one or more of the wt-myomaker polypeptides). In other examples, the polypeptide does not encompass any of the wt-myomaker polypeptides. In some examples, the polypeptide does not encompass any naturally occurring polypeptide (e.g., does not encompass any of the wt-myomaker polypeptides or any other naturally occurring polypeptide).

In some examples, one or more modifications to a wt-myomaker polypeptide can include one or more substitutions, one or more insertions, or one or more deletions (or combinations thereof) to one or more amino acids in a hydrophobic region of a wt-myomaker polypeptide, to one or more amino acids in a hydrophilic region of a wt-myomaker polypeptide, or in a combination thereof. In some examples, one or more modifications to a wt-myomaker polypeptide can include one or more substitutions or one or more deletions (or combinations thereof) to one or more amino acids in a hydrophobic region of a wt-myomaker polypeptide, to one or more amino acids in a hydrophilic region of a wt-myomaker polypeptide, or in a combination thereof.

In some examples, the myomaker polypeptide can have a polypeptide sequence with an amino acid sequence identity to a wt-myomaker polypeptide (e.g., SEQ ID NO:50 or SEQ ID NO:53) of about 70%, about 75%, about 80%, about 85%, about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 70%, at least about 80%, at least about 90%, at least about 95%, at least about 99%, or at least about 99.5%. In some embodiments, the myomaker polypeptide sequence has an amino acid sequence identity to SEQ ID NO:50 or SEQ ID NO:53 of about 70%, about 75%, about 80%, about 85%, about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 70%, at least about 80%, at least about 90%, at least about 95%, at least about 99%, or at least about 99.5%. The amino acid sequence identity (e.g., percent identity) can be determined by any suitable method, such as using BLAST, BLAST-2, ALIGN, ALIGN-2, Clustal Omega, or Megalign software. Unless otherwise indicated, the amino acid sequence identity (e.g., percent identity) is determined using BLAST-2.

Nucleic acid molecules that encode for the myomaker polypeptide are termed "myomaker nucleic acid molecules." In certain examples, the myomaker nucleic acid molecule is included in a vector (e.g., a viral vector, a retroviral vector, a lentiviral vector, an adenoviral vector, an adeno-associated viral vector, a herpesviral vector, a chimeric viral vector, a plasmid, an expression vector, a conjugative vector, or a nonconjugative vector). In certain examples, the myomerger nucleic acid molecule is in the same vector as the myomaker nucleic acid molecule. In certain examples, the myomaker nucleic acid molecule is in a cell, such as an insect cell (e.g., an Sf9 cell) or mammalian cell (e.g., a human cell, a rat cell a mouse cell, a muscle cell, a non-muscle cell, a myoblast, a fibroblast, a C2C12 cell, a 10T ½ fibroblast, a NIH/3T3 cell, a CHO cell, a mesenchymal stem cell (MSC), a hematopoietic stem cell, a blood cell, a bone marrow cell, or an adipose stem cell). In certain examples, the myomerger nucleic acid molecule is in the same cell as the myomaker nucleic acid molecule.

In other examples, the myomaker nucleic acid molecule comprises one or more nucleic acid sequences that are not used to encode for the myomaker polypeptide (e.g., one or more introns). For example, the myomaker nucleic acid molecule can include one or more nucleic acid molecules as found in nature (e.g., including introns). In certain examples, the myomaker nucleic acid molecule differs from the one or more nucleic acid molecules in nature because the myomaker nucleic acid molecule does not include one or more introns. In some examples, the myomaker nucleic acid molecule is a cDNA molecule ("myomaker cDNA molecule"). In certain examples, the myomaker cDNA molecule is identical to a nucleic acid molecule found in nature. In other examples, the myomaker cDNA molecule is not identical to a nucleic acid molecule found in nature (e.g., due to the myomaker cDNA molecule not including one or more introns in the nucleic acid molecule found in nature).

In some examples, the myomaker nucleic acid molecule sequence has a sequence identity to a nucleic acid molecule encoding a wt-myomaker polypeptide (e.g., SEQ ID NO:56, SEQ ID NO:59, SEQ ID NO:62, SEQ ID NO:63, SEQ ID NO:64, or SEQ ID NO:65) of about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 90%, at least about 95%, at least about 99%, or at least about 99.5%. In some examples, the myomaker nucleic acid molecule sequence has a sequence identity to SEQ ID NO:56, SEQ ID NO:59, SEQ ID NO:62, SEQ ID NO:63, SEQ ID NO:64, or SEQ ID NO:65 of about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, about 99.9%, about 99.95%, about 99.99%, less than about 100%, at least about 90%, at least about 95%, at least about 99%, or at least about 99.5%. Nonlimiting examples of wt-myomaker polypeptides and wt-myomaker nucleic acid molecules can be found in Table 2. The nucleic acid sequence identity (e.g., percent identity) can be determined by any suitable method, such as using BLAST, BLAST-2, ALIGN, ALIGN-2, Clustal Omega, CRISPor Megalign software. Unless otherwise indicated, the nucleic acid sequence identity (e.g., percent identity) is determined using BLAST-2.

In some examples, the myomaker nucleic acid molecule encodes for a myomaker polypeptide that has one or more modifications to wt-myomaker polypeptide in a hydrophobic region, in a hydrophilic region, or in a combination thereof.

The myomaker nucleic acid molecule can be made using any suitable technique, such as but not limited to, those found in WO 2014/210448 A1, chemical synthesis, enzymatic production or biological production. Chemical synthesis of a nucleic acid molecule can include, for example, a nucleic acid molecule made by *in vitro* chemical synthesis using phosphotriester, phosphite or phosphoramidite chemistry and solid phase techniques, or via deoxynucleoside H-phosphonate intermediates. Enzymatically produced nucleic acid molecules can be accomplished using any suitable method including but not limited to Polymerase Chain Reaction (PCR). Biologically produced nucleic acid molecules can be accomplished using any suitable method including but not limited to a recombinant nucleic acid produced (i.e., replicated) in a living cell, such as a recombinant DNA vector replicated in bacteria.

Modifications or changes made in the structure of the myomaker nucleic acid molecules and/or myomaker polypeptides can be used. In certain examples, a myomaker polypeptide can be modified (e.g., by one or more insertions, one or more deletions, or one or more substitutions (e.g., conservative substitutions)). In some examples, the myomaker polypeptide which was modified does not have an appreciable loss (e.g., a decrease in a function of less than about 1%, less than about 5%, less than about 10%, less than about 25%, less than about 50%, less than about 75%, less than about 90%, less than about 95%, less than about 99%, or less than about 100%) of one or more functions of the unmodified myomaker polypeptide such as, for example, the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some examples, the myomaker polypeptide which was modified retains desired levels (e.g., at least about 20%, at least about 40%, at least about 50%, at least about 75%, at least about 80%, at least about 90%, at least about 95%, or at least about 99%) of one or more functions of the unmodified myomaker polypeptide, such as, for example, the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some examples, the myomaker polypeptide after modification has an increased level of one or more functions as compared to the unmodified myomaker polypeptide. Nucleic acid molecules can be designed to encode for such a modified myomaker polypeptide,.

A "functional myomaker polypeptide" is defined as a myomaker polypeptide (e.g., a modified polypeptide) that has desired levels (e.g., at least about 20%, at least about 40%, at least about 50%, at least about 75%, at least about 80%, at least about 90%, at least about 95%, or at least about 99%, as compared to another myomaker polypeptide, such as a naturally occurring myomaker polypeptide) of one or more functions such as, for example, the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some examples, the function myomaker polypeptide has an increased level of one or more functions as compared to another myomaker polypeptide (e.g., a naturally occurring myomaker polypeptide). Nucleic acid molecules can be designed to encode for functional myomaker polypeptides,.

A "functionally equivalent myomaker polypeptide" is defined as a myomaker polypeptide that has been modified (e.g., by one or more insertions, one or more deletions, or one or more substitutions (e.g., conservative substitutions)) from an original myomaker polypeptide and that modified myomaker polypeptide retains desired levels (e.g., at least about 20%, at least about 40%, at least about 50%, at least about 75%, at least about 80%, at least about 90%, at least about 95%, or at least about 99%) of one or more functions of the original myomaker polypeptide, such as, for example, the ability to activate fusion of two cells, the ability to make a cell fusion capable (e.g., a protein confers fusion capable properties to a cell if upon adding the protein, the cell is capable of fusing to another cell if that other cell comprises myomaker and myomerger), the ability to confer fusogenicity to a cell (e.g., a protein confers fusogenic properties to a cell if upon adding the protein, the cell will fuse with another cell if that other cell comprises myomaker), the level of expression during embryonic development, the level of expression during myogenesis in adult organisms (e.g., older than embryonic), the level of induction of myogenesis in adult organisms (e.g., older than embryonic), the affinity for membranes, or the level of association with membrane compartment. In some embodiments, the functionally equivalent myomaker polypeptide can have an increased level of one or more functions compared to the original myomaker polypeptide. Nucleic acid molecules can be designed to encode for functionally equivalent myomaker polypeptides.

In certain examples, the shorter the length of a myomerger polypeptide, the fewer the modifications (e.g., substitutions) that can be made within the polypeptide while retaining, for example, a desired level of a chosen function. In some instances, longer domains can have a greater number of such changes while retaining, for example, a desired level of a chosen function. In other examples, a full-length polypeptide can have more tolerance for a fixed number of changes while retaining, for example, a desired level of a chosen function, as compared to a shorter length of that polypeptide.

The design of substitutions can take many forms, including but not limited to those described herein. In some examples, the hydropathic index of amino acids may be considered in designing substitutions. In the hydropathic index, each amino acid is assigned a hydropathic index on the basis of their hydrophobicity or charge characteristics, as follows: isoleucine (+4.5); valine (+4.2); Leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); or arginine (-4.5). In some instances, certain amino acids may be substituted for other amino acids having a similar hydropathic index. In making changes based upon the hydropathic index, the substitution of amino acids with hydropathic indices can be made with amino acids that have an index difference of no more than ±2, no more than ±1, or no more than ±0.5.

In some examples, substitutions can also be made based on hydrophilicity values. As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, the substitution of amino acids with hydrophilicity values can be made with amino acids that have a value of no more than ±2, no more than ±1, or no more than ±0.5.

A "conservative substitution" in an amino acid sequence or polypeptide indicates that a given amino acid residue is replaced by a residue having similar physiochemical characteristics (e.g., no more than ±1 when based on hydropathic index or no more than ±1 when base on hydrophilicity values). Examples of conservative substitutions include (a) substitution of one aliphatic residue for another with an aliphatic residue, (b) substitution of one of Ile, Val, Leu, or Ala for one another of He, Val, Leu, or Ala, (c) substitution of one of Gly, Ile, Val, Leu, or Ala for one another of Gly, Ile, Val, Leu, or Ala, (d) substitution of one polar residue for another polar residue, (e) substitution of one of Lys and Arg with another of Lys and Arg, (f) substitution of one of Glu and Asp with another of Glu and Asp, (g) substitution of one of Gln and Asn with another of Gln and Asn, (h) substitution of one hydroxyl or sulfur containing residue with another hydroxyl or sulfur containing residue, (i) substitution of one of Ser, Cys, Thr, or Met with another of Ser, Cys, Thr, or Met, (j) substitution of one aromatic residue for another with an aromatic residue, (k) substitution of one of Phe, Tyr, or Trp with another of Phe, Tyr, or Trp, (l) substitution of one basic residue for another basic residue, (m) substitution of one of His, Lys, or Arg with another of His, Lys, or Arg, (n) substitution of an acidic/amide residue with another acidic/amide residue, (o) substitution of one of Asp, Glu, Asn, or Gln with another of Asp, Glu, Asn, or Gln, (p) substitution of a residue with another residue of a similar size, and (q) substitution of one of Ala, Gly, or Ser with another of Ala, Gly, or Ser. In some embodiments, each amino acid in a hydrophobic region of a polypeptide can be substituted with conservative substitutions (e.g., any combination of conservative substitutions relating to hydrophobic residues).

While discussion has focused on amino acid changes, it will be appreciated that these changes may occur by alteration of the encoding DNA; taking into consideration also that the genetic code is degenerate and that two or more codons may code for the same amino acid. Tables A and B of amino acids and their codons are presented herein for use in such examples, as well as for other uses, such as in the design of probes and primers and the like.

The term "functionally equivalent codon" is used herein to refer to codons that encode the same amino acid, such as the six codons for arginine or serine.

In certain instances, the nucleic acid molecule can be engineered to contain distinct sequences while at the same time retaining the capacity to encode a desired polypeptide. In some examples, this can be accomplished owing to the degeneracy of the genetic code (i.e., the presence of multiple codons) which encode for the same amino acids. In other instances, it can be accomplished by including, adding, or excluding introns in the nucleic acid molecule.

In certain examples, a restriction enzyme recognition sequence can be introduced into a nucleic acid sequence while maintaining the ability of that nucleic acid molecule to encode a desired polypeptide. In other examples, a CRISPR system (e.g., a CRISPR system comprising one or more of guide RNA, crRNA, tracrRNA, sgRNA, DNA repair template, and Cas protein, such as but not limited to CRISPR/Cas9) can be used to introduce a nucleic acid molecule while maintaining the ability of that nucleic acid molecule to encode a desired polypeptide.

It will also be understood that amino acid sequences (e.g., polypeptides) and nucleic acid sequences may include additional residues, such as additional N- or C-terminal amino acids or 5' or 3' sequences, and yet still be essentially as set forth in one of the sequences disclosed herein, so long as the sequence meets the criteria set forth above, including the maintenance of biological activity where polypeptide expression is concerned. The addition of terminal sequences particularly applies to nucleic acid sequences that may, for example, include various non-coding sequences flanking either of the 5' or 3' portions of the coding region or may include various internal sequences, (i.e., introns) which can occur within genes.

Some examples use synthesis of polypeptides *in cyto*, via transcription and translation of appropriate nucleic acid molecules (e.g., nucleic acid sequences as discussed herein). These polypeptides will include the twenty "natural" amino acids, and post-translational modifications thereof. *In vitro* peptide synthesis permits the use of modified or unusual amino acids. In some embodiments, the myomaker polypeptide encompasses modifications (e.g., one or more substitutions or one or more insertions) that include one or more modified or unusual amino acids. A table of exemplary, but not limiting, modified or unusual amino acids is provided in Table C.

Described herein is also a method of producing a myomaker polypeptide (e.g., a mutant myomaker polypeptide or a wt- myomaker polypeptide), which does not form part of the invention. Any suitable method can used to make the myomaker polypeptides including but not limited to expression through any suitable molecular biological technique (e.g., using a prokaryotic or eukaryotic expression system), isolation from a source in nature, or chemical synthesis. Eukaryotic expression systems include plant-based systems; insect cell systems via recombinant baculoviruses; whole insect systems via recombinant baculoviruses; genetically engineered yeast systems, including but not limited to *Saccharomyces sp.* and *Picchia spp*.; and mammalian cell systems, including but not limited to C2C12 cells, 10T 1/2 fibroblasts, NIH/3T3 fibroblasts, mesenchymal stem cells (MSCs), hematopoietic stem cells, Chinese hamster ovary cells or other cell lines commonly used for industrial scale expression of recombinant proteins. In some examples, useful plant-based expression systems can include transgenic plant systems. In some examples, useful plant-based expression systems can include transplastomic plant systems.

In some examples, a method of producing the myomaker polypeptide includes providing a host cell comprising a myomaker nucleic acid molecule, as disclosed herein, operatively linked to a promoter operable under conditions whereby the encoded myomaker polypeptide is expressed; and recovering the myomaker polypeptide from the host cell.

### Cells Including Modified Cells

The methods and the pharmaceutical composition according to the invention include modified cells. Exemplarily, a modified cell is a cell that comprises one or more modifications of a cell, where at least one of the one or more modifications was implemented by a human (e.g., by human activity, either directly or indirectly). Exemplarily, the cell to be modified can be an unmodified cell or can be a cell that has been previously modified (e.g. modified as disclosed herein). A cell can be modified in any desired manner, including but not limited to (a) adding a nucleic acid molecule such as but not limited to one or more nucleic acid molecules disclosed herein, (b) diminishing the effect of one or more nucleic acid molecules (e.g., a naturally occurring nucleic acid molecule or an added nucleic acid molecule) such as a gene, (c) adding one or more polypeptides, including but not limited to polypeptides disclosed herein, (d) diminishing the effect of one or more polypeptides (e.g., a naturally occurring polypeptide or an added polypeptide), or (e) a combination thereof. In some instances, a modified cell can result from a further modification of another modified cell.

Adding a nucleic acid molecule to modify a cell can be accomplished using any suitable method including but not limited to one or more of transformation (as used herein transfection methods are encompassed by the term transformation), viral transformation (e.g., using a viral vector, a retroviral vector, a lentiviral vector, an adenoviral vector, an adeno-associated viral vector, a herpesviral vector, a chimeric viral vector, a plasmid, a cosmid, an artificial chromosome, a bacteriophage, a virus, an animal virus, a plant virus, an expression vector, a conjugative vector, or a nonconjugative vector), injection, microinjection, electroporation, sonication, calcium ion treatment, calcium phosphate precipitation, PEG-DMSO treatment, DE-Dextran treatment, liposome mediated transformation, or a receptor mediated transformation. Adding a polypeptide to modify a cell can be accomplished using any suitable method including but not limited to one or more of injection, microinjection, electroporation, sonication, calcium ion treatment, calcium phosphate precipitation, PEG-DMSO treatment, DE-Dextran treatment, or liposome mediated. The added nucleic acid molecule can be part of a vector (e.g., a viral vector, a retroviral vector, a lentiviral vector, an adenoviral vector, an adeno-associated viral vector, a herpesviral vector, a chimeric viral vector, a plasmid, a cosmid, an artificial chromosome, a bacteriophage, an animal virus, a plant virus, an expression vector, a conjugative vector, or a nonconjugative vector), a plasmid, a cosmid, an artificial chromosome, a bacteriophage, a virus, an animal virus, or a plant virus. In some examples, the added nucleic acid molecule is exogenous; "exogenous" means (a) that the added nucleic acid molecule originates from outside of the cell (e.g., is foreign to the cell) or (b) that the added nucleic acid molecule can be found inside the cell, but the added nucleic acid molecule is placed in the cell where it is not normally found (e.g., a different part of the chromosome or on an added plasmid). In some examples, the added polypeptide is exogenous; "exogenous" in this context means that the added polypeptide originates from outside of the cell (e.g., is foreign to the cell).

In some examples, the modification to the cell can be the diminution of the effect of a nucleic acid molecule in the cell; the nucleic acid molecule can be added to the cell or not, or can be exogenous. Diminishing the effect of a nucleic acid molecule includes but is not limited to decreasing (e.g., stopping) the expression of a polypeptide (e.g., myomaker, myomerger, or both) or expressing a less active form of a polypeptide (e.g., by changing the polypeptide's amino acid sequence or expressing only a fragment of a polypeptide). Diminishing the effect of a nucleic acid molecule can be accomplished using any suitable method including but not limited to removal of the nucleic acid molecule (e.g., from a chromosome or from a plasmid), removal of one or more parts of the nucleic acid molecule (e.g., from a chromosome or from a plasmid), altering the nucleic acid sequence of the nucleic acid molecule, diminishing or preventing transcription of the nucleic acid molecule (e.g., via a repressor, inhibitor, blocker (e.g., via a molecule that blocks part of the transcription), or stabilization of a non-transcribing form), or diminishing or preventing translation (e.g., via an inhibitor or an RNA inhibitor).

The cell to be modified can be any suitable cell including but not limited to an insect cell (e.g., an Sf9 cell), a vertebrate cell, or a mammalian cell (e.g., a human cell, a rat cell a mouse cell, a muscle cell, a non-muscle cell, a myoblast, a fibroblast, a C2C12 cell, a 10T ½ fibroblast, a NIH/3T3 cell, a CHO cell, a mesenchymal stem cell (MSC), a hematopoietic stem cell, a blood cell, a bone marrow cell, a stem cell, or an adipose stem cell). In certain examples, an unmodified cell can be any suitable cell including but not limited insect cell, a vertebrate cell, or a mammalian cell (e.g., a human cell, a rat cell a mouse cell, a muscle cell, a non-muscle cell, a myoblast, a fibroblast, a NIH/3T3 cell, a CHO cell, a mesenchymal stem cell (MSC), a hematopoietic stem cell, a blood cell, a bone marrow cell, a stem cell, or an adipose stem cell).

In some examples, a modified cell can be but is not limited to a modified animal cell, a modified vertebrate cell, a modified mammalian cell, a modified human cell, a modified rat cell, a modified mouse cell, a modified muscle cell, a modified non-muscle cell, a modified myoblast, a modified fibroblast, a C2C12 cell, a modified C2C12 cell, a 10T ½ fibroblast, a modified 10T ½ fibroblast, a modified NIH/3T3 cell, a modified CHO cell, a modified mesenchymal stem cell (MSC), a modified hematopoietic stem cell, a modified blood cell, a modified bone marrow cell, a modified stem cell, or a modified adipose stem cell. In other embodiments, the modified cell is a modified non-muscle cell (e.g., a modified fibroblast, a 10T ½ fibroblast, a modified 10T ½ fibroblast, a modified NIH/3T3 cell, a modified CHO cell, a modified mesenchymal stem cell (MSC), a modified hematopoietic stem cell, a modified blood cell, a modified bone marrow cell, a modified stem cell, or a modified adipose stem cell).

The first cell is a modified cell and is a non-muscle cell with a myomerger nucleic acid molecule added (e.g., where the myomerger nucleic acid molecule is exogenous), .

### Compositions including Pharmaceutical Compositions

One or more polypeptides (e.g., a wt-myomerger polypeptide or mutant myomerger polypeptide) or one or more myomerger nucleic acid molecules (e.g., in the form of a bare nucleic acid molecule, a vector, a virus, a plasmid or any suitable form) can be part of a composition and can be in an amount (by weight of the total composition) of at least about 0.0001%, at least about 0.001%, at least about 0.10%, at least about 0.15%, at least about 0.20%, at least about 0.25%, at least about 0.50%, at least about 0.75%, at least about 1%, at least about 10%, at least about 25%, at least about 50%, at least about 75%, at least about 90%, at least about 95%, at least about 99%, at least about 99.99%, no more than about 75%, no more than about 90%, no more than about 95%, no more than about 99%, or no more than about 99.99%, from about 0.0001% to about 99%, from about 0.0001% to about 50%, from about 0.01% to about 95%, from about 1% to about 95%, from about 10% to about 90%, or from about 25% to about 75%. A myomaker polypeptide, is both can be part of the composition (e.g., together with a myomerger polypeptide in the composition or a myomerger nucleic acid molecule in the composition) at any amount indicated herein (e.g., indicated above). In certain embodiments, cells, such as modified cells (e.g., as disclosed herein) can be part of the composition at any amount indicated herein (e.g., indicated above).

One or more polypeptides (e.g., a wt-myomerger polypeptide or mutant myomerger polypeptide) or one or more myomerger nucleic acid molecules (e.g., in the form of a bare nucleic acid molecule, a vector, a virus, a plasmid or any suitable form) can be purified or isolated in an amount (by weight of the total composition) of at least about 0.0001%, at least about 0.001%, at least about 0.10%, at least about 0.15%, at least about 0.20%, at least about 0.25%, at least about 0.50%, at least about 0.75%, at least about 1%, at least about 10%, at least about 25%, at least about 50%, at least about 75%, at least about 90%, at least about 95%, at least about 99%, at least about 99.99%, no more than about 75%, no more than about 90%, no more than about 95%, no more than about 99%, no more than about 99.99%, from about 0.0001% to about 99%, from about 0.0001% to about 50%, from about 0.01% to about 95%, from about 1% to about 95%, from about 10% to about 90%, or from about 25% to about 75%. In some examples, isolated or purified means that impurities (e.g., cell components or unwanted solution components if chemically synthesized) were removed by one or more of any suitable technique (e.g., column chromatography, HPLC, centrifugation, fractionation, gel, precipitation, or salting out).

Some examples of the pharmaceutical composition of the invention comprise one or more polypeptides (e.g., a wt-myomerger polypeptide or mutant myomerger polypeptide) and one or more myomerger nucleic acid molecules (e.g., in the form of a bare nucleic acid molecule, a vector, a virus, a plasmid or any suitable form). The composition is a pharmaceutical composition, such as compositions that are suitable for administration to animals (e.g., mammals, primates, monkeys, humans, canine, porcine, mice, rabbits, or rats). In some examples, there may be inherent side effects (e.g., it may harm the patient or may be toxic or harmful to some degree in some patients).

In some examples, one or more polypeptides (e.g., a wt-myomerger polypeptide or mutant myomerger polypeptide) and one or more myomerger nucleic acid molecules (e.g., in the form of a bare nucleic acid molecule, a vector, a virus, a plasmid or any suitable form) can be part of the pharmaceutical composition and can be in an amount (by weight of the total composition) of at least about 0.0001%, at least about 0.001%, at least about 0.10%, at least about 0.15%, at least about 0.20%, at least about 0.25%, at least about 0.50%, at least about 0.75%, at least about 1%, at least about 10%, at least about 25%, at least about 50%, at least about 75%, at least about 90%, at least about 95%, at least about 99%, at least about 99.99%, no more than about 75%, no more than about 90%, no more than about 95%, no more than about 99%, no more than about 99.99%, from about 0.001% to about 99%, from about 0.001% to about 50%, from about 0.1% to about 99%, from about 1% to about 95%, from about 10% to about 90%, or from about 25% to about 75%. A myomaker polypeptide is be part of the pharmaceutical composition (e.g., together with a myomerger polypeptide in the pharmaceutical composition or a myomerger nucleic acid molecule in the pharmaceutical composition) at any amount indicated herein (e.g., indicated above). The modified first cell (e.g., as disclosed herein) is part of the pharmaceutical composition at any amount indicated herein (e.g., indicated above).

In some examples, the pharmaceutical composition can be presented in a dosage form which is suitable for the topical, subcutaneous, intrathecal, intraperitoneal, oral, parenteral, rectal, cutaneous, nasal, vaginal, or ocular administration route. In other examples, the pharmaceutical composition can be presented in a dosage form which is suitable for parenteral administration, a mucosal administration, intravenous administration, subcutaneous administration, topical administration, intradermal administration, oral administration, sublingual administration, intranasal administration, or intramuscular administration. The pharmaceutical composition can be in the form of, for example, tablets, capsules, pills, powders granulates, suspensions, emulsions, solutions, gels (including hydrogels), pastes, ointments, creams, plasters, drenches, delivery devices, suppositories, enemas, injectables, implants, sprays, aerosols or other suitable forms.

In some examples, the pharmaceutical composition can include one or more formulary ingredients. A "formulary ingredient" can be any suitable ingredient (e.g., suitable for the drug(s), for the dosage of the drug(s), for the timing of release of the drugs(s), for the disease, for the disease state, for the organ, or for the delivery route) including, but not limited to, water (e.g., boiled water, distilled water, filtered water, pyrogen-free water, or water with chloroform), sugar (e.g., sucrose, glucose, mannitol, sorbitol, xylitol, or syrups made therefrom), ethanol, glycerol, glycols (e.g., propylene glycol), acetone, ethers, DMSO, surfactants (e.g., anionic surfactants, cationic surfactants, zwitterionic surfactants, or nonionic surfactants (e.g., polysorbates)), oils (e.g., animal oils, plant oils (e.g., coconut oil or arachis oil), or mineral oils), oil derivatives (e.g., ethyl oleate , glyceryl monostearate, or hydrogenated glycerides), excipients, preservatives (e.g., cysteine, methionine, antioxidants (e.g., vitamins (e.g., A, E, or C), selenium, retinyl palmitate, sodium citrate, citric acid, chloroform, or parabens, (e.g., methyl paraben or propyl paraben)), or combinations thereof. In some embodiments, the concentration of any individual formulary ingredient in a composition (e.g., pharmaceutical composition) can be in an amount (by weight of the total composition) of at least about 0.0001%, at least about 0.001%, at least about 0.10%, at least about 0.15%, at least about 0.20%, at least about 0.25%, at least about 0.50%, at least about 0.75%, at least about 1%, at least about 10%, at least about 25%, at least about 50%, at least about 75%, at least about 90%, at least about 95%, at least about 99%, at least about 99.99%, no more than about 75%, no more than about 90%, no more than about 95%, no more than about 99%, no more than about 99.99%, from about 0.001% to about 99%, from about 0.001% to about 50%, from about 0.1% to about 99%, from about 1% to about 95%, from about 10% to about 90%, or from about 25% to about 75%. In some examples, the concentration of at least one formulary ingredient is not that same as that found in the natural system in which polypeptide (e.g., wt-myomerger polypeptide) is found. In some embodiments, the concentration of at least one formulary ingredient is not that same as that found in one or more natural systems (e.g., any natural system found in nature) in which the nucleic acid molecule which encodes apolypeptide (e.g., wt-myomerger polypeptide) is found.

In certain examples, pharmaceutical compositions can be formulated to release the active ingredient (e.g., one or more compounds of Formula (I)) substantially immediately upon the administration or any substantially predetermined time or time after administration. Such formulations can include, for example, controlled release formulations such as various controlled release compositions and coatings.

Other formulations (e.g., formulations of a pharmaceutical composition) can, in certain examples, include those incorporating the drug (or control release formulation) into food, food stuffs, feed, or drink.

### Methods of Using Cells Including Modified Cells

The methods according to the invention include cells, such as modified cells.

The inventive in vitro method for fusing two or more cells comprise contacting a first cell with a second cell to form a third cell, where the first cell is a modified cell (e.g., as disclosed herein). In certain examples, the term "fuse" (and related terms such as "fusing", "fusion" etc.) means to combine two cells to form a a third cell. The fusion results in a multinuclear cell (e.g., syncytium). The first cell comprises a first myomerger polypeptide and a first myomaker polypeptide. The second cell comprises a second myomaker polypeptide and a second myomerger polypeptide. The third cell is a multinucleated cell.

The first cell is a non-muscle cell. In some examples, the the second cell both can be any suitable cell including but not limited to an insect cell (e.g., an Sf9 cell), a vertebrate cell, or a mammalian cell (e.g., a human cell, a rat cell a mouse cell, a muscle cell, a non-muscle cell, a myoblast, a fibroblast, a C2C12 cell, a 10T ½ fibroblast, a NIH/3T3 cell, a CHO cell, a dendritic cell, a cancer cell, a mesenchymal stem cell (MSC), a hematopoietic stem cell, a blood cell, a bone marrow cell, a stem cell, or an adipose stem cell). In some examples, the first cell and the second cell are the same type of cell (e.g., homotypic cell fusion which can in certain instances form syncytium). In other embodiments, the first cell and the second cell are different types of cell (e.g., heterotypic cell fusion). Cell fusion can, in some instances, result in nuclear fusion. In other instances, cell fusion does not result in nuclear fusion.

In some examples, the second cell can be a modified cell that can be but is not limited to a modified animal cell, a modified vertebrate cell, a modified mammalian cell, a modified human cell, a modified rat cell, a modified mouse cell, a modified muscle cell, a modified non-muscle cell, a modified myoblast, a modified fibroblast, a C2C12 cell, a modified C2C12 cell, a 10T ½ fibroblast, a modified 10T ½ fibroblast, a modified NIH/3T3 cell, a modified CHO cell, a modified dendritic cell, a modified cancer cell, a modified mesenchymal stem cell (MSC), a modified hematopoietic stem cell, a modified blood cell, a modified bone marrow cell, a modified stem cell, or a modified adipose stem cell. In other examples, the first cell and the second cell can be a modified cell that is a modified non-muscle cell (e.g., a modified fibroblast, a 10T ½ fibroblast, a modified 10T ½ fibroblast, a modified NIH/3T3 cell, a modified CHO cell, a modified dendritic cell, a modified cancer cell, a modified mesenchymal stem cell (MSC), a modified hematopoietic stem cell, a modified blood cell, a modified bone marrow cell, a modified stem cell, or a modified adipose stem cell).

In certain examples of the method, the first cell is a non-muscle cell, and optionally the second cell is also a non-muscle cell. In other examples of the method, the first cell is a non-muscle cell and the second cell is a muscle cell.

In some instances of the method, the second cell is an isolated muscle cell (e.g., myoblast). In still other examples of the method, the second cell is a muscle cell or is a cell that is part of a muscle, muscle tissue, or non-muscle tissue. In some embodiments, the muscle, muscle tissue, or non-muscle tissue is diseased. In other embodiments, the muscle, muscle tissue, or non-muscle tissue (e.g., diseased or not diseased) is part of the circulation system (e.g., heart), respiratory system (e.g., diaphragm), head, neck, gastrointestinal system (e.g., tongue, esophageal muscles, or intestinal muscles), skeletal muscles, or genitourinary tract.

The contacting of the cells in the method can occur by any suitable manner, such as but not limited to those disclosed herein. The contacting occurs *in vitro.*

The invention also includes a method for delivering a gene of interest comprising contacting a first cell with a second cell in vitro, which fuse to form a third cell. In certain examples, the term "fuse" (and related terms such as "fusing", "fusion" etc.) means to combine two cells to form a a third cell. The fusion results in a multinuclear cell (e.g., syncytium). The first cell is a modified cell (e.g., as disclosed here) and comprises (a) a gene of interest and (b) a first myomerger polypeptide and a first myomaker polypeptide. The second cell comprises a second myomaker polypeptide, and optionally a second myomerger polypeptide. The third cell is a multinucleated cell. In some examples, the first cell and the second cell are the same type of cell (e.g., homotypic cell fusion which can in certain instances form syncytium). In yet other examples, the first cell and the second cell are different types of cell (e.g., heterotypic cell fusion). Cell fusion can, in some instances, result in nuclear fusion. In other instances, cell fusion does not result in nuclear fusion.

In certain embodiments, the gene of interest encodes for a therapeutic polypeptide, a therapeutic protein, or a therapeutic oligopeptide, where the gene of interest can be part of a treatment of a disease. In other examples, the gene of interest can be genomic DNA or can be cDNA.

In certain embodiments, the second cell can underexpress the gene of interest, does not express the gene of interest, expresses a defective version of the gene of interest, or a combination thereof. In other embodiments, the second cell does not express the gene of interest.

In certain examples of the method, the first cell is a non-muscle cell and the second cell is a non-muscle cell. In other examples of the method, wherein the first cell is a non-muscle cell and the second cell is a muscle cell.

In some instances of the method, the second cell is an isolated muscle cell (e.g., myoblast). In still other embodiments of the method, the second cell is a muscle cell or is a cell that is part of a muscle, muscle tissue, or non-muscle tissue. In some embodiments, the muscle, muscle tissue, or non-muscle tissue is diseased. In other embodiments, the muscle, muscle tissue, or non-muscle tissue (e.g., diseased or not diseased) is part of the circulation system (e.g., heart), respiratory system (e.g., diaphragm), head, neck, gastrointestinal system (e.g., tongue, esophageal muscles, or intestinal muscles), skeletal muscles, or genitourinary tract.

The contacting of the cells in the method can occur by any suitable manner such as but not limited to those disclosed herein. The contacting occurs *in vitro.* In other exemplary embodiments, contacting can occur *ex vivo.*

Animals include but are not limited to mammals, primates, monkeys (e.g., macaque, rhesus macaque, or pig tail macaque), humans, canine, feline, bovine, porcine, avian (e.g., chicken), mice, rabbits, and rats. As used herein, the term "subject" refers to both human and animal subjects.

In certain examples, the inventive method to delivery of a gene of interest can be part of a treatment of a disease. In some examples, the disease can be a disease, such as but not limited to, diseases where cells underexpress the gene of interest, do not express the gene of interest, express a defective version of the gene of interest, or a combination thereof. In some examples, the disease can be a non-muscle-related disease, such as but not limited to, non-muscle diseases where cells underexpress the gene of interest, do not express the gene of interest, express a defective version of the gene of interest, or a combination thereof. In some examples, the disease can be a muscle-related disease, such as but not limited to, muscle diseases where cells underexpress the gene of interest, do not express the gene of interest, express a defective version of the gene of interest, or a combination thereof. In certain examples, the treated disease can be a myopathy, muscular dystrophy, amyotrophic lateral sclerosis (ALS or also called Lou Gehrig's disease), glycogen storage disease type II (also called Pompe disease), rhabdomyosarcoma (RMS), sarcopenia, or a combination thereof. In some examples, the disease can be cancer. As used herein, the term "treating" (and its variations, such as "treatment") is to be considered in its broadest context. In particular, the term "treating" does not necessarily imply that an animal is treated until total recovery. Accordingly, "treating" includes amelioration of the symptoms, relief from the symptoms or effects associated with a condition, decrease in severity of a condition, or preventing, preventively ameliorating symptoms, or otherwise reducing the risk of developing a particular condition. As used herein, reference to "treating" an animal includes but is not limited to prophylactic treatment and therapeutic treatment. Any of the methods or compositions (e.g., pharmaceutical compositions) described herein can be used to treat an animal.

In yet other examples, the delivery of the gene of interest can occur by any suitable administration route. Administration routes can be, but are not limited to the oral route, the parenteral route, the cutaneous route, the nasal route, the rectal route, the vaginal route, and the ocular route. In other examples, administration routes can be parenteral administration, a mucosal administration, intravenous administration, depot injection, subcutaneous administration, topical administration, intradermal administration, oral administration, sublingual administration, intranasal administration, or intramuscular administration (e.g., intramuscular injection). The delivery, which is not encompassed by the claimed method of the incention, can comprise an injection or an intramuscular injection. In certain , the delivery comprises an injection comprising the first cell, the second cell, or both (e.g., in a composition or in a pharmaceutical composition). In other examples, the delivery comprises an intramuscular injection comprising the first cell, the second cell, or both (e.g., in a composition or in a pharmaceutical composition).

In still other examples, the delivery can further comprise one or more of the contacting steps.

The presently-disclosed subject matter is further illustrated by the following specific but non-limiting examples. The following examples may include compilations of data that are representative of data gathered at various times during the course of development and experimentation related to the present disclosure.

### EXAMPLES

### Materials and Methods

### Cell Culture

C2C12 cells, 10T 1/2 fibroblasts, and NIH/3T3 fibroblasts were purchased from American Type Culture Collection and propagated in DMEM (Gibco) containing 10% heat-inactivated bovine growth serum (BGS) and supplemented with antibiotics. C2C12 cells were differentiated by switching to media containing 2% heat-inactivated horse serum (HS) and antibiotics. MSCs were a gift from Jose Cancelas. GONZALEZ-NIETO et al., "Connexin-43 in the osteogenic bm niche regulates its cellular composition and the bidirectional traffic of hematopoietic stem cells and progenitors" Blood (2012) Vol. 119, pp. 5144-5154.

### Bioinformatic Analysis

Microarray data from the GEO DataSet GSE34907³⁴ was interrogated using GEO2R analysis to identify 1826 genes displaying an increase greater than 1 log fold-change in MyoD-expressing fibroblasts. In parallel, a transcriptional profile of 10T 1/2 fibroblasts transduced with empty virus was generated using RNA-seq analysis (paired-end library layout using Illumina sequencing platform) and a list of all genes with RPKM values below 1.5 compiled using Strand NGS software (Ver. 2.6; Build: Mouse mm10 (UCSC) using Ensembl transcript annotations). These two gene lists were then compared to generate a final tally comprised of 531 genes that were both upregulated in MyoD-expressing fibroblasts and had low or no detectable expression in 10T 1/2 fibroblasts. Finally, the top 100 genes were interrogated for genes that contain transmembrane domains and not previously studied for their role during myoblast fusion.

### Animals

We used a dual sgRNA targeting strategy to create *Gm7325*^{*-*/*-*} mice. We selected the sgRNAs according to the on- and off-target scores from the web tool CRISPOR. (HAEUSSLER et al. "Evaluation of off-target and on-target scoring algorithms and integration into the guide rna selection tool crispor" Genome Biol (2016) Vol. 17, article 148 (12 pages).) The selected gRNAs were 5'-GCAGCGATCGAAGCACCATC-3' (SEQ ID NO: 1) and 5'-GAGGCCTCTCCAGAATCCGG-3' (SEQ ID NO: 2) that target exon 3 of *Gm7325.* The sgRNAs were *in vitro* synthesized using the MEGAshortscript T7 kit (ThermoFisher) and purified by the MEGAclear Kit (ThermoFisher). sgRNAs (50 ng/ul of each) were mixed with 100 ng/ul Cas9 protein (ThermoFisher) and incubated at 37°C for 15 min to form a ribonucleoprotein complex. We then injected the mix into the cytoplasm of one-cell-stage embryos of the C57BL/6 genetic background using a piezo-driven microinjection technique. (YANG et al., "Generating genetically modified mice using crispr/cas-mediated genome engineering" Nat Protoc (2014) Vol. 9, pp. 1956-1968) Injected embryos were immediately transferred into the oviducal ampulla of pseudopregnant CD-1 females. Live born pups were genotyped by PCR with primers spanning the mutated region (Table E1). The edited allele was further confirmed by Sanger sequencing. One heterozygous founder was obtained and mated with WT C57B16 mice to eventually generate KO mice. The gender of analyzed embryos was not determined. *Mdx^{4cv}* mice were purchased from Jackson Laboratory (#002378) and male mice were used. Muscle overload of the plantaris muscle was achieved through bilateral synergistic ablation of soleus and gastrocnemius muscles. Specifically, the soleus and gastrocnemius muscles were exposed by making an incision on the posterior-lateral aspect of the lower limb. The distal and proximal tendons of the soleus, lateral and medial gastrocnemius were subsequently cut and carefully excised. All animal procedures were approved by Cincinnati Children's Hospital Medical Center's Institutional Animal Care and Use Committee.

**Table E1**

| Description | Forward Primer | Reverse Primer |
|---|---|---|
| 1. Genotyping for *Gm7325* mutation | GAAGGGAGGACTCCACA CCC (SEQ ID NO: 3) | CGCCTGGACTAACCGGC TCC (SEQ ID NO: 4) |
| 2. Cloning Gm7325 locus containing short and long isoforms | AGTGATGCTGAATCCAC CGCA (SEQ ID NO: 5) | CCAATAACAACACACTG TCCT (SEQ ID NO: 6) |
| 3. Cloning of *Gm7325* - long | ATGCCAGAAGAAAGCTG CACTG (SEQ ID NO: 7) | TCACTTCTGGGGGCCCA ATCTC (SEQ ID NO: 8) |
| 4. Cloning of *Gm7325* - short | ATGCCCGTTCCATTGCTC CCGA (SEQ ID NO: 9) | TCACTTCTGGGGGCCCA ATCTC (SEQ ID NO: 10) |
| 5. myomerger-short SYBR | CAGGAGGGCAAGAAGTT CAG (SEQ ID NO: 11) | ATGTCTTGGGAGCTCAG TCG (SEQ ID NO: 12) |
| 6. myomerger-long SYBR | ACCAGCTTTCATGCCAGA AG (SEQ ID NO: 13) | ATGTCTTGGGAGCTCAG TCG (SEQ ID NO: 14) |
| 7. myomaker SYBR | ATCGCTACCAAGAGGCG TT (SEQ ID NO: 15) | CACAGCACAGACAAACC AGG (SEQ ID NO: 16) |
| 8. Tm6sf1 SYBR | TTAGTGGTCCCTGGATGC TC (SEQ ID NO: 17) | GACGCACCAATGTGAGA AAA (SEQ ID NO: 18) |
| 9. Tspan33 SYBR | GGGGACGAGTTCTCCTTC | TGCTTCTGCGTGCTTCAT |
| | G (SEQ ID NO: 19) | TAG (SEQ ID NO: 20) |
| 10. Tmem182 SYBR | GGCTCTCTTCGGAGCTTT GG (SEQ ID NO: 21) | GGTGGCTGATTGGTGTA CCAG (SEQ ID NO: 22) |
| 11. Myogenin SYBR | CTACAGGCCTTGCTCAGC TC (SEQ ID NO: 23) | GTGGGAGTTGCATTCAC TGG (SEQ ID NO: 24) |
| 12. Ckm SYBR | ACCTCCACAGCACAGAC AGA (SEQ ID NO: 25) | CAGCTTGAACTTGTTGT GGG (SEQ ID NO: 26) |
| 13. Myh4 SYBR | GCAGGACTTGGTGGACA AAC (SEQ ID NO: 27) | ACTTGGCCAGGTTGACA TTG (SEQ ID NO: 28) |
| 14. GAPDH SYBR | TGCGACTTCAACAGCAA CTC (SEQ ID NO: 29) | GCCTCTCTTGCTCAGTGT CC (SEQ ID NO: 30) |

### CRISPR-Mediated Genome Editing in C2C12 Cells

Freshly plated low passage C2C12 cells were transfected with 4µg of a modified pX458 plasmid (Addgene #48138, gift from Yueh-Chiang Hu), which contained a high fidelity Cas9, an optimized sgRNA scaffold, and an IRES-GFP cassette. The same gRNAs used to generate KO animals were used for C2C12 cells. 16 µL of Lipofectamine 2000 was used for this transfection. 5 × 10⁵ C2C12 cells were transfected in a 60 mm culture dish. Forty-eight hours after transfection GFP⁺ cells were sorted into 96 well plates using FACS. These cells were maintained in DMEM containing 20% FBS with antibiotics at subconfluent densities. The cell lines were genotyped by amplifying a 420 bp region surrounding the site of Cas9 activity using the primers used to genotype *Gm7325*^{*-*/*-*} animals.

### Cloning and viral infection

We initially cloned a region of the *Gm7325* locus, containing all genomic information for expression of myomerger-short and myomerger-long, from C57B16 mouse genomic DNA. We cloned myomerger-short and long coding sequences from cDNA of differentiating C2C12 cells. Cloning primers are listed in Table E1. Myomerger cDNA and genomic DNA was cloned into the retroviral vector pBabe-X using EcoRI. Myomaker and GFP retroviral plasmids have been described previously. (MILLAY et al. "Myomaker is a membrane activator of myoblast fusion and muscle formation" Nature (2013) Vol. 499, pp. 301-305.) NLS-TdTomato was subcloned from pQC-NLS-TdTomato (Addgene #37347) into the retroviral vector pMX (Cell Biolabs). Plasmids containing cDNA for Tmem182, Tspan33, and Tm6sf1 from the Mammalian Gene Collection were purchased from Open Biosystems and subcloned into pBabe-X. Ten micrograms of retroviral plasmid DNA were transfected with FuGENE 6 (Roche) into Platinum E cells (Cell Biolabs), which were plated 24 hours before transfection on a 10 cm culture dish at a density of 3-4×10⁶ cells per dish. Forty-eight hours after transfection, viral media were collected, filtered through a 0.45 µm cellulose syringe filter and mixed with polybrene (Sigma) at a final concentration of 6 µg/ml. Target cells were plated on 10 cm culture dishes at a density of 4×10⁵ cells per dish 16-18 hours before infection. Eighteen hours after infection, virus was removed, cells were washed with PBS and split into new 10 cm dishes.

### Cell fusion assays

Cells were split 18 hours after retroviral infection and split again 24-48 hours later. At the second split, cells were seeded for the fusion assay on 35-mm dishes (3-4×10⁵ cells per dish) or on 8-well Ibidi slides (2×10⁴ cells/well). Fusion was assessed 24-48 hours after seeding. For heterologous fusion, cultures of fibroblasts and myoblasts mixed at a ratio of 1:1 (1.5×10⁵ cells for each) were induced to differentiate 24 hours after seeding and fusion was assessed on day 4 of differentiation.

### RNA extraction and quantitative RT-PCR (qRT-PCR)

Total RNA was extracted from either mouse tissue or cultured cells with TRIZOL (Life Technologies) according to manufacturer's protocol. cDNA was synthesized using High-Capacity cDNA Reverse Transcription Kit (Applied Biosystems) with random primers. Gene expression was assessed using standard quantitative PCR approach with Power Sybr^{®} Green PCR mastermix (Applied Biosystems). Analysis was performed on StepOnePlus Real-Time PCR system (Applied Biosystems) with gene-specific primers (Table E1).

### Western blot analysis

Cultured cells were washed two times with ice cold PBS, scraped into a conical tube, pelleted, resuspended in lysis buffer (50 mM Tris-HCl, pH 6.8, 1mM EDTA, 2% SDS) and sonicated for a total of 15 seconds (three 5 second pulses). Skeletal muscle tissues from mice were homogenized with a bead homogenizer (TissueLyser II; Qiagen) in lysis buffer (10 mM Tris (pH 7.4), 1 mM EDTA, 1mM dithiothreitol, 0.5% Triton X-100, 2.1 mg/ml NaF) containing protease and phosphatase inhibitor cocktails (5 µl/ml; Sigma-Aldrich). Both cells and tissue lysates were centrifuged to pellet insoluble material and protein concentration was determined using Bradford protein assay. Equal amounts of protein (5 µg for cells and 20 µg for tissues) were prepared with loading buffer (1× Laemmli (Bio-Rad) with reducing agent (5% β-mercaptoethanol for cells and 100 mM DTT for tissues). Samples were heated at 37°C for 30 minutes and separated on a 20% SDS-PAGE. The gels were subsequently transferred to a PVDF membrane (Millipore), blocked in 5% milk in Tris-buffered saline/0.1% Tween-20 (TBS-T) and incubated with anti-sheep ESGP antibody (1 mg/µl; R&D Systems) overnight at 4°C. Membranes were then washed with TBS-T and incubated with Alexa-Fluor 647 donkey anti-sheep secondary antibody (1:5,000; Invitrogen). Bands were visualized using the Odyssey^{®} infrared detection system (LI-COR Biosciences). GAPDH (1:5,000; Millipore) was used as a loading control.

### Subcellular fractionation

C2C12 cells were harvested on day 2 of differentiation in ice cold hypotonic buffer (10 mM Tris-HCl pH 8, 2 mM EDTA) and lysed using a dounce homogenizer. Lysates were then centrifuged at 800 × g for 5 minutes at 4°C to separate nuclei and cell debris. That supernatant was then centrifuged at 5000 × g for 10 minutes to pellet mitochondria and ER. ER and heavy vesicles were further pelleted through centrifugation at 17,000 × g for 10 minutes. Finally, plasma membrane, light vesicles, and organelles were pelleted at 100,000 × g for 20 minutes and the supernatant from this spin was collected as the cytosolic fraction. All pellets were resuspended in lysis buffer (50 mM Tris-HCl, pH 6.8, 1mM EDTA, 2% SDS) at volumes equal to the supernatant. Eight µl of each fraction was separated by SDS-PAGE and analyzed for presence of myomerger, caveolin-3, and tubulin. Caveolin-3 antibody (BD Transduction Laboratories #610421) was used at 1:6700 and tubulin (Santa Cruz #SC-8035) at 1:50.

### Immunocytochemistry

Cultured cells were rinsed with PBS and fixed in 4% paraformaldehyde (PFA)/PBS for 15 minutes at room temperature. Cells were subsequently permeabilized and blocked in 0.01% Triton X-100/5% donkey serum/PBS for one hour at room temperature. Primary antibody diluted in permeabilization/blocking buffer was incubated overnight. Cells were then washed with PBS and incubated with secondary Alexa-Fluor antibodies (1:250) for 1 hour. A myomaker custom antibody was generated through YenZym Antibodies LLC. Rabbits were immunized with amino acids #137-152 of mouse myomaker (MKEKKGLYPDKSIYTQ (SEQ ID NO: 31)) after conjugation to KLH. We used antigen-specific affinity purified products at a concentration of 4.3 µg/mL for immunostaining. Esgp (myomerger) antibody was used at a concentration of 1 µg/mL. Anti-mouse myosin (my32, MA5-11748, ThermoFisher Scientific) antibody was used at 1:100. Hoechst 33342 solution (ThermoFisher Scientific) was used to stain nuclei. Cells were imaged using Nikon A1R+ confocal on a FN1 microscope (35 mm dishes) or Nikon A1R confocal on Eclipse T1 inverted microscope (Ibidi slides).

### Histology and immunohistochemistry

For cryosections, embryos were dissected, fixed in 4% PFA/PBS overnight at 4°C, washed in PBS, incubated in 30% sucrose/PBS overnight and then in 1:1 mix of optimal cutting temperature (O.C.T.) formulation and 30% sucrose prior to embedding in O.C.T. Sections were cut at 10 µm and then permeabilized with 0.2% Triton X-100/PBS, blocked with 1% BSA/1% heat inactivated goat serum/0.025% Tween20/PBS and incubated with primary antibody overnight. Anti-mouse myosin (my32, MA5-11748, ThermoFisher Scientific) antibody was used at 1:100, whereas myogenin (F5D, Developmental Hybridomas) was used at a concentration of 2.56 µg/mL. Secondary goat anti-mouse IgG1-488 Alexa-Fluor antibody (Invitrogen) was incubated at a dilution of 1:250 for 1 hour. Slides were mounted with VectaShield containing DAPI (Vector Laboratories) and visualized using Nikon A1R confocal on Eclipse T1 inverted microscope. Images were analyzed with Fiji.

### Statistical analysis

For quantitation of cell fusion in FIGs. 1H and 2B, cells with 3 or more nuclei were considered syncytial cells. The number of nuclei in syncytial cells and total number of nuclei were manually counted. To quantify fusion between myomaker⁺ myomerger⁺ GFP⁺ fibroblasts with either myomaker⁺ NLS-Tom⁺ or myomerger⁺ NLS-Tom⁺ fibroblasts (FIG. 2A), we calculated the percentage of GFP⁺ NLS-Tom⁺ syncytial cells. In FIG. 2D, the number of myosin⁺ myotubes (myosin structures with 3 or more nuclei) and GFP⁺ myosin⁺ myotubes were manually counted. The differentiation index (FIG. 4E) was calculated as the percentage of nuclei in myosin⁺ cells, and the fusion index (FIG. 4F) as the percentage of myosin⁺ cells with the indicated number of nuclei. For FIG. 4E, fusion was expressed as the percentage of myosin⁺ cells with ≥ 3 nuclei. Quantitative data sets are presented as means ± SEM. For each quantitation, at least 3 independent experiments were performed in duplicate and 4-6 fields were randomly chosen for imaging. Histological analysis of embryos was performed on 3-4 embryos per genotype per time point. Multiple histological levels within each muscle were examined. The data were analyzed using an unpaired Student's t-test (two-tailed) with GraphPad Prism 6 software. A value of *P* < 0.05 was considered statistically significant.

### Results

### Identification and fusogenic activity of myomerger.

To uncover potential fusion factors, we compared genes induced by expression of MyoD to their level of expression in 10T ½ fibroblasts. Of the top 100 MyoD-regulated genes not expressed in fibroblasts (data not shown), we eliminated genes not likely to be directly involved in fusion (transcription factors, sarcomeric and metabolic genes) and focused on genes with transmembrane domains. This analysis yielded the following five genes: *Tmem182*, *Gm7325*, *Cdh15, Tspan33*, and *Tm6sf1*, however *Cdh15* was omitted from further analysis because it has previously been shown as not necessary for myoblast fusion or muscle formation. We retrovirally expressed each gene in myomaker⁺ GFP⁺ fibroblasts and assayed for fusion. Appropriate expression in fibroblasts was verified through quantitative reverse transcription polymerase chain reaction (qRT-PCR) analysis (FIG. 1A). We observed mainly mono-nucleated GFP⁺ cells in all cultures except when *Gm7325* was expressed where widespread multi-nucleated cells were present (FIG. 1B). Based on the ability of *Gm7325* to induce fusion of myomaker⁺ fibroblasts and the observations described below we named the protein myomerger.

Multiple *Gm7325* transcripts are annotated in the University of California, Santa Cruz, mouse genome. The shorter transcript contains a single exon and yields a protein with 84 amino acids. In contrast, the longer transcript utilizes an upstream exon with an alternative start site and results in a protein of 108 amino acids (FIG. 1C). The single coding exon of the short transcript is conserved in other mammalian genomes, including humans, while the upstream alternative exon leading to the longer transcript is not highly conserved (FIG. 1D). For the initial screen, we cloned the *Gm7325* locus into a retroviral vector, allowing normal splicing and expression of both short and long transcripts. Transduction of myomaker⁺ fibroblasts with either myomerger-short (S) or myomerger-long (L) induced formation of multi-nucleated cells, indicating both proteins are sufficient for fusion (FIG. 1E). Additionally, myomerger and myomaker together induced fusion of 3T3 fibroblasts and MSCs (FIG. 1F), suggesting these two genes could activate fusion in a multitude of cell types.

Given that multi-nucleated cells could arise from fusion or replication associated with incomplete cytokinesis, we designed a system to validate that multi-nucleated cells observed in fibroblasts expressing both myomerger and myomaker were generated through fusion. We engineered two fibroblast cell lines that both express myomaker, with one expressing GFP and the other expressing nuclear-localized TdTomato (NLS-Tom). Myomaker⁺ GFP⁺ and myomaker⁺ NLS-Tom⁺ fibroblasts were infected with a myomerger retrovirus or a control empty retrovirus, mixed, and fusion was assessed (FIG. 1G). We observed cells with multiple nuclei containing both GFP and NLS-Tom in fibroblasts expressing myomaker and myomerger indicating fusion (FIG. 1G). Quantification of fusion revealed approximately 20% of nuclei were contained in syncytia in cultures where fibroblasts were expressing both myomaker and myomerger (FIG. 1H). These results confirm that the observed syncytial cells are formed through fusion and that expression of myomaker and myomerger is sufficient to confer fusogenicity in non-fusogenic fibroblasts.

We also sought to determine the cell biology of fusion induced by myomaker and myomerger. We mixed myomaker⁺ myomerger⁺ GFP⁺ fibroblasts with NLS-Tom⁺ fibroblasts expressing myomaker or myomerger (FIG. 2A). Here we observed fusion of myomaker⁺ myomerger⁺ GFP⁺ fibroblasts with myomaker⁺ NLS-Tom⁺ but not myomerger⁺ NLS-Tom⁺ fibroblasts (FIG. 2A). We detected 10% of nuclei in syncytia (FIG. 2A), lower than the fusion observed when both cells express myomaker and myomerger (FIG. 1H) suggesting an enhanced fusogenic efficiency when cells express both proteins. Nonetheless, these data indicate that myomerger does not appear to be sufficient for fusion in the absence of myomaker. This heterotypic nature of fibroblast fusion (where myomaker appears to be required on both cells and myomerger only appears to be required on one cell) are consistent with our previously reported heterologous fusion system between myoblasts and fibroblasts. In that system, myomaker⁺ fibroblasts that do not express myomerger fused with muscle cells, which express both myomaker and myomerger. To confirm this concept, we utilized our heterologous fusion system where fibroblasts were infected with GFP and either empty, myomaker, or myomerger retrovirus, and then mixed with C2C12 myoblasts (FIG. 2C). In this assay, fusion is detected through co-localization of GFP (fibroblasts) with myosin⁺ myotubes. Compared to empty-infected GFP⁺ fibroblasts, we detected an increase in fusion between myosin⁺ cells with either myomaker⁺ GFP⁺ fibroblasts or myomerger⁺ GFP⁺ fibroblasts (FIG. 2C and FIG. 2D). However, quantification of myosin⁺ GFP⁺ cells revealed that myomerger did not drive the fusion of fibroblasts with muscle cells to the levels observed with myomaker (FIG. 2D). These data confirm that myomaker appears to be required in both fusing cells for *in vitro* fusion, while myomerger is appears to be required in one fusing cell.

### Myomerger is muscle-specific and associates with membranes

We interrogated *Gm7325* expression pattern more thoroughly. We performed qRT-PCR on multiple tissues from postnatal (P) day 5 mice with primers to distinguish the two potential mouse transcripts (FIG. 3A). In neonatal tissues, we detected expression of both myomerger transcripts only in skeletal muscle (FIG. 3B). Despite the evidence of two myomerger transcripts, immunoblot analysis of skeletal muscle lysates from P5 mice using a commercially available antibody identified a single band at approximately 12 kDa. This band was absent in P28 lysates indicating that myomerger is downregulated after neonatal development (FIG. 3C). Skeletal muscle exhibits a robust ability to regenerate due to the presence of muscle stem cells, also known as satellite cells. We analyzed expression of myomerger in *mdx*^{4cv} mice, which is a mouse model of muscular dystrophy that leads to chronic cycles of degeneration and regeneration. (DURBEEJ et al. "Muscular dystrophies involving the dystrophin-glycoprotein complex: An overview of current mouse models" (2002) Curr Opin Genet Dev, Vol. 12, pp. 349-361.) Myomerger expression was detected in diaphragm lysates from *mdx*^{4cv} mice, but not control diaphragms (FIG. 3D). Finally, we analyzed expression of myomerger in a model of skeletal muscle overload-induced (MOV) hypertrophy and observed up-regulation (FIG. 3E). Collectively, these data demonstrate that myomerger is expressed during development and is induced during adult myogenesis.

We next sought to determine if myomerger is regulated as myoblasts differentiate. In C2C12 cells, both *Gm7325* transcripts were significantly elevated during differentiation (FIG. 3F). Similarly, myomerger protein levels were low in proliferating myoblasts (day 0), but increased upon differentiation with expression maintained during myoblast differentiation and fusion into myotubes (Fig. 3G). The short mouse myomerger protein, but not the long form, is highly conserved among vertebrate species (FIG. 3H). After transduction of C2C12 cells with empty, myomerger-S, or myomerger-L, we detected an increased upper band in cells expressing either myomerger-S or myomerger-L that co-migrated with the 12 kDa endogenous protein in the empty-infected C2C12 cells (FIG. 3I). A lower band was identified exclusively in myomerger-S lysates suggesting that complex mRNA or post-translational processing results in the endogenous single 12 kDa band observed in WT C2C12 cells and muscle homogenates. Both myomerger proteins harbor a hydrophobic region close to the N-terminus, where computational analysis of this region appears to indicate a signal peptide or transmembrane domain (FIG. 3J). Both variants were found to confer fusogenicity. To understand subcellular localization, we fractionated C2C12 cells on day 2 of differentiation and identified myomerger in membrane fractions containing caveolin-3, a protein known to associate with both heavy and light vesicles (FIG. 3K). Immunostaining of fibroblasts expressing myomerger-S or myomerger-L shows that both proteins exhibit similar diffuse and vesicular localization (FIG. 3L). Thus, myomerger associates with membrane compartments which is consistent with its ability to induce fusion.

### Role of myomerger in myoblast fusion

The ability of myomerger to induce fusion of myomaker-fibroblasts, and its muscle-specific expression in the mouse, suggests that myomerger may play a role during myogenesis. To begin to decipher the role of myomerger in myogenesis, we evaluated its function during myoblast differentiation. We utilized CRISPR/Cas9 genome editing to disrupt myomerger in C2C12 myoblasts. Two guide RNAs (gRNA) were designed to target the largest exon of *Gm7325,* which resulted in a 166 base pair deletion thereby disrupting both mouse transcripts (FIG. 4A). C2C12 cells were transfected with a plasmid containing Cas9 with an IRES-GFP and myomerger gRNAs, or transfected with only Cas9-IRES-GFP as a control. Flow cytometry of GFP⁺ cells followed by genotyping through PCR analysis revealed disruption of the myomerger locus (FIG. 4B). Myomerger was not detectable in myomerger KO C2C12 cells confirming efficient disruption of the locus (FIG. 4C). Control and myomerger KO C2C12 cells were then analyzed for their ability to differentiate and form myotubes. WT myoblasts differentiated, as indicated by myosin⁺ cells, and fused to form multi-nucleated myotubes (FIG. 4D). In contrast, myomerger KO C2C12 cells exhibited the ability to differentiate but lacked fusogenic activity to form myotubes (FIG. 4D). Indeed, quantification of the differentiation index revealed no difference in the percentage of myosin⁺ cells between WT and myomerger KO cultures (FIG. 4E). Additionally, quantification of fusion demonstrated that myomerger KO myosin⁺ cells remain mono-nucleated while WT cells fuse (FIG. 4F). qRT-PCR analysis for the myogenic genes *Myogenin*, *Myh4*, *Ckm*, and *Tmem8c* (myomaker) further indicated that myomerger KO myoblasts activate the differentiation program (FIG. 4G). Interestingly, myogenic transcripts were elevated in myomerger KO cells potentially suggesting a feedback mechanism by which non-fusogenic cells attempt to further differentiate (FIG. 4G). Infection of myomerger KO C2C12 cells with either myomerger-S or myomerger-L rescued the fusion defect demonstrating that the phenotype in these cells is specifically due to the loss of myomerger and not an off-target effect of Cas9 (FIG. 4H). Western blot analysis from these lysates shows re-expression of myomerger in KO cells (FIG. 4I). As a potential mechanism for the lack of fusion in myomerger KO myocytes, we examined expression and localization of myomaker. On day 2 of differentiation, myomerger KO cells exhibited normal expression and localization of myomaker (FIG. 5A). Moreover, we did not detect widespread co-localization between myomaker and myomerger suggesting that myomerger does not directly regulate myomaker distribution (FIG. 5B). These data reveal that myomerger is used in myoblast fusion *in vitro* through a mechanism that does not involve regulation of myomaker levels or localization.

### Role of myomerger in muscle formation in vivo

To examine the function of myomerger *in vivo*, we disrupted exon 3 using the same CRISPR/Cas9 strategy described for C2C12 myoblasts. Injection of Cas9 and myomerger gRNAs into blastocysts resulted in lethality of 9 of the 10 F₀ pups, suggesting that the high efficiency of Cas9 lead to homozygous deletion of myomerger. The one remaining pup was heterozygous for myomerger (FIG. 6A) and sequencing of the mutant PCR product revealed the presence of the same mutation as was achieved in C2C12 cells. The heterozygous founder was mated to WT mice for multiple generations, which controlled for potential off-target effects given that we only selected pups with the *Gm7325* mutation. Heterozygous mice from these litters were then crossed to generate *Gm7325*^{*-*/*-*} mice. We failed to observe any *Gm7325*^{*-*/*-*} mice upon genotyping at P7 suggesting that myomerger is essential for life. Indeed, E17.5 *Gm7325*^{*-*/*-*} embryos exhibited minimal skeletal muscle upon gross examination (Fig. 6B). Specifically, bones of the limbs and rib cage were noticeable due to a scarcity of surrounding muscle as observed in WT embryos. Myomerger KO mice also displayed a hunched appearance with elongated snouts, hallmark characteristics of embryos with improper muscle formation (FIG. 6B). Detection of myomerger by western blot of WT and *Gm7325*^{*-*/*-*} tongues showed elimination of myomerger protein in KO samples (FIG. 6C). E15.5 forelimb sections showed that myomerger KO myoblasts express myogenin indicating that specification and differentiation were activated despite loss of myomerger (FIG. 6D). Moreover, histological analysis of multiple muscle groups at E15.5 revealed the presence of myosin⁺ muscle cells and sarcomeric structures in myomerger KO mice, (FIG. 6E and FIG. 6F). While multi-nucleated myofibers were present in WT mice, these structures were not readily detected in myomerger KO mice indicating that genetic loss of myomerger renders myocytes non-fusogenic (FIG. 6E and FIG. 6F). Analysis of forelimbs from E17.5 WT and myomerger KO embryos confirm that myomerger KO myoblasts are unable to properly fuse, although we did detect myocytes with two nuclei (FIG. 6G, FIG. 6H, and FIG. 6I). These results, together with our *in vitro* analysis, reveal that myomerger is used in muscle formation during mammalian development through regulation of myoblast fusion.

### Discussion

In summary, we report the discovery of an additional muscle-specific factor used in myoblast fusion and developmental myogenesis. While myomaker and myomerger appear to be used in muscle formation, E17.5 myomerger KO embryos grossly exhibit more myocytes compared to embryos lacking myomaker suggesting that these two myoblast fusion proteins may have distinct functions. We did not detect robust co-localization indicating that there is no physical interaction between myomaker and myomerger or that any potential interaction is transient or at discrete cellular locations.

Our data from the cell mixing experiments reveal that myomaker appears to be necessary in both fusing cells while myomerger only appears to be required in one fusing cell. This indicates that, for reconstitution of cell fusion, both fusing cells must become fusion competent (amenable to fuse), while only one cell needs to become fusogenic to allow syncytial formation. With this concept in mind, our data suggest that myomaker allows the cell to become fusion competent, whereas myomerger confers fusogencity. The cell mixing experiments also indicate that myomerger may require myomaker activity for fusogenic function because myomerger-expressing fibroblasts do not fuse to fibroblasts expressing both myomaker and myomerger. Without wishing to bound by theory, these data potentially suggest that myomerger acts downstream of myomaker, where myomaker acts as an initiator of fusion and myomerger executes the final steps to drive syncytial formation.

Without wishing to bound by theory, the distinct consequences of myomaker and myomerger expression in fibroblasts are consistent with the idea that the two proteins regulate different aspects of fusion. Membrane fusion appears to be a complex process that often includes membrane apposition and tethering, mixing of the outer membranes (hemifusion), pore formation, and pore expansion. Classical viral fusogens, as well as Eff-1, are large proteins with long ectodomains that are able to accomplish all of the steps necessary for fusion. The discovery of myomerger as an additional myoblast fusion factor could indicate that in higher organisms these multiple functions of viral proteins have been delegated to different myocyte proteins. Without wishing to bound by theory, this evolutionary strategy, at least in the case of muscle fusion, could provide more regulatory control to ascertain that cells are compatible for fusion.

Without wishing to bound by theory, the myomerger association with membranes could indicate that it functions to alter membrane dynamics that overcomes the thermodynamic barriers for fusion. Without wishing to bound by theory, myomerger activation of fusion through cytoskeletal alterations would be consistent with induction of fusogencity, as cytoskeletal alterations provide the necessary tension to induce membrane fusion in various systems.

The headings used in the disclosure are not meant to suggest that all disclosure relating to the heading is found within the section that starts with that heading. Disclosure for any subject may be found throughout the specification.

It is noted that terms like "preferably," "commonly," and "typically" are not used herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

As used in the disclosure, "a" or "an" means one or more than one, unless otherwise specified. As used in the claims, when used in conjunction with the word "comprising" the words "a" or "an" means one or more than one, unless otherwise specified. As used in the disclosure or claims, "another" means at least a second or more, unless otherwise specified. As used in the disclosure, the phrases "such as", "for example", and "e.g." mean "for example, but not limited to" in that the list following the term ("such as", "for example", or "e.g.") provides some examples but the list is not necessarily a fully inclusive list. The word "comprising" means that the items following the word "comprising" may include additional unrecited elements or steps; that is, "comprising" does not exclude additional unrecited steps or elements.

Unless otherwise indicated, all numbers expressing quantities of ingredients, properties such as reaction conditions, and so forth used in the specification and claims are to be understood as being modified in all instances by the term "about". Accordingly, unless indicated to the contrary, the numerical parameters set forth in this specification and claims are approximations that can vary depending upon the desired properties sought to be obtained by the presently-disclosed subject matter.

As used herein, the term "about," when referring to a value or to an amount of mass, weight, time, volume, concentration or percentage is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed method.

## Claims

1. An *in vitro* method for fusing two or more cells comprising:
- contacting a first cell with a second cell to form a third cell;
wherein
- the first cell is a modified cell comprising a myomerger nucleic acid, a first myomerger polypeptide, and a first myomaker polypeptide; wherein the first cell is a non-muscle cell;
- the second cell comprises a second myomaker polypeptide and optionally comprises a second myomerger polypeptide;
- the third cell is a multinucleated cell;
- the myomerger nucleic acid sequence encodes a polypeptide comprising the first myomerger polypeptide and has at least an 80% identity to one or more of SEQ ID NO: 43, SEQ ID NO: 44, or SEQ ID NO: 45,; and
- the first myomerger polypeptide sequence and the second myomerger polypeptide sequence each have at least an 80% identity to one or more of SEQ ID NO: 36, SEQ ID NO: 37, and SEQ ID NO: 38.

2. The *in vitro* method of claim 1, wherein the second cell comprises the second myomerger polypeptide.

3. The *in vitro* method of any of claims 1-2, wherein the second cell is a muscle cell, preferably a myoblast.

4. The *in vitro* method of any of claims 1-3, wherein the contacting occurs *ex vivo.*

5. An *in vitro* method for delivering a gene of interest comprising:
- contacting a first cell with a second cell *in vitro,* which fuse to form a third cell;
wherein
- the first cell is a modified cell comprising a myomerger nucleic acid, a first myomerger polypeptide, a first myomaker polypeptide, and a gene of interest, and wherein the first cell is a non-muscle cell;
- the second cell comprises a second myomaker polypeptide and optionally comprises a second myomerger polypeptide;
- the third cell is a multinucleated cell and the gene of interest is delivered to the third cell upon fusion of the first cell with the second cell;
- the myomerger nucleic acid sequence has at least an 80% identity to one or more of SEQ ID NO: 43, SEQ ID NO: 44, or SEQ ID NO: 45,; and
- the first myomerger polypeptide sequence and the second myomerger polypeptide sequence each have at least an 80% identity to one or more of SEQ ID NO: 36, SEQ ID NO: 37, and SEQ ID NO: 38.

6. The *in vitro* method of claim 5, wherein the second cell comprises the second myomerger polypeptide.

7. The *in vitro* method of claim 5 or claim 6, wherein the second cell is a muscle cell, preferably a myoblast.

8. The *in vitro* method of any of claims 5-7, wherein the contacting is *ex vivo.*

9. The *in vitro* method of any of claims 5-8, wherein the second cell underexpresses the gene of interest, does not express the gene of interest, or expresses a defective version of the gene of interest.

10. A pharmaceutical composition for use in the treatment of a disease, the composition comprising:
- a first cell that is a modified cell comprising a myomerger nucleic acid, a first myomerger polypeptide, a first myomaker polypeptide, and a gene of interest, and wherein the first cell is a non-muscle cell; and
- a second cell comprising a second myomaker polypeptide and optionally comprising a second myomerger polypeptide,
wherein
- the myomerger nucleic acid sequence has at least an 80% identity to one or more of SEQ ID NO: 43, SEQ ID NO: 44, or SEQ ID NO: 45,; and
- the first myomerger polypeptide sequence and the second myomerger polypeptide sequence each have at least an 80% identity to one or more of SEQ ID NO: 36, SEQ ID NO: 37, and SEQ ID NO: 38.

11. The pharmaceutical composition for use of claim 10, wherein the disease is a disease of the heart, diaphragm, head, neck, tongue, esophageal muscles, intestinal muscles, skeletal muscles, or genitourinary tract; or is selected from: myopathy, muscular dystrophy, amyotrophic lateral sclerosis, glycogen storage disease type II, rhabdomyosarcoma, sarcopenia, and cancer.

12. The *in vitro* method of any of claims 5-9, or the pharmaceutical composition for use of claims 10 or 11, wherein the gene of interest encodes for a therapeutic polypeptide, a therapeutic protein, or a therapeutic oligopeptide.

13. The *in vitro* method of any of claims 5-9, or the pharmaceutical composition for use of claims 10 or 11, wherein the first myomerger polypeptide and the second myomerger polypeptide each induce fusogenicity and/or confer fusogenic activity to normally non-fusogenic cells.

## Patentansprüche

1. In-vitro-Verfahren zum Verschmelzen von zwei oder mehr Zellen, umfassend:
- Inkontaktbringen einer ersten Zelle mit einer zweiten Zelle, um eine dritte Zelle zu bilden;
wobei
- die erste Zelle eine modifizierte Zelle ist, die eine Myomerger-Nukleinsäure, ein erstes Myomerger-Polypeptid und ein erstes Myomaker-Polypeptid umfasst; wobei die erste Zelle eine nicht-Muskelzelle ist;
- die zweite Zelle ein zweites Myomaker-Polypeptid umfasst und optional ein zweites Myomerger-Polypeptid umfasst;
- die dritte Zelle eine mehrkernige Zelle ist;
- die Myomerger-Nukleinsäuresequenz für ein Polypeptid kodiert, das das erste Myomerger-Polypeptid umfasst und mindestens 80 % Identität aufweist mit einer oder mehreren von SEQ ID NO: 43, SEQ ID NO: 44, oder SEQ ID NO: 45; und
- die erste Myomerger-Polypeptidsequenz und die zweite Myomerger-Polypeptidsequenz jeweils mindestens 80 % Identität aufweisen mit einer oder mehreren der Sequenzen SEQ ID NO: 36, SEQ ID NO: 37, und SEQ ID NO: 38.

2. In-vitro-Verfahren nach Anspruch 1, wobei die zweite Zelle das zweite Myomerger-Polypeptid umfasst.

3. In-vitro-Verfahren nach einem der Ansprüche 1-2, wobei die zweite Zelle eine Muskelzelle, vorzugsweise ein Myoblast, ist.

4. In-vitro-Verfahren nach einem der Ansprüche 1-3, wobei das Inberührungbringen *ex vivo* erfolgt.

5. In-vitro-Verfahren zur Abgabe eines Gens von Interesse, umfassend:
- Inkontaktbringen einer ersten Zelle mit einer zweiten Zelle *in vitro,* die eine dritte Zelle zu bilden;
wobei
- die erste Zelle eine modifizierte Zelle ist, die eine Myomerger-Nukleinsäure, ein erstes Myomerger-Polypeptid, ein erstes Myomaker-Polypeptid und ein Gen von Interesse umfasst, und wobei die erste Zelle eine nicht-Muskelzelle ist;
- die zweite Zelle ein zweites Myomaker-Polypeptid umfasst und optional ein zweites Myomerger-Polypeptid umfasst;
- die dritte Zelle eine mehrkernige Zelle ist und das Gen von Interesse bei der Fusion der ersten Zelle mit der zweiten Zelle an die dritte Zelle abgegeben wird;
- die Myomerger-Nukleinsäuresequenz mindestens 80 % Identität aufweist mit einer oder mehreren von SEQ ID NO: 43, SEQ ID NO: 44, oder SEQ ID NO: 45; und
- die erste Myomerger-Polypeptidsequenz und die zweite Myomerger-Polypeptidsequenz jeweils mindestens 80 % Identität aufweist mit einer oder mehreren der Sequenzen SEQ ID NO: 36, SEQ ID NO: 37, und SEQ ID NO: 38.

6. In-vitro-Verfahren nach Anspruch 5, wobei die zweite Zelle das zweite Myomerger-Polypeptid umfasst.

7. In-vitro-Verfahren nach Anspruch 5 oder Anspruch 6, wobei die zweite Zelle eine Muskelzelle, vorzugsweise ein Myoblast, ist.

8. In-vitro-Verfahren nach einem der Ansprüche 5-7, wobei das Inkontaktbringen *ex vivo* erfolgt.

9. In-vitro-Verfahren nach einem der Ansprüche 5-8, wobei die zweite Zelle das Gen von Interesse unterexprimiert, das Gen von Interesse nicht exprimiert oder eine fehlerhafte Version des Gens von Interesse exprimiert.

10. Pharmazeutische Zusammensetzung zur Verwendung bei der Behandlung einer Krankheit, wobei die Zusammensetzung umfasst:
- eine erste Zelle, die eine modifizierte Zelle ist, die eine Myomerger-Nukleinsäure, ein erstes Myomerger-Polypeptid, ein erstes Myomaker-Polypeptid und ein Gen von Interesse umfasst, und wobei die erste Zelle eine nicht-Muskelzelle ist; und
- eine zweite Zelle, die ein zweites Myomaker-Polypeptid umfasst und optional ein zweites Myomerger-Polypeptid umfasst,
wobei
- die Myomerger-Nukleinsäuresequenz mindestens 80 % Identität aufweist mit einer oder mehreren von SEQ ID NO: 43, SEQ ID NO: 44, oder SEQ ID NO: 45; und
- die erste Myomerger-Polypeptidsequenz und die zweite Myomerger-Polypeptidsequenz jeweils mindestens 80 % Identität aufweisen mit einer oder mehreren der Sequenzen SEQ ID NO: 36, SEQ ID NO: 37, und SEQ ID NO: 38.

11. Pharmazeutische Zusammensetzung zur Verwendung nach Anspruch 10, wobei die Krankheit eine Krankheit des Herzens, des Zwerchfells, des Kopfes, des Halses, der Zunge, der Speiseröhrenmuskeln, der Darmmuskeln, der Skelettmuskeln oder des Urogenitaltrakts ist; oder ausgewählt ist aus: Myopathie, Muskeldystrophie, amyotropher Lateralsklerose, Glykogenspeicherkrankheit Typ II, Rhabdomyosarkom, Sarkopenie und Krebs.

12. In-vitro-Verfahren nach einem der Ansprüche 5-9 oder die pharmazeutische Zusammensetzung zur Verwendung nach Anspruch 10 oder 11, wobei das Gen von Interesse für ein therapeutisches Polypeptid, ein therapeutisches Protein oder ein therapeutisches Oligopeptid kodiert.

13. In-vitro-Verfahren nach einem der Ansprüche 5-9 oder pharmazeutische Zusammensetzung zur Verwendung nach Anspruch 10 oder 11, wobei das erste Myomerger-Polypeptid und das zweite Myomerger-Polypeptid jeweils Fusogenität induzieren und/oder normalerweise nicht-fusogenen Zellen fusogene Aktivität verleihen.

## Revendications

1. Procédé *in vitro* de fusion de deux ou plusieurs cellules comprenant :
- la mise en contact d'une première cellule avec une deuxième cellule pour former une troisième cellule ;
dans lequel
- la première cellule est une cellule modifiée comprenant un acide nucléique myomerger, un premier polypeptide myomerger et un premier polypeptide myomaker ; dans lequel la première cellule est une cellule non musculaire ;
- la deuxième cellule comprend un second polypeptide myomaker et comprend éventuellement un second polypeptide myomerger ;
- la troisième cellule est une cellule multinucléée ;
- la séquence d'acide nucléique de myomerger code pour un polypeptide comprenant le premier polypeptide myomerger et a une identité d'au moins 80 % avec une ou plusieurs des séquences SEQ ID NO: 43, SEQ ID NO: 44, ou SEQ ID NO: 45, ; et
- la première séquence polypeptidique myomerger et la seconde séquence polypeptidique myomerger ont chacune une identité d'au moins 80 % avec une ou plusieurs séquences SEQ ID NO: 36, SEQ ID NO: 37, et SEQ ID NO: 38.

2. Procédé *in vitro* selon la revendication 1, dans lequel la deuxième cellule comprend le second polypeptide myomerger.

3. Procédé *in vitro* selon l'une quelconque des revendications 1-2, dans lequel la deuxième cellule est une cellule musculaire, de préférence un myoblaste.

4. Procédé *in vitro* selon l'une quelconque des revendications 1-3, dans lequel la mise en contact a lieu *ex vivo.*

5. Procédé *in vitro* d'administration d'un gène d'intérêt comprenant :
- la mise en contact d'une première cellule avec une deuxième cellule in vitro, lesquelles fusionnent pour former une troisième cellule ;
dans lequel
- la première cellule est une cellule modifiée comprenant un acide nucléique myomerger, un premier polypeptide myomerger, un premier polypeptide myomaker et un gène d'intérêt, et dans lequel la première cellule est une cellule non musculaire ;
- la deuxième cellule comprend un second polypeptide myomaker et comprend éventuellement un second polypeptide myomerger ;
- la troisième cellule est une cellule multinucléée et le gène d'intérêt est administré à la troisième cellule lors de la fusion de la première cellule avec la deuxième cellule ;
- la séquence d'acide nucléique myomerger a une identité d'au moins 80 % avec une ou plusieurs séquences SEQ ID NO: 43, SEQ ID NO: 44, ou SEQ ID NO: 45, ; et
- la première séquence polypeptidique myomerger et la seconde séquence polypeptidique myomerger ont chacune une identité d'au moins 80 % avec une ou plusieurs séquences SEQ ID NO: 36, SEQ ID NO: 37, et SEQ ID NO: 38.

6. Procédé *in vitro* selon la revendication 5, dans lequel la deuxième cellule comprend le second polypeptide myomerger.

7. Procédé *in vitro* selon la revendication 5 ou la revendication 6, dans lequel la deuxième cellule est une cellule musculaire, de préférence un myoblaste.

8. Procédé *in vitro* selon l'une quelconque des revendications 5-7, dans lequel la mise en contact est *ex vivo.*

9. Procédé *in vitro* selon l'une quelconque des revendications 5-8, dans lequel la deuxième cellule sous-exprime le gène d'intérêt, n'exprime pas le gène d'intérêt, ou exprime une version défectueuse du gène d'intérêt.

10. Composition pharmaceutique pour une utilisation dans le traitement d'une maladie, la composition comprenant :
- une première cellule qui est une cellule modifiée comprenant un acide nucléique myomerger, un premier polypeptide myomerger, un premier polypeptide myomaker et un gène d'intérêt, et dans laquelle la première cellule est une cellule non musculaire ; et
- une deuxième cellule comprenant un second polypeptide myomaker et comprenant éventuellement un second polypeptide myomerger,
dans laquelle
- la séquence d'acide nucléique myomerger a une identité d'au moins 80 % avec une ou plusieurs séquences SEQ ID NO: 43, SEQ ID NO: 44, ou SEQ ID NO: 45, ; et
- la première séquence polypeptidique myomerger et la seconde séquence polypeptidique myomerger ont chacune une identité d'au moins 80 % avec une ou plusieurs séquences SEQ ID NO: 36, SEQ ID NO: 37, et SEQ ID NO: 38.

11. Composition pharmaceutique pour une utilisation selon la revendication 10, dans laquelle la maladie est une maladie du coeur, du diaphragme, de la tête, du cou, de la langue, des muscles oesophagiens, des muscles intestinaux, des muscles du squelette, ou de l'appareil génito-urinaire ; ou est choisie parmi : la myopathie, la dystrophie musculaire, la sclérose latérale amyotrophique, la maladie de stockage du glycogène de type II, le rhabdomyosarcome, la sarcopénie et le cancer.

12. Procédé *in vitro* selon l'une quelconque des revendications 5-9, ou composition pharmaceutique pour une utilisation selon les revendications 10 ou 11, dans lequel le gène d'intérêt code pour un polypeptide thérapeutique, une protéine thérapeutique ou un oligopeptide thérapeutique.

13. Procédé *in vitro* selon l'une quelconque des revendications 5-9, ou composition pharmaceutique pour une utilisation selon les revendications 10 ou 11, dans lequel le premier polypeptide myomerger et le second polypeptide myomerger induisent chacun la fusogénicité et/ou confèrent une activité fusogénique à des cellules normalement non fusogéniques.
